# EUROPÄISCHE PATENTSCHRIFT

(11) **EP 1 633 697 B1**
(45) Veröffentlichungstag und Bekanntmachung des Hinweises auf die Patenterteilung: **11.03.2009**
(21) Anmeldenummer: 04739583.5
(22) Anmeldetag: 04.06.2004
(51) Int. Cl.: C07C 217/62, C07C 211/27, C07C 211/28

(54) **VERFAHREN ZUR HERSTELLUNG SUBSTITUIERTER 3-ARYL-BUTYL-AMINVERBINDUNGEN**
METHOD FOR THE PRODUCTION OF SUBSTITUTED 3-ARYL-BUTYL AMINE COMPOUNDS
PROCEDE DE PRODUCTION DES COMPOSES D'AMINE 3-ARYLE-BUTYLE SUBSTITUE

(30) Priorität: 06.06.2003 DE 10326097
(43) Veröffentlichungstag der Anmeldung: 15.03.2006
(73) Patentinhaber: Grünenthal GmbH, 52078 Aachen (DE)
(72) Erfinder: HELL, Wolfgang, 52066 Aachen (DE); KEGEL, Markus, 52078 Aachen (DE); AKTERIES, Bernhard, 52078 Aachen (DE); BUSCHMANN, Helmut, E-08950 Esplugues de Llobregat (ES); HOLENZ, Jörg, E-08800 Vilanova i la Geltru (ES); LÖBERMANN, Hartmut, 52078 Aachen (DE); HELLER, D., Inst. für organische Katalyseforschung, 18055 Rostock (DE); DREXLER, H.J., Inst. für org. Katalyseforschung, 18055 Rostock (DE); GLADOW, Stefan, Fluka Production GmbH, CH-9471 Buchs (CH)
(86) Internationale Anmeldenummer: PCT/EP2004/006027
(87) Internationale Veröffentlichungsnummer: WO 2004/108658

(56) Entgegenhaltungen:
- EP-A- 0 693 475
- EP-A- 0 799 819
- WO-A-01/49651

## Beschreibung

Die vorliegende Erfindung betrifft ein Verfahren zur Dehydratisierung substituierter 1-Amino-3-aryl-butan-3-ol-Verbindungen zur Herstellung substituierter 3-Aryl-butyl-, amin-Verbindungen.

Die Behandlung chronischer und nichtchronischer Schmerzzustände hat in der Medizin eine große Bedeutung. Dies spiegelt sich in der Vielzahl an Veröffentlichungen wieder.

Aus der EP 0 693 475 ist eine Wirkstoffklasse von 3-Aryl-butyl-amin-Verbindungen, insbesondere Dimethyl-(3-aryl-butyl)-amin-Verbindungen mit exzellenter analgetischer Wirksamkeit und sehr guter Verträglichkeit bekannt. WO 01/049 651 offenbart Aminomethyl-Phenyl-Cyclohexanon-Derivate sowie Verfahren zu Ihrer Herstellung.

Die Herstellung dieser pharmazeutischen Wirkstoffe geht von tertiären Alkoholen aus, wobei diese zunächst in die entsprechende Chloridverbindung überführt und anschließend mit Zinkborhydrid, Zinkcyanoborhydrid oder Zinncyanoborhydrid reduziert werden. Dieses Verfahren hat den Nachteil, daß die Herstellung der Chloridverbindung unter Verwendung vergleichsweise aggressiver Chlorierungsmittel wie Thionylchlorid erfolgt und dieses auch noch in hohem Überschuß verwendet werden muss. Außerdem geht von den Hydrierungsreagenzien eine beträchtliche Brand- und gesundheitsgefahr aus. Des Weiteren verläuft dieses Verfahren nicht in allen Fällen mit zufriedenstellender Ausbeute.

Aufgabe der vorliegenden Erfindung war es daher, ein Verfahren zur Eliminierung der tertiären Alkoholfunktion aus substituierten 4-Amino-2-aryl-butan-2-ol-Verbindungen zur Verfügung zu stellen, mit dem die entsprechend substituierten 3-Aryl-butyl-aminverbindungen unter umweltschonenden Bedingungen in guten Ausbeuten erhalten werden. Weiteres Ziel des Verfahrens ist es, im Fall von eingesetzten substituierten stereochemisch reinen Verbindungen die Enantiomerenreinheit zu erhalten.

Erfindungsgemäß wird diese Aufgabe durch die Bereitstellung der nachstehend beschriebenen Verfahren zur Dehydratisierung substituierter 1-Amino-3-aryl-butan-3-ol-Verbindungen der nachstehend angegebenen allgemeinen Formel II zur Herstellung substituierter 3-Aryl-butyl-amin-Verbindungen der nachstehend angegebenen allgemeinen Formel I gelöst. Die Verbindungen der allgemeinen Formel I werden vorzugsweise als pharmazeutische Wirkstoffe in Arzneimitteln eingesetzt und eignen sich insbesondere zur Schmerzbekämpfung.

Ein Gegenstand der vorliegenden Erfindung ist daher ein Verfahren zur Herstellung einer substituierten 3-Aryl-butyl-amin-Verbindung der allgemeinen Formel I, 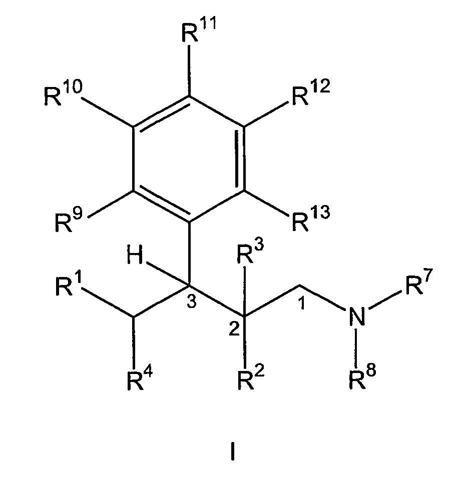 , worin
R¹ ausgewählt ist aus H, C₁₋₃-Alkyl, verzweigt oder unverzweigt, gesättigt oder ungesättigt, unsubstituiert oder ein- oder mehrfach substituiert,
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄₋₇-Cycloalkylrest bilden, unsubstituiert oder ein- oder mehrfach substituiert,
R⁴ ausgewählt ist aus H, C₁₋₃-Alkyl, verzweigt oder unverzweigt, gesättigt oder ungesättigt, unsubstituiert oder ein- oder mehrfach substituiert,
R⁷ und R⁸ jeweils unabhängig voneinander ausgewählt sind aus H oder C₁₋₃-Alkyl, verzweigt oder unverzweigt, gesättigt oder ungesättigt, unsubstituiert oder ein- oder mehrfach substituiert
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R¹⁸ jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates,
dadurch gekennzeichnet, daß in einem ersten Schritt a) eine 1-Amino-3-aryl-butan-3-ol-Verbindung der allgemeinen Formel II 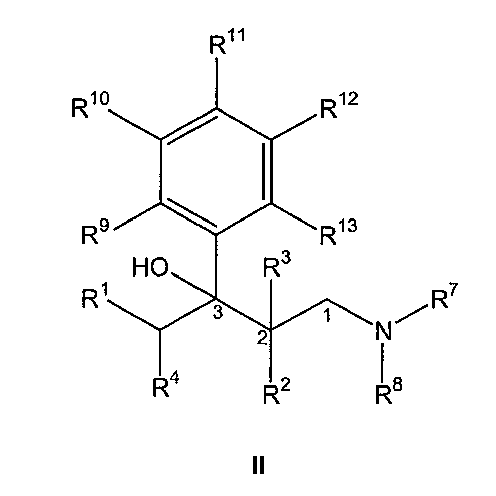 worin R¹, R², R³, R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, eingesetzt und unter Einwirkung einer Säure eliminiert wird zu einer substituierten 3-Aryl-but-3-enyl-amin-Verbindung der allgemeinen Formel III, 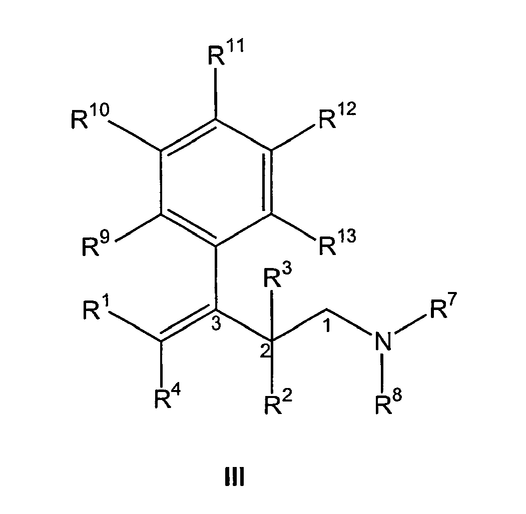 worin R¹, R², R³,R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, und in einem zweiten Schritt b) die entstehende substituierte 3-Aryl-but-3-enyl-amin-Verbindung gemäß der allgemeinen Formel III dann zu einer substituierten 3-Aryl-butyl-amin-Verbindung der allgemeinen Formel I unter Beteiligung eines Metallkatalysators und Wasserstoff hydriert wird.

Dieses Verfahren erlaubt eine Synthese mit hohen Ausbeuten, guter Umweltverträglichkeit und hoher Stereoselektivität.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4-oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7-oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3-oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cycloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und -CH₂-CH₂-CH₂-CH₂-CH₂-CH₂- zu verstehen, unter (CH₂)₁₋₄ ist -CH₂-, - CH₂-CH₂-, -CH₂-CH₂-CH₂- und -CH₂-CH₂-CH₂-CH₂- zu verstehen, unter (CH₂)₄₋₅ ist - CH₂-CH₂-CH₂-CH₂- und -CH₂-CH₂-CH₂-CH₂-CH₂- zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit R²³, OR²³ einem Halogen, vorzugsweise F und/oder Cl, einem CF₃, einem CN, einem NO₂, einem NR R , einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über eine C₁₋₃-Alkylen-Gruppe gebundenen Aryl- oder Heteroaryl-Rest, wobei diese Aryl und Heteroarylreste nicht selbst mit Aryl-oder Heteroaryl-Resten substituiert sein dürfen.

Unter dem Begriff Salz ist im Sinne dieser Erfindung jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes (insbesondere mit Kationen oder Basen) versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes (insbesondere mit Anionen oder Säuren) versteht man im Sinne dieser Erfindung weiter Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Äpfelsäure, Weinsäure, Mandelsäure, Fumarsäure, Milchsäure, Zitronensäure, Glutaminsäure, 1,1-Dioxo-1,2-dihydro1β6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt ist das Hydrochlorid-Salz.

Geeignete Salze im Sinne dieser Erfindung und in jeder beschriebenen Verwendung und jedem der beschriebenen Arzneimittel sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Verbindungen gemäß Formel I sowie gemäß Formel II und deren Herstellung sind aus der DE 44 26 245 A1 bzw. der US 6,248,737 bekannt. Verbindungen gemäß Formel III sind aus der EP 799 819 bzw. der US 5,811,582 bekannt.

Es ist in manchen Fällen bevorzugt, wenn das Produkt zwischen Schritt a und Schritt b isoliert wird. Dazu wird nach der Elimination gemäß Schritt a) mit einer Base, vorzugsweise einer Ammonium-Verbindung oder einer Hydroxid-Verbindung, insbesondere einer Lösung eines Alkali- oder Erdalkali-Hydroxids, bevorzugt NaOH-oder KOH-Lösung, zunächst neutralisiert und/oder zunächst ein basischer pH, vorzugsweise ≥ pH 9, insbesondere ≥ pH 10, bevorzugt zwischen pH 10 und pH 12,5, eingestellt. Anschließend wird ein organisches Lösungsmittel, vorzugsweise ein schwach wasserlösliches, polares organisches Lösungsmittel, insbesondere ein organischer Säureester, bevorzugt Essigsäureethylester oder Essigsäuremethylester, zugegeben und gerührt. Dieser Schritt ist auch ohne Lösungsmittel oder unter Verwendung von Diisopropylester möglich. Anschließend wird die verbleibende wässrige Phase verworfen und das gewünschte Produkt aus der organischen Phase isoliert, vorzugsweise durch Destillation, insbesondere im Vakuum.

Es ist für das erfindungsgemäße Verfahren bevorzugt, wenn für Verbindungen gemäß **Formel I, Formel II und Formel III** gilt, daß
R⁴ ausgewählt ist aus H oder CH₃,
vorzugsweise R⁴ H bedeutet.

Es ist für das erfindungsgemäße Verfahren bevorzugt, wenn für Verbindungen gemäß **Formel I, Formel II und Formel III** gilt, daß
R¹ ausgewählt ist aus C₁₋₃-Alkyl, gesättigt oder ungesättigt, substituiert oder unsubstituiert, verzweigt oder unverzweigt.

Es ist für das erfindungsgemäße Verfahren bevorzugt, wenn für Verbindungen gemäß **Formel I, Formel II** und **Formel III** gilt, daß
R⁴ ausgewählt ist aus H oder CH₃,
vorzugsweise R⁴ H bedeutet,
**und/oder**
R¹ ausgewählt ist aus C₁₋₃-Alkyl, gesättigt oder ungesättigt, substituiert oder unsubstituiert, verzweigt oder unverzweigt.

Es ist für das erfindungsgemäße Verfahren bevorzugt, wenn für Verbindungen gemäß **Formel I, Formel 11 und Formel III** gilt, daß
R⁷ und R⁸ jeweils unabhängig voneinander ausgewählt sind aus H oder CH₃,
vorzugsweise R⁷ und R⁸ H bedeuten oder R⁷ und R⁸ CH₃ bedeuten oder R⁷ H und R³ CH₃ bedeutet,
insbesondere R⁷ und R⁸ CH₃ bedeuten.

Es ist für das erfindungsgemäße Verfahren bevorzugt, wenn für Verbindungen gemäß **Formel I, Formel 11 und Formel III** gilt, daß
R¹ ausgewählt ist aus
C₁₋₃-Alkyl, gesättigt und unsubstituiert, verzweigt oder unverzweigt, vorzugsweise aus CH₃, C₂H₅, i-Propyl oder n-Propyl, insbesondere aus CH₃ oder C₂H₅.

Es ist für das erfindungsgemäße Verfahren bevorzugt, wenn für Verbindungen gemäß **Formel I, Formel 11 und Formel III** gilt, daß
R² und R³ unabhängig voneinander ausgewählt sind aus H, C₁₋₄-Alkyl, gesättigt und unsubstituiert, verzweigt oder unverzweigt; vorzugsweise aus H, CH₃, C₂H₅, i-Propyl oder t-Butyl, insbesondere aus H oder CH₃ oder C₂H₅.

Es ist für das erfindungsgemäße Verfahren bevorzugt, wenn für Verbindungen gemäß **Formel I, Formel II und Formel III** gilt, daß
R³ = H und R² ≠ H,
vorzugsweise R³ =H und R² = CH₃ oder C₂H₅,
insbesondere R³ = H und R² = CH₃.

Es ist für das erfindungsgemäße Verfahren bevorzugt, wenn für Verbindungen gemäß **Formel 1, Formel II und Formel III** gilt, daß
R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.

Es ist für das erfindungsgemäße Verfahren bevorzugt, wenn für Verbindungen gemäß **Formel I, Formel II und Formel III** gilt, daß
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise aus H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise aus OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise aus F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Es ist für das erfindungsgemäße Verfahren bevorzugt, wenn für Verbindungen gemäß **Formel I, Formel II und Formel III** gilt, daß
R¹ ausgewählt ist aus
C₁₋₃-Alkyl, gesättigt und unsubstituiert, verzweigt oder unverzweigt; vorzugsweise CH₃, C₂H₅ oder C₃H₇, insbesondere CH₃ oder C₂H₅,
**und/oder**
R² und R³ unabhängig voneinander ausgewählt sind aus
H, C₁₋₄-Alkyl, gesättigt und unsubstituiert, verzweigt oder unverzweigt; vorzugsweise aus H, CH₃, C₂H₅, i-Propyl oder t-Butyl, insbesondere aus H oder CH₃ oder C₂H₅,
vorzugsweise gilt:
R³ = H und R² ≠ H,
vorzugsweise R³ =H und R² = CH₃ oder C₂H₅,
insbesondere R³ = H und R² = CH₃.
oder
R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
**und/oder**
R⁴ ausgewählt ist aus H,
**und/oder**
R⁷ und R⁸ jeweils unabhängig voneinander ausgewählt sind aus
H oder CH₃,
vorzugsweise R⁷ und R⁸ H bedeuten oder R⁷ und R⁸ CH₃ bedeuten oder R⁷ H und R⁸ CH₃ bedeutet,
insbesondere R⁷ und R⁸ CH₃ bedeuten;
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise aus H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise aus OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise aus F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Es ist für das erfindungsgemäße Verfahren bevorzugt, wenn für Verbindungen gemäß **Formel I** mit R³ = H und R² ≠ H gilt, daß diese in den Konfigurationen la oder Ib 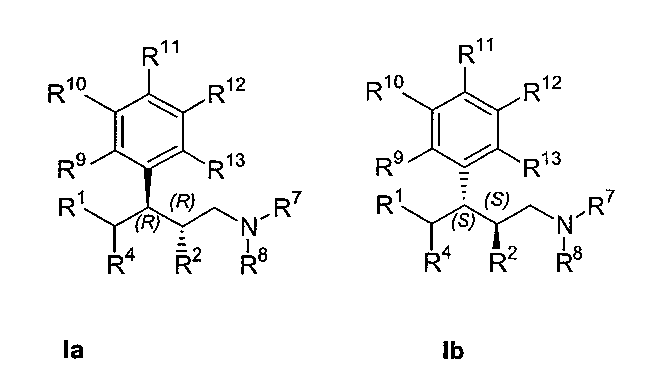 vorliegen.

Es ist für das erfindungsgemäße Verfahren bevorzugt, wenn für Verbindungen gemäß **Formel II** mit R³ = H und R2 ≠ H gilt, daß diese in den Konfigurationen IIa oder IIb 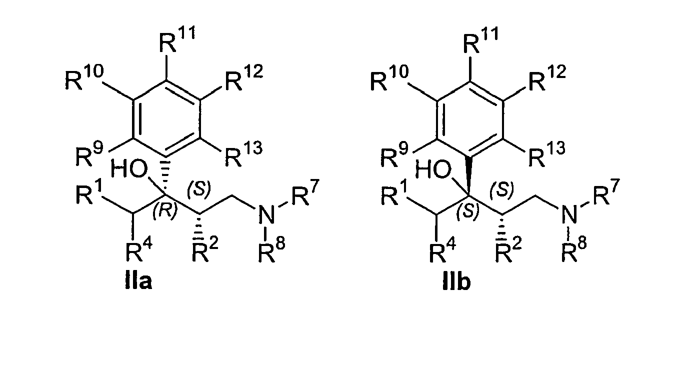 oder in den Konfigurationen IIc und IId 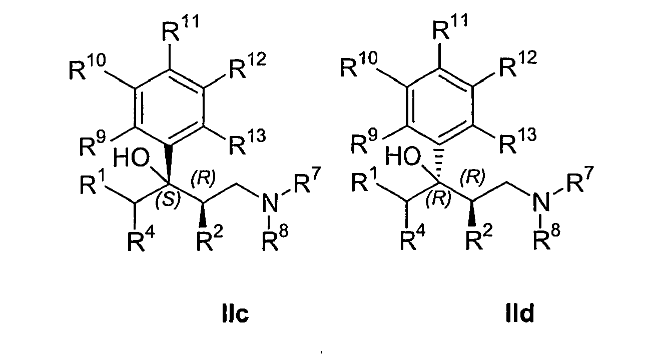 vorliegen.

Es ist für das erfindungsgemäße Verfahren bevorzugt, wenn für Verbindungen gemäß **Formell III** mit R³ = H, R² ≠ H, R⁴ = H und R¹ ≠ H gilt, daß diese in den Konfigurationen IIIa oder IIIb vorliegen 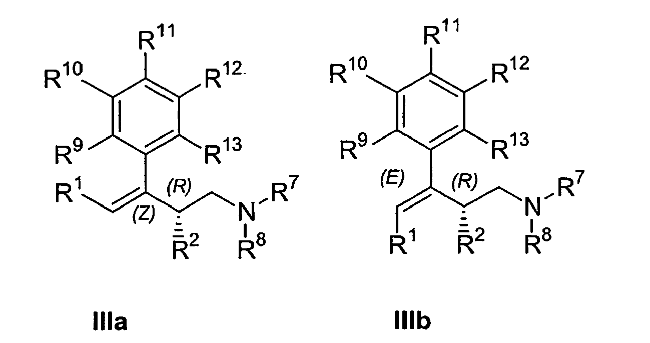 oder für Verbindungen gemäß **Formell III** mit R³ = H, R² ≠ H, R⁴ = H und R¹ ≠ H gilt, daß diese in den Konfigurationen IIIc oder IIId 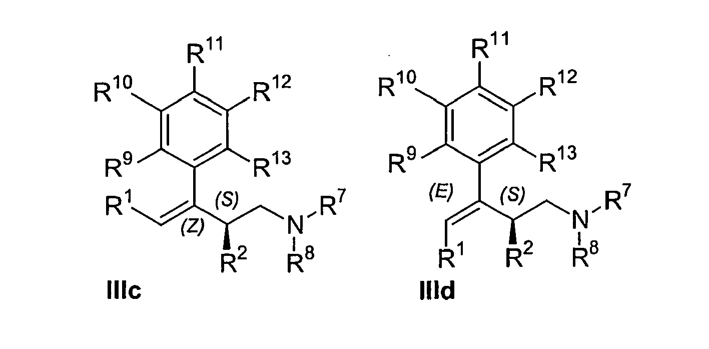 vorliegen.

Es ist für das erfindungsgemäße Verfahren bevorzugt, wenn für die Verbindung/Verbindungen gemäß **Formel I** gilt, daß mindestens eine davon, vorzugsweise als freie Base oder als Hydrochlorid, ausgewählt ist aus folgender Gruppe:
■ 3-(3-Dimethylamino-1-ethyl-2-methyl-propyl)-phenol,
■ (1 R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
■ (±)-(2RS,3RS)-[3-(3-Methoxy-phenyl)-2-methyl-pentyl]-dimethylamin,
■ *rac*(2RS,3RS)-[3-(3-Methoxy-phenyl)-2-methyl-pentyl]-dimethylamin,
■ 3{[3-(p-Isopropyl-phenyl-carbamoyl)-oxy-phenyl]-2-methylpentyl}-dimethylamin,
■ (2R,3R)-{3[3-(p-Isopropyl-phenyl-carbamoyl)-oxy-phenyl]-2-methyl-pentyl}-dimethylamin,
■ (2S,3S)-{3[3-(p-Isopropyl-phenyl-carbamoyl)-oxy-phenyl]-2-methyl-pentyl}-dimethylamin,
■ (2SR,3SR)-{3[3-(p-Isopropyl-phenyl-carbamoyl)-oxy-phenyl]-2-methyl-pentyl}-dimethylamin,
vorzugsweise
■ 3-(3-Dimethylamino-1-ethyl-2-methyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
insbesondere
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin.

Es ist für das erfindungsgemäße Verfahren bevorzugt, wenn für die eingesetzte Verbindung/eingesetzten Verbindungen gemäß **Formel II** gilt, daß mindestens eine davon, vorzugsweise als freie Base oder als Hydrochlorid, ausgewählt ist aus folgender Gruppe:
■ 3-(3-Dimethylamino-1-ethyl-1-hydroxy-2-methyl-propyl)-phenol,
■ (1S,2S)-3-(3-Dimethylamino-1-ethyl-1-hydroxy-2-methylpropyl)-phenol,
■ (1R,2S)-3-(3-Dimethylamino-1-ethyl-1-hydroxy-2-methylpropyl)-phenol,
■ (1RS,2SS)-3-(3-Dimethylamino-1-ethyl-1-hydroxy-2-methylpropyl)-phenol,
■ (1S,2R)-3-(3-Dimethylamino-1-ethyl-1-hydroxy-2-methylpropyl)-phenol,
■ (1R,2R)-3-(3-Dimethylamino-1-ethyl-1-hydroxy-2-methylpropyl)-phenol,
■ (1RS,2RR)-3-(3-Dimethylamino-1-ethyl-1-hydroxy-2-methylpropyl)-phenol,
■ [3-(3-Methoxy-phenyl)-2-methyl-pentan-3-ol]-dimethylamin,
■ (2S,3S)-[3-(3-Methoxy-phenyl)-2-methyl-pentan-3-ol]-dimethylamin,
■ (2S,3R)-[3-(3-Methoxy-phenyl)-2-methyl-pentan-3-ol]-dimethylamin,
■ (2SS,3RS)-[3-(3-Methoxy-phenyl)-2-methyl-pentan-3-ol]-dimethylamin,
■ (2R,3S)-[3-(3-Methoxy-phenyl)-2-methyl-pentan-3-ol]-dimethylamin,
■ (2R,3R)-[3-(3-Methoxy-phenyl)-2-methyl-pentan-3-ol]-dimethylamin,
■ (2RR,3RS)-[3-(3-Methoxy-phenyl)-2-methyl-pentan-3-ol]-dimethylamin,
■ {3[3-(p-Isopropyl-phenyl-carbamoyl)-oxy-phenyl]-2-methyl-pentan-3-ol}-dimethylamin,
■ (2S,3R)-{3[3-(p-Isopropyl-phenyl-carbamoyl)-oxy-phenyl]-2-methyl-pentan-3-ol}-dimethylamin,
■ (2S,3S)-{3[3-(p-Isopropyl-phenyl-carbamoyl)-oxy-phenyl]-2-methyl-pentan-3-ol}-dimethylamin,
■ (2SS,3RS)-{3[3-(p-Isopropyl-phenyl-carbamoyl)-oxy-phenyl]-2-methyl-pentan-3-ol}-dimethylamin,
■ (2R,3S)-{3[3-(p-Isopropyl-phenyl-carbamoyl)-oxy-phenyl]-2-methyl-pentan-3-ol}-dimethylamin,
■ (2R,3R)-{3[3-(p-Isopropyl-phenyl-carbamoyl)-oxy-phenyl]-2-methyl-pentan-3-ol}-dimethylamin,
■ (2RR,3RS)-{3[3-(p-Isopropyl-phenyl-carbamoyl)-oxy-phenyl]-2-methyl-pentan-3-ol}-dimethylamin.
vorzugsweise
■ 3-(3-Dimethylamino-1-ethyl-1-hydroxy-2-methyl-propyl)-phenol,
■ (1S,2S)-3-(3-Dimethylamino-1-ethyl-1-hydroxy-2-methylpropyl)-phenol,
■ (1R,2S)-3-(3-Dimethylamino-1-ethyl-1-hydroxy-2-methylpropyl)-phenol,
■ (1RS,2SS)-3-(3-Dimethylamino-1-ethyl-1-hydroxy-2-methylpropyl)-phenol,
■ (1S,2R)-3-(3-Dimethylamino-1-ethyl-1-hydroxy-2-methylpropyl)-phenol,
■ (1R,2R)-3-(3-Dimethylamino-1-ethyl-1-hydroxy-2-methylpropyl)-phenol,
■ (1RS,2RR)-3-(3-Dimethylamino-1-ethyl-1-hydroxy-2-methylpropyl)-phenol,
■ [3-(3-Methoxy-phenyl)-2-methyl-pentan-3-ol]-dimethylamin,
■ (2S,3S)-[3-(3-Methoxy-phenyl)-2-methyl-pentan-3-ol]-dimethylamin,
■ (2S,3R)-[3-(3-Methoxy-phenyl)-2-methyl-pentan-3-ol]-dimethylamin,
■ (2SS,3RS)-[3-(3-Methoxy-phenyl)-2-methyl-pentan-3-ol]-dimethylamin,
■ (2R,3S)-[3-(3-Methoxy-phenyl)-2-methyl-pentan-3-ol]-dimethylamin,
■ (2R,3R)-[3-(3-Methoxy-phenyl)-2-methyl-pentan-3-ol]-dimethylamin,
■ (2RR,3RS)-[3-(3-Methoxy-phenyl)-2-methyl-pentan-3-ol]-dimethylamin,
insbesondere
■ [3-(3-Methoxy-phenyl)-2-methyl-pentan-3-ol]-dimethylamin,
■ (2S,3S)-[3-(3-Methoxy-phenyl)-2-methyl-pentan-3-ol]-dimethylamin,
■ (2S,3R)-[3-(3-Methoxy-phenyl)-2-methyl-pentan-3-ol]-dimethylamin,
■ (2SS,3RS)-[3-(3-Methoxy-phenyl)-2-methyl-pentan-3-ol]-dimethylamin,
■ (2R,3S)-[3-(3-Methoxy-phenyl)-2-methyl-pentan-3-ol]-dimethylamin,
■ (2R,3R)-[3-(3-Methoxy-phenyl)-2-methyl-pentan-3-ol]-dimethylamin,
■ (2RR,3RS)-[3-(3-Methoxy-phenyl)-2-methyl-pentan-3-ol]-dimethylamin,
bevorzugt
■ (2S,3S)-[3-(3-Methoxy-phenyl)-2-methyl-pentan-3-ol]-dimethylamin,
■ (2S,3R)-[3-(3-Methoxy-phenyl)-2-methyl-pentan-3-ol]-dimethylamin,
oder
■ (2R,3R)-[3-(3-Methoxy-phenyl)-2-methyl-pentan-3-ol]-dimethylamin oder
■ (2R,3S)-[3-(3-Methoxy-phenyl)-2-methyl-pentan-3-ol]-dimethylamin.

Es ist für das erfindungsgemäße Verfahren bevorzugt, wenn für die Verbindung/Verbindungen gemäß **Formel III** gilt, daß mindestens eine davon, vorzugsweise als freie Base oder als Hydrochlorid, ausgewählt ist aus folgender Gruppe:
■ 3-(3-Dimethylamino-1-ethenyl-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl-2-methyl-propyl)-phenol,
■ (Z,E)-(2S)-3-(3-Dimethylamino-1-ethenyl-2-methyl-propyl)-phenol,
■ 3-(3-Dimethylamino-1-etheny -2-methyl-propyl)-phenol,
■ (Z)-(2R)-3-(3-Dimethylamino-1-ethenyl-1- 2-methyl-propyl)-phenol,
■ (E)-(2R)-3-(3-Dimethylamino-1-ethenyl-1- 2-methyl-propyl)-phenol,
■ (Z,E)-(2R)-3-(3-Dimethylamino-1-ethenyl-1- 2-methylpropyl)-phenol,
■ (Z)-(2S)-3-(3-Dimethylamino-1-ethenyl-1- 2-methyl-propyl)-phenol,
■ (E)-(2S)-(3-Dimethylamino-1-ethenyl-1-2-methyl-propyl)-phenol,
■ (Z,E)-(2S)-3-(3-Dimethylamino-1-ethenyl-2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin.
■ {3[3-(p-Isopropyl-phenyl-carbamoyl)-oxy-phenyl]-2-methyl-pent-3-enyl}-dimethylamin,
■ (Z)-(2R)-(3[3-(p-Isopropyl-phenyl-carbamoyl)-oxy-phenyl]-2-methyl-pent-3-enyl}-dimethylamin,
■ (E)-(2R)-{3[3-(p-Isopropyl-phenyl-carbamoyl)-oxy-phenyl]-2-methyl-pent-3-enyl}-dimethylamin,
■ (Z,E)-(2R)-{3[3-(p-Isopropyl-phenyl-carbamoyl)-oxy-phenyl]-2-methyl-pent-3-enyl}-dimethylamin,
■ (Z)-(2S)-{3[3-(p-Isopropyl-phenyl-carbamoyl)-oxy-phenyl]-2-methyl-pent-3-enyl}-dimethylamin,
■ (E)-(2S)-{3[3-(p-Isopropyl-phenyl-carbamoyl)-oxy-phenyl]-2-methyl-pent-3-enyl}-dimethylamin,
■ (Z,E)-(2S)-{3[3-(p-Isopropyl-phenyl-carbamoyl)-oxy-phenyl]-2-methyl-pent-3-enyl}-dimethylamin,
, vorzugsweise
■ 3-(3-Dimethylamino-1-ethenyl-1-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl-2-methyl-propyl)-phenol,
■ (Z,E)-(2S)-3-(3-Dimethylamino-1-ethenyl-2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin,
insbesondere
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin,
bevorzugt
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin oder
■ (Z,E)-(2R)-[3-(3-Methoxy-phenyl)-2-methyl-pent-3-enyl]-dimethylamin,
oder
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin oder
■ (Z,E)-(2S)-[3-(3-Methoxy-phenyl)-2-methyl-pent-3-enyl]-dimethylamin.

Es ist für das erfindungsgemäße Verfahren besonders bevorzugt, wenn bei der eingesetzten Verbindung gemäß Formel II an Position 2 gemäß Formel II ein chirales Zentrum vorliegt.

Es ist für das erfindungsgemäße Verfahren besonders bevorzugt, wenn bei der Verbindung gemäß Formel I an Position 2 gemäß Formel I ein chirales Zentrum vorliegt.

Es ist für das erfindungsgemäße Verfahren bevorzugt, wenn bei der Verbindung gemäß Formel III an Position 2 gemäß Formel III ein chirales Zentrum vorliegt.

Es ist für das erfindungsgemäße Verfahren bevorzugt, wenn die eingesetzte Verbindung nach Formel II enantiomerenrein ist.

Es ist für das erfindungsgemäße Verfahren bevorzugt, wenn die eingesetzte Verbindung nach Formel II diastereomerenrein ist.

Es ist für das erfindungsgemäße Verfahren bevorzugt, wenn die eingesetzte Verbindung nach Formel II enantiomeren- und diastereomerenrein ist.

Es ist für das erfindungsgemäße Verfahren besonders bevorzugt, wenn die eingesetzte Verbindung gemäß Formel II ausgewählt ist aus:
- (2S,3S)-[3-(3-Methoxy-phenyl)-2-methyl-pentan-3-ol]-dimethylamin,
- (2S,3R)-[3-(3-Methoxy-phenyl)-2-methyl-pentan-3-ol]-dimethylamin
oder
eine Mischung von (2S,3S)-[3-(3-Methoxy-phenyl)-2-methyl-pentan-3-ol]-dimethylamin und (2S,3R)-[3-(3-Methoxy-phenyl)-2-methyl-pentan-3-ol]-dimethylamin, bzw. (2SS,3RS)-[3-(3-Methoxy-phenyl)-2-methyl-pentan-3-ol]-dimethylamin ist.

Es ist für das erfindungsgemäße Verfahren besonders bevorzugt, wenn die eingesetzte Verbindung gemäß Formel II ausgewählt ist aus:
- (2R,3R)-[3-(3-Methoxy-phenyl)-2-methyl-pentan-3-ol]-dimethylamin,
- (2R,3S)-[3-(3-Methoxy-phenyl)-2-methyl-pentan-3-ol]-dimethylamin
oder
eine Mischung von (2R,3R)-[3-(3-Methoxy-phenyl)-2-methyl-pentan-3-ol]-dimethylamin und (2R,3S)-[3-(3-Methoxy-phenyl)-2-methyl-pentan-3-ol]-dimethylamin, bzw. (2RR,3RS)-[3-(3-Methoxy-phenyl)-2-methyl-pentan-3-ol]-dimethylamin ist.

Es ist für das erfindungsgemäße Verfahren bevorzugt, wenn die eingesetzte Verbindung gemäß Formel II ausgewählt ist aus:
- (2S,3S)-1-Dimethylamino-3-(3-methoxy-phenyl)-2-methyl-pentan-3-ol,
- (2S,3R)-1-Dimethylamino-3-(3-methoxy-phenyl)-2-methyl-pentan-3-ol
oder
eine Mischung von (2S,3S)-1-Dimethylamino-3-(3-methoxy-phenyl)-2-methyl-pentan-3-ol und (2S,3R)-1-Dimethylamino-3-(3-methoxy-phenyl)-2-methyl-pentan-3-ol, bzw. (2SS,3RS)-[3-(3-Methoxy-phenyl)-2-methyl-pentan-3-ol]-dimethylamin ist.

Es ist für das erfindungsgemäße Verfahren bevorzugt, wenn die eingesetzte Verbindung gemäß Formel II ausgewählt ist aus:
- (2R,3R)-1-Dimethylamino-3-(3-methoxy-phenyl)-2-methyl-pentan-3-ol,
- (2R,3S)-1-Dimethylamino-3-(3-methoxy-phenyl)-2-methyl-pentan-3-ol
oder
eine Mischung von (2R,3R)-1-Dimethylamino-3-(3-methoxy-phenyl)-2-methyl-pentan-3-ol und (2R,3S)-1-Dimethylamino-3-(3-methoxy-phenyl)-2-methyl-pentan-3-ol, bzw. (2RR,3RS)-[3-(3-Methoxy-phenyl)-2-methyl-pentan-3-ol]-dimethylamin ist.

Es ist für das erfindungsgemäße Verfahren bevorzugt, wenn in Schritt a) organische Säuren oder Halogen/Wasserstoffsäuren verwendet werden.

Es ist für das erfindungsgemäße Verfahren bevorzugt, wenn in Schritt a) Ameisensäure, Salzsäure oder Bromwasserstoffsäure verwendet werden.

Es ist für das erfindungsgemäße Verfahren bevorzugt, wenn in Schritt a) Ameisensäure verwendet wird.

Es ist für das erfindungsgemäße Verfahren bevorzugt, wenn in Schritt a) Salzsäure verwendet wird.

Es ist für das erfindungsgemäße Verfahren bevorzugt, wenn in Schritt a) Bromwasserstoffsäure verwendet wird.

Es ist für das erfindungsgemäße Verfahren bevorzugt, wenn die Säure in Schritt a) in hoher Konzentration eingesetzt wird.

Es ist für das erfindungsgemäße Verfahren bevorzugt, wenn die Salzsäure in Schritt a) >20%-ig, vorzugsweise > 30%-ig, insbesondere > 35%-ig ist.

Es ist für das erfindungsgemäße Verfahren bevorzugt, wenn nach dem Schritt a) die eliminierten Verbindungen gemäß Formel III mit Salzsäuregas kristallisiert werden.

Es ist für das erfindungsgemäße Verfahren bevorzugt, wenn die Reaktionszeit von Schritt a) zwischen 2 und 10h, vorzugsweise zwischen 3 und 8 h, insbesondere zwischen 4 und 6h beträgt.

Es ist für das erfindungsgemäße Verfahren bevorzugt, wenn in Schritt a) die Reaktionstemperatur zwischen 35 und 100°C, vorzugsweise 45 und 80°C, insbesondere zwischen 50 und 60°C beträgt.

Es ist für das erfindungsgemäße Verfahren bevorzugt, wenn in Schritt a) das Lösungsmittel ausgewählt ist aus:
H₂O oder Alkohol oder wässrigen alkoholischen Lösungen.

Es ist für das erfindungsgemäße Verfahren bevorzugt, wenn in Schritt a) das Lösungsmittel wässrige Säure ist.

Es ist für das erfindungsgemäße Verfahren bevorzugt, wenn in Schritt a) die eingesetzte Verbindung gemäß Formel II in wässriger Säure gelöst wird.

Es ist für das erfindungsgemäße Verfahren bevorzugt, wenn in Schritt a) die eingesetzte Verbindung gemäß Formel II in wässriger Salzsäure gelöst wird.

Es ist für das erfindungsgemäße Verfahren bevorzugt, wenn in Schritt b) das Lösungsmittel ausgewählt ist aus:
H₂O oder Alkohol oder wässrigen alkoholischen oder wässrigen sauren Lösungen, vorzugsweise aus wässrigen sauren Lösungen.

Es ist für das erfindungsgemäße Verfahren bevorzugt, wenn in Schritt b) das Lösungsmittel ausgewählt ist aus:
H₂O oder Ethanol oder wässriger ethanolischer Lösung oder wässriger Salzsäure, vorzugsweise aus wässriger Salzsäure.

Es ist für das erfindungsgemäße Verfahren bevorzugt, wenn in Schritt b) der verwendete Katalysator ein Edelmetall enthält, vorzugsweise Platin, Gold oder Palladium, insbesondere Palladium.

Es ist für das erfindungsgemäße Verfahren bevorzugt, wenn in Schritt b) der verwendete Katalysator Palladium auf Aktivkohle oder Palladium(II)-Chlorid ist.

Es ist für das erfindungsgemäße Verfahren bevorzugt, wenn in Schritt b) der verwendete Katalysator Palladium auf Aktivkohle (1 - 10 Gew.-%, bevorzugt 5 Gew.-%) ist.

Es ist für das erfindungsgemäße Verfahren bevorzugt, wenn die Temperatur in Schritt b) zwischen 20 und 40°C, vorzugsweise zwischen 20 und 35, insbesondere 25°C gehalten wird.

Es ist für das erfindungsgemäße Verfahren bevorzugt, wenn in Schritt b) vor der Hydrierung eine Schutzgasatmosphäre, insbesondere ein Stickstoffschutzgas, angelegt wird.

Es ist für das erfindungsgemäße Verfahren bevorzugt, wenn in Schritt b) der Hydrierungsschritt bei einem Wasserstoffvordruck von 3-10bar, vorzugsweise 4-7 bar, insbesondere 5 bar, stattfindet
und/ oder
der Hydrierungsschritt bei einem Wasserstoffinnendruck von 0,5-3 bar, vorzugsweise 0,75-2 bar, insbesondere 1 bar, stattfindet.

Es ist für das erfindungsgemäße Verfahren bevorzugt, wenn in Schritt b) zu Beginn die Ausgangsprodukte im Lösungsmittel stark verdünnt sind/werden.

Es ist für das erfindungsgemäße Verfahren bevorzugt, wenn das Lösungsmittel für beide Schritte a) und b) wässrige saure Lösung, vorzugsweise wässrige Salzsäure ist.

Es ist für das erfindungsgemäße Verfahren bevorzugt, wenn zwischen Schritt a) und Schritt b) kein Produkt isoliert wird. Dabei ist es besonders bevorzugt, wenn zu Beginn die Ausgangsprodukte im Lösungsmittel stark verdünnt sind/werden oder die eingesetzte Verbindung gemäß Formel II in wässriger Säure gelöst wird, insbesondere die eingesetzte Verbindung gemäß Formel II in wässriger Salzsäure gelöst wird.

Ein weiterer Gegenstand der Erfindung ist ein im folgenden Teilverfahren genanntes Verfahren zur Herstellung einer substituierten 3-Aryl-butyl-amin-Verbindung der allgemeinen Formel I, 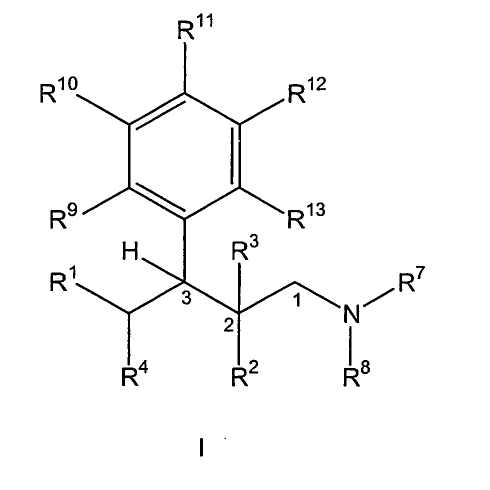 , worin
R¹ ausgewählt ist aus H, C₁₋₃-Alkyl, verzweigt oder unverzweigt, gesättigt oder ungesättigt, unsubstituiert oder ein- oder mehrfach substituiert,
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄-₇-Cycloalkylrest bilden, unsubstituiert oder ein- oder mehrfach substituiert,
R⁴ ausgewählt ist aus H, C₁₋₃-Alkyl, verzweigt oder unverzweigt, gesättigt oder ungesättigt, unsubstituiert oder ein- oder mehrfach substituiert,
R⁷ und R⁸ jeweils unabhängig voneinander ausgewählt sind aus H oder C₁₋₃-Alkyl, verzweigt oder unverzweigt, gesättigt oder ungesättigt, unsubstituiert oder ein- oder mehrfach substituiert
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF_{3;} SO₂CH₃, SO₂CF₃, CN, COOR¹⁴, NO₂, CONR¹⁷R¹⁸_{;} C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R¹⁸ jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates,
dadurch gekennzeichnet, daß eine substituierte 3-Aryl-but-3-enyl-amin-Verbindung der allgemeinen Formel III, 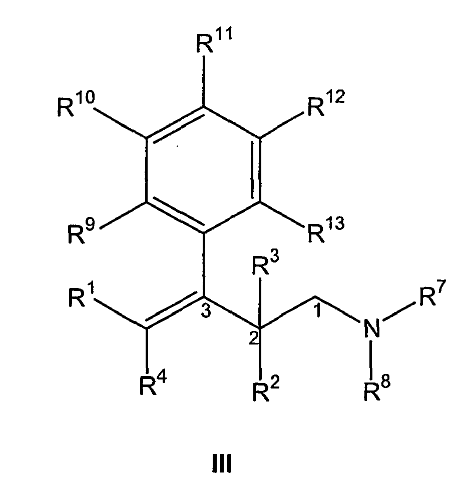 worin R¹, R², R³,R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, unter Beteiligung eines Metallkatalysators und Wasserstoff zu einer substituierten 3-Aryl-butyl-amin-Verbindung der allgemeinen Formel I hydriert wird.

Es ist für das erfindungsgemäße Teilverfahren besonders bevorzugt, wenn für Verbindungen gemäß **Formel I und Formel III** gilt, daß
R⁴ ausgewählt ist aus H oder CH₃,
vorzugsweise R⁴ H bedeutet.

Es ist für das erfindungsgemäße Teilverfahren besonders bevorzugt, wenn für Verbindungen gemäß **Formel I und Formel III** gilt, daß
R¹ ausgewählt ist aus C₁₋₃-Alkyl, gesättigt oder ungesättigt, substituiert oder unsubstituiert, verzweigt oder unverzweigt.

Es ist für das erfindungsgemäße Teilverfahren besonders bevorzugt, wenn für Verbindungen gemäß **Formel I und Formel III** gilt, daß
R⁴ ausgewählt ist aus H oder CH₃,
vorzugsweise R⁴ H bedeutet,
**und/oder**
R¹ ausgewählt ist aus C₁₋₃-Alkyl, gesättigt oder ungesättigt, substituiert oder unsubstituiert, verzweigt oder unverzweigt.

Es ist für das erfindungsgemäße Teilverfahren besonders bevorzugt, wenn für Verbindungen gemäß **Formel I und Formel III** gilt, daß
R⁷ und R⁸ jeweils unabhängig voneinander ausgewählt sind aus H oder CH₃,
vorzugsweise R⁷ und R⁸ H bedeuten oder R⁷ und R⁸ CH₃ bedeuten oder R⁷ H und R⁸ CH₃ bedeutet,
insbesondere R⁷ und R⁸ CH₃ bedeuten.

Es ist für das erfindungsgemäße Teilverfahren besonders bevorzugt, wenn für Verbindungen gemäß **Formel I und Formel III** gilt, daß
R¹ ausgewählt ist aus
C₁₋₃-Alkyl, gesättigt und unsubstituiert, verzweigt oder unverzweigt, vorzugsweise aus CH₃, C₂H₅, i-Propyl oder n-Propyl, insbesondere aus CH₃ oder C₂H₅.

Es ist für das erfindungsgemäße Teilverfahren besonders bevorzugt, wenn für Verbindungen gemäß **Formel I und Formel III** gilt, daß
R² und R³ unabhängig voneinander ausgewählt sind aus
H, C₁₋₄-Alkyl, gesättigt und unsubstituiert, verzweigt oder unverzweigt; vorzugsweise aus H, CH₃, C₂H₅, i-Propyl oder t-Butyl, insbesondere aus H oder CH₃ oder C₂H₅,

Es ist für das erfindungsgemäße Teilverfahren besonders bevorzugt, wenn für Verbindungen gemäß **Formel I und Formel III** gilt, daß
R³ = H und R² ≠ H,
vorzugsweise R³ =H und R² = CH₃ oder C₂H₅,
insbesondere R³ = H und R² = CH₃.

Es ist für das erfindungsgemäße Teilverfahren besonders bevorzugt, wenn für Verbindungen gemäß **Formel I und Formel III** gilt, daß
R² und R³ zusammen einen C₅-₆-Cycloalkylrest bilden, gesättigt oder ungesättigt, unsubstituiert oder ein- oder mehrfach substituiert, vorzugsweise gesättigt und unsubstituiert, insbesondere Cyclohexyl.

Es ist für das erfindungsgemäße Teilverfahren besonders bevorzugt, wenn für Verbindungen gemäß **Formel I und Formel III** gilt, daß
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise aus H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise aus OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise aus F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Es ist für das erfindungsgemäße Teilverfahren besonders bevorzugt, wenn für Verbindungen gemäß **Formel I und Formel III** gilt, daß
R¹ ausgewählt ist aus
C₁₋₃-Alkyl, gesättigt und unsubstituiert, verzweigt oder unverzweigt; vorzugsweise CH₃, C₂H₅ oder C₃H₇, insbesondere CH₃ oder C₂H₅,
**und/oder**
R² und R³ unabhängig voneinander ausgewählt sind aus
H, C₁₋₄-Alkyl, gesättigt und unsubstituiert, verzweigt oder unverzweigt; vorzugsweise aus H, CH₃, C₂H₅, i-Propyl oder t-Butyl, insbesondere aus H oder CH₃ oder C₂H₅,
vorzugsweise gilt:
R³ = H und R² ≠ H,
vorzugsweise R³ =H und R² = CH₃ oder C₂H₅,
insbesondere R³ = H und R² = CH₃.
oder
R² und R³ zusammen einen C₅-₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
**und/oder**
R⁴ ausgewählt ist aus H,
und/oder
R⁷ und R⁸ jeweils unabhängig voneinander ausgewählt sind aus
H oder CH₃,
vorzugsweise R⁷ und R⁸ H bedeuten oder R⁷ und R⁸ CH₃ bedeuten oder R⁷ H und R⁸ CH₃ bedeutet,
insbesondere R⁷ und R⁸ CH₃ bedeuten;
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise aus H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise aus OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise aus F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Es ist für das erfindungsgemäße Teilverfahren besonders bevorzugt, wenn für Verbindungen gemäß **Formel I** mit R³ = H und R² ≠ H gilt, daß diese in den Konfigurationen Ia oder Ib 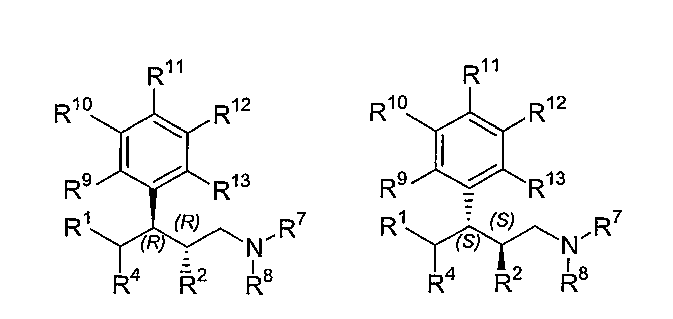 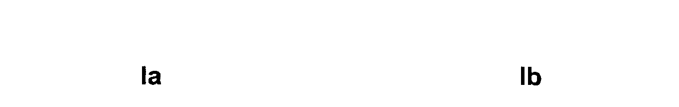 vorliegen.

Es ist für das erfindungsgemäße Teilverfahren besonders bevorzugt, wenn für Verbindungen gemäß **Formell III** mit R³ = H, R² ≠ H, R⁴ = H und R¹ ≠ H gilt, daß diese in den Konfigurationen IIIa oder IIIb vorliegen 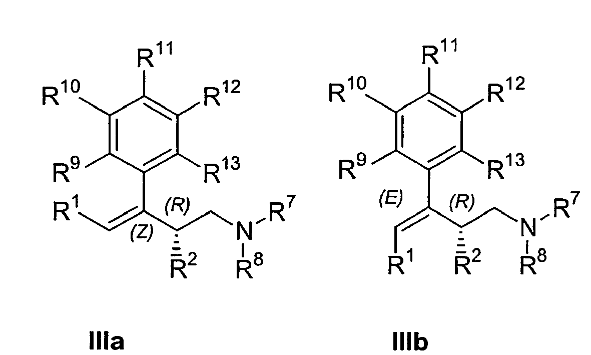 oder für Verbindungen gemäß **Formell III** mit R³ = H, R² ≠ H, R⁴ = H und R¹ ≠ H gilt, daß diese in den Konfigurationen IIIc oder IIId 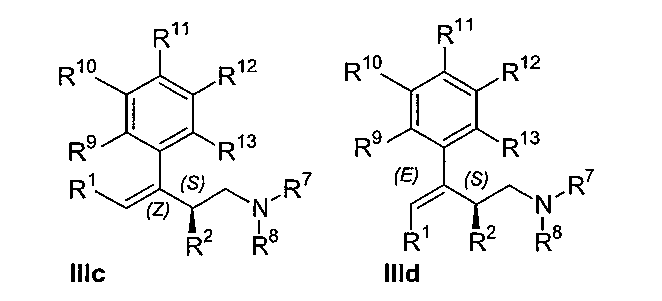 vorliegen.

Es ist für das erfindungsgemäße Teilverfahren besonders bevorzugt, wenn für die VerbindungNerbindungen gemäß **Formel I** gilt, daß mindestens eine davon, vorzugsweise als freie Base oder als Hydrochlorid, ausgewählt ist aus folgender Gruppe:
■ 3-(3-Dimethylamino-1-ethyl-2-methyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
■ (±)-(2RS,3RS)-[3-(3-Methoxy-phenyl)-2-methyl-pentyl]-dimethylamin,
■ *rac*(2RS,3RS)-[3-(3-Methoxy-phenyl)-2-methyl-pentyl]-dimethylamin,
■ 3{[3-(p-Isopropyl-phenyl-carbamoyl)-oxy-phenyl]-2-methylpentyl}-dimethylamin,
■ (2R,3R)-{3[3-(p-Isopropyl-phenyl-carbamoyl)-oxy-phenyl]-2-methyl-pentyl}-dimethylamin,
■ (2S,3S)-{3[3-(p-Isopropyl-phenyl-carbamoyl)-oxy-phenyl]-2-methyl-pentyl}-dimethylamin,
■ (2SR,3SR)-{3[3-(p-Isopropyl-phenyl-carbamoyl)-oxy-phenyl]-2-methyl-pentyl}-dimethylamin,
vorzugsweise
■ 3-(3-Dimethylamino-1-ethyl-2-methyl-propyl)-phenol,
■ (1R,2R)-3-(3-Dimethylamino-1-ethyl-2-methyl-propyl)-phenol,
■ (-)-(1 R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
insbesondere
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin.

Es ist für das erfindungsgemäße Teilverfahren besonders bevorzugt, wenn für die eingesetzte/n VerbindungNerbindungen gemäß **Formel III** gilt, daß mindestens eine davon, vorzugsweise als freie Base oder als Hydrochlorid, ausgewählt ist aus folgender Gruppe:
■ 3-(3-Dimethylamino-1-ethenyl-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl-2-methyl-propyl)-phenol,
■ (Z,E)-(2S)-3-(3-Dimethylamino-1-ethenyl-2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin.
■ {3[3-(p-Isopropyl-phenyl-carbamoyl)-oxy-phenyl]-2-methyl-pent-3-enyl}-dimethylamin,
■ (Z)-(2R)-{3[3-(p-Isopropyl-phenyl-carbamoyl)-oxy-phenyl]-2-methyl-pent-3-enyl}-dimethylamin,
■ (E)-(2R)-{3[3-(p-Isopropyl-phenyl-carbamoyl)-oxy-phenyl]-2-methyl-pent-3-enyl}-dimethylamin,
■ (Z,E)-(2R)-{3[3-(p-Isopropyl-phenyl-carbamoyl)-oxy-phenyl]-2-methyl-pent-3-enyl}-dimethylamin,
■ (Z)-(2S)-{3[3-(p-Isopropyl-phenyl-carbamoyl)-oxy-phenyl]-2-methyl-pent-3-enyl}-dimethylamin,
■ (E)-(2S)-{3[3-(p-Isopropyl-phenyl-carbamoyl)-oxy-phenyl]-2-methyl-pent-3-enyl}-dimethylamin,
■ (Z,E)-(2S)-{3[3-(p-Isopropyl-phenyl-carbamoyl)-oxy-phenyl]-2-methyl-pent-3-enyl}-dimethylamin,
, vorzugsweise
■ 3-(3-Dimethylamino-1-ethenyl-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl- 2-methyl-propyl)-phenol,
■ (Z,E)-(2S)-3-(3-Dimethylamino-1-ethenyl--2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin,
insbesondere
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin,
bevorzugt
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin oder
■ (Z,E)-(2R)-[3-(3-Methoxy-phenyl)-2-methyl-pent-3-enyl]-dimethylamin,
oder
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin oder
■ (Z,E)-(2S)-[3-(3-Methoxy-phenyl)-2-methyl-pent-3-enyl]-dimethylamin.

Es ist für das erfindungsgemäße Teilverfahren besonders bevorzugt, wenn bei der eingesetzten Verbindung gemäß Formel III an Position 2 gemäß Formel III ein chirales Zentrum vorliegt.

Es ist für das erfindungsgemäße Teilverfahren besonders bevorzugt, wenn bei der Verbindung gemäß Formel I an Position 2 gemäß Formel I ein chirales Zentrum vorliegt.

Es ist für das erfindungsgemäße Teilverfahren besonders bevorzugt, wenn die eingesetzte Verbindung nach Formel III enantiomerenrein ist.

Es ist für das erfindungsgemäße Teilverfahren besonders bevorzugt, wenn die eingesetzte Verbindung nach Formel III diastereomerenrein ist.

Es ist für das erfindungsgemäße Teilverfahren besonders bevorzugt, wenn die eingesetzte Verbindung nach Formel III enantiomeren- und diastereomerenrein ist.

Es ist für das erfindungsgemäße Teilverfahren besonders bevorzugt, wenn die eingesetzte Verbindung gemäß Formel III ausgewählt ist aus:
- (Z)-(2R)-[3-(3-Methoxy-phenyl)-2-methyl-pent-3-enyl]-dimethylamin,
- (E)-(2R)-[3-(3-Methoxy-phenyl)-2-methyl-pent-3-enyl]-dimethylamin
oder
eine Mischung von (Z)-(2R)-[3-(3-Methoxy-phenyl)-2-methyl-pent-3-enyl]-dimethylamin und (E)-(2R)-[3-(3-Methoxy-phenyl)-2-methyl-pent-3-enyl]-dimethylamin bzw. (Z,E)-(2R)-[3-(3-Methoxy-phenyl)-2-methyl-pent-3-enyl]-dimethylamin ist.

Es ist für das erfindungsgemäße Teilverfahren besonders bevorzugt, wenn die eingesetzte Verbindung gemäß Formel III ausgewählt ist aus:
• (Z)-(2S)-[3-(3-Methoxy-phenyl)-2-methyl-pent-3-enyl]-dimethylamin,
• (E)-(2S)-[3-(3-Methoxy-phenyl)-2-methyl-pent-3-enyl]-dimethylamin
oder
eine Mischung von (Z)-(2S)-[3-(3-Methoxy-phenyl)-2-methyl-pent-3-enyl]-dimethylamin und (E)-(2S)-[3-(3-Methoxy-phenyl)-2-methyl-pent-3-enyl]-dimethylamin, bzw. (Z,E)-(2S)-[3-(3-Methoxy-phenyl)-2-methyl-pent-3-enyl]-dimethylamin ist.

Es ist für das erfindungsgemäße Teilverfahren besonders bevorzugt, wenn das Lösungsmittel ausgewählt ist aus:
H₂O oder Alkohol oder wässrigen alkoholischen oder wässrigen sauren Lösungen, vorzugsweise aus wässrigen sauren Lösungen.

Es ist für das erfindungsgemäße Teilverfahren besonders bevorzugt, wenn das Lösungsmittel ausgewählt ist aus:
H₂O oder Ethanol oder wässriger ethanolischer Lösung oder wässriger Salzsäure, vorzugsweise aus wässriger Salzsäure.

Es ist für das erfindungsgemäße Teilverfahren besonders bevorzugt, wenn der verwendete Katalysator ein Edelmetall enthält, vorzugsweise Platin, Gold oder Palladium, insbesondere Palladium.

Es ist für das erfindungsgemäße Teilverfahren besonders bevorzugt, wenn der verwendete Katalysator Palladium auf Aktivkohle oder Palladium(II)-Chlorid ist.

Es ist für das erfindungsgemäße Teilverfahren besonders bevorzugt, wenn der verwendete Katalysator Palladium auf Aktivkohle (1 - 10 Gew.-%, bevorzugt 5 Gew.-%) ist.

Es ist für das erfindungsgemäße Teilverfahren besonders bevorzugt, wenn die Temperatur zwischen 20 und 40°C, vorzugsweise zwischen 20 und 35, insbesondere 25°C gehalten wird.

Es ist für das erfindungsgemäße Verfahren bevorzugt, wenn in Schritt b) vor der Hydrierung eine Schutzgasatmosphäre, insbesondere ein Stickstoffschutzgas, angelegt wird.

Es ist für das erfindungsgemäße Teilverfahren besonders bevorzugt, wenn die Hydrierung bei einem Wasserstoffvordruck von 3-10bar, vorzugsweise 4-7 bar, insbesondere 5 bar, stattfindet
und/ oder
der Hydrierungsschritt bei einem Wasserstoffinnendruck von 0,5-3 bar, vorzugsweise 0,75-2 bar, insbesondere 1 bar, stattfindet.

Es ist für das erfindungsgemäße Teilverfahren besonders bevorzugt, wenn zu Beginn die Ausgangsprodukte im Lösungsmittel stark verdünnt sind/werden.

Es ist für das erfindungsgemäße Teilverfahren besonders bevorzugt, wenn die eingesetzte Verbindung gemäß Formel III in wässriger Säure gelöst wird.

Es ist für das erfindungsgemäße Teilverfahren besonders bevorzugt, wenn die eingesetzte Verbindung gemäß Formel III in wässriger Salzsäure gelöst wird.

Sowohl für das erfindungsgemäße Verfahren wie für das erfindungsgemäße Teilverfahren ist es besonders bevorzugt, wenn am Ende des Verfahren die Produkte in organischem Lösungsmittel gefällt werden.

Sowohl für das erfindungsgemäße Verfahren wie für das erfindungsgemäße Teilverfahren ist es besonders bevorzugt, wenn am Ende des Verfahren die Produkte mit Säure bzw. Säuregas, vorzugsweise Salzsäure oder Salzsäuregas, insbesondere Salzsäuregas, gefällt werden.

Sowohl für das erfindungsgemäße Verfahren wie für das erfindungsgemäße Teilverfahren ist es besonders bevorzugt, wenn am Ende des Verfahren die Produkte in organischem Lösungsmittel mit Säure bzw. Säuregas, vorzugsweise Salzsäure oder Salzsäuregas, insbesondere Salzsäuregas, gefällt werden.

### Allgemeines:

### Der Eliminierungsschritt (Schritt a) des erfindungsgemäßen Verfahrens

Es zeigte sich, daß in Schritt a) des erfindungsgemäßen Verfahrens Ameisensäure, Salzsäure und Bromwasserstoffsäure sehr gut geeignet sind, die tertiäre OH-Gruppe zu eliminieren und hohe Ausbeuten zu erreichen.

Ist der OH-Gruppe ein chirales Zentrum benachbart, ist es erforderlich die Eliminierung regioselektiv durchzuführen, um zu verhindern, daß die chirale Information verloren geht. Durch den Einsatz von Ameisensäure, Salzsäure und Bromwasserstoffsäure gelingt dies erstaunlicherweise sehr gut. Insbesondere wird in diesem Verfahren der Einsatz der preiswerten Salzsäure bevorzugt, die nach Ende der Reaktion durch Neutralisation in Natriumchlorid umgewandelt werden kann. Durch Änderung der Reaktionszeit, Reaktionstemperatur und Konzentration der Säure kann die Regioselektivität der Eliminierung weiter positiv beeinflußt werden. Hohe Konzentration der Säure führen vermehrt zu den gewünschten Verbindungen. Gut geeignete Reaktionsbedingungen sind 36%ige Salzsäure bei 5 Stunden Reaktionszeit und einer Temperatur von 55°C.

Durch eine Kristallisation der eliminierten Verbindungen mit Salzsäuregas in Lösungsmitteln werden in guten Ausbeuten die Z-Isomeren erhalten. Geringe Mengen an den für dieses Verfahren nicht gewünschten (Z,E)-Dimethyl-(3-aryl-pent-2-enyl)-aminverbindungen bleiben in Lösung oder können durch Umkristallisation abgereichert werden.

### Der Hydrierungsschritt (Schritt b) im erfindungsgemäßen Verfahren und erfindungsgemäßes Teilverfahren

Dieses Teilverfahren bzw. Schritt b) ist für Verbindungen interessant, die in Nachbarschaft zur OH-Gruppe ein chirales Zentrum besitzen.

Wie zuvor im Eliminierungsschritt beschrieben ist es möglich die Eliminierung so zu steuern, daß das chirale Zentrum nur in geringem Maße an der Eliminierung beteiligt ist. Durch die Kristallisation der eliminierten Verbindungen werden die (Z,E)-Dimethyl-(3-aryl-pent-2-enyl)-aminverbindungen abgereichert, so daß nach der Hydrierung keine Racemisierung am Nachbar C-Atom zur OH-Gruppe auftreten kann.
Erstaunlicherweise sind die (Z,E)-Dimethyl-(3-aryl-pent-2-enyl)-aminverbindungen unter den in diesem Verfahren beschriebenen Hydrierbedingungen an der Doppelbindung nicht hydrierbar, vielmehr kommt es in einer ersten Reaktion zu einer einem Verlust der Dimethyaminogruppe mit Folgehydrierungen.
Aus diesem Grund ist es möglich, die eliminierten Produkte ohne Reinigung in die Hydrierung einzusetzen. Restmengen von (Z,E)-Dimethyl-(3-aryl-pent-2-enyl)-aminverbindungen die in den Eliminierungsrohprodukten enthalten sind, werden während der Eliminierung einer Dimethylaminabspaltung unterzogen.
Bei der Fällung der hydrierten Verbindungen mit Salzsäuregas in organischen Lösungsmitteln können die deaminierten Verbindungen keine Salze bilden und bleiben daher in der organischen Mutterlauge gelöst.
Dadurch kann erstaunlicherweise auch dann keine Racemisierung eintreten, selbst wenn die Ausgangsprodukte für den Hydrierschritt noch Reste von (Z,E)-Dimethyl-(3-aryl-pent-2-enyl)-aminverbindungen enthalten.

Die erste Hydrierung wurde in Ethanol unter Zusatz von Palladium/C 10% durchgeführt und erstaunlicherweise ein Diastereomerenverhältnis von 70:30 erhalten zugunsten der in diesem Verfahren gewünschten Diastereomeren, der (R,R)-(3-Aryl-2-methyl-pentyl)-aminen.
Es wurde herausgefunden, daß bei großer Verdünnung der Ausgangsstoffe im Lösungsmittel der Anteil des gewünschten Diastereomeren bis auf 90% weiter zunimmt.
Erstaunlicherweise läßt sich durch langsame Zugabe der Doppelbindungskomponente in das vorgelegte Lösungsmittel mit Katalysator und Wasserstoff eine Diastereomerenanreicherung von 75% erreichen.
Ein Zusatz von katalytischen Mengen Salzsäure erbringt auch bei geringerer Verdünnung ein Anwachsen des gewünschten Diastereomeren auf 85%.
Die Kombination aus Verdünnung und Ansäuern mit wässriger Salzsäure erbringt ein Anwachsen des gewünschte Diastereomeren auf 90%.

Neben Palladium kann auch Palladiumchlorid eingesetzt werden. Auch hier wird in guter Ausbeute das gewünschte Produkt mit einem Diastereomerenüberschuß von 70% erhalten. Dieses Verfahren hat den großen Vorteil, daß das anfallende Palladium nach der Hydrierung wieder in Salpetersäure aufgelöst werden kann und fast ohne Verlust in die nächste Hydrierung eingesetzt werden kann.

### Kombination der beiden Verfahren (erfindungsgemäßes Verfahren)

Insbesondere erstaunlich und erfreulich war es, dass Eliminierung und Hydrierung in einem Eintopfverfahren durchgeführt werden kann.

Erstaunlicherweise erbrachten Untersuchungen, daß das Z,E-Verhältnis der (Z,E)-(2RS)-Dimethyl-(3-aryl-2-methyl-pent-3-enyl)-aminverbindungen Verbindungen keinen Einfluß auf das Diastereomerenverhältnis der hydrierten Endprodukte hat. Daher war es nicht erforderlich durch Kristallisation das eliminierte reine Z-Produkt zu isolieren.
Zuerst wurde die Eliminierung in wässriger Salzsäure durchgeführt, anschließend der Palladiumkatalysator zugesetzt und dann hydriert. Man erhält das gewünschte (R,R)-Diastereomere mit 73%.

Im folgenden wird die Erfindung anhand von Beispielen erläutert. Diese Erläuterungen sind lediglich beispielhaft und schränken den allgemeinen Erfindungsgedanken nicht ein.

### BEISPIELE

### Beispiel 1:

In einer 100I Doppelmantelreaktionsanlage mit elektrischem Ankerrüher, Pt100 Temperaturmeßeinrichtung und ölbasierendem Kühl/Heizsystem wurden 15kg (59,7 mol)
(2S,3S)-1-Dimethylamino-3-(3-methoxy-phenyl)-2-methyl-pentan-3-ol bei 20°C und einer Rührdrehzahl von 100 U/min vorgelegt und innerhalb von 10 min mit 26,25 I 36 Gew.-%iger (308 mol) wässriger Salzsäure versetzt. Die Reaktionsmischung wird auf 50°C innerhalb von 20 min erwärmt und bei dieser Temperatur 4-6 Stunden gerührt. Danach wird der Ansatz auf 25°C abgekühlt und mit 13I Wasser verdünnt. Unter Kühlung mit einer Manteltemperatur von 5°C wurden bei einer Innentemperatur von 20°C ca. 32 I 32 Gew.-%ige (256mol) Natronlauge zugegeben bis ein pH - Wert von
10 - 12,5 erreicht wird. Danach wurden 22,5 I Essigsäureethyester zugegeben und nach 10 min unter Rühren der Rührer zur Phasentrennung abgeschaltet. Die untere wässrige Phase wurde abgelassen und die obere organische Phase bei einer maximalen Innentemperatur von 50°C im Vakuum bis 10mbar abdestilliert. Der verbleibende hellgelbe ölige Rückstand ist das gewünschte (Z,E)-(2R)- [3-(3-Methoxy-phenyl)-2-methyl-pent-3-enyl]-dimethyl-amin. Die Ausbeute beträgt 13,6 kg (98% der Theorie) mit einer HPLC Reinheit von 90% und einem Z/E Verhältnis von 70:30.

### Beispiel 2:

In einer 100I Doppelmantelreaktionsanlage mit elektrischem Ankerrüher, Pt100 Temperaturmeßeinrichtung und ölbasierendem Kühl/Heizsystem wurden 15kg (52,15 mol) (2S,3S)-1-Dimethylamino-3-(3-methoxy-phenyl)-2-methyl-pentan-3-ol hydrochlorid bei 20°C und einer Rührdrehzahl von 100 U/min vorgelegt und innerhalb von 10 min mit 26,25 I 36 Gew.-%iger (308mol) wässriger Salzsäure versetzt. Die Reaktionsmischung wird auf 50°C innerhalb von 20 min erwärmt und bei dieser Temperatur 4-6 Stunden gerührt. Danach wird der Ansatz auf 25°C abgekühlt und mit 13I Wasser verdünnt. Unter Kühlung mit einer Manteltemperatur von 5°C wurden bei einer Innentemperatur von 20°C ca. 32 I 32 Gew.-%ige (256 mol) Natronlauge zugegeben bis ein pH - Wert von 10 - 12,5 erreicht wurde. Danach wurden 22,5 I Essigsäureethyester zugegeben und nach 10 min unter Rühren der Rührer zur Phasentrennung abgeschaltet. Die untere wässrige Phase wurde abgelassen und die obere organische Phase bei einer maximalen Innentemperatur von 50°C im Vakuum bis 10mbar abdestilliert. Der verbleibende hellgelbe ölige Rückstand ist das gewünschte (Z,E)-(2R)- [3-(3-Methoxy-phenyl)-2-methyl-pent-3-enyl]-dimethyl-amin. Die Ausbeute beträgt 11,9 kg (98% der Theorie) (54,4 mol) mit einer HPLC Reinheit von 90% und einem Z/E Verhältnis von 70:30.

### Beispiel 3:

In einer 100I Doppelmantelreaktionsanlage mit elektrischem Ankerrüher, Pt100 Temperaturmeßeinrichtung und ölbasierendem Kühl/Heizsystem wurden 15kg (59,68 mol) einer 70:30 Mischung aus (2S,3S)-1-Dimethylamino-3-(3-methoxyphenyl)-2-methyl-pentan-3-ol und (2S,3R)-1-Dimethylamino-3-(3-methoxy-phenyl)-2-methyl-pentan-3-ol
bei 20°C und einer Rührdrehzahl von 100 U/min vorgelegt und innerhalb von 10 min mit 26,25 I 36 Gew.-%iger (307,9 mol) wässriger Salzsäure versetzt. Die Reaktionsmischung wird auf 50°C innerhalb von 20 min erwärmt und bei dieser Temperatur 4-6 Stunden gerührt. Danach wird der Ansatz auf 25°C abgekühlt und mit 13I Wasser verdünnt. Unter Kühlung mit einer Manteltemperatur von 5°C wurden bei einer Innentemperatur von 20°C ca. 32 I 32 Gew.-%ige Natronlauge (256 mol) zugegeben bis ein pH - Wert von 10 - 12,5 erreicht wurde. Danach wurden 22,5 I Essigsäureethyester zugegeben und nach 10 min unter Rühren der Rührer zur Phasentrennung abgeschaltet. Die untere wässrige Phase wurde abgelassen und die obere organische Phase bei einer maximalen Innentemperatur von 50°C im Vakuum bis 10mbar abdestilliert. Der verbleibende hellgelbe ölige Rückstand ist das gewünschte (Z,E)-(2R)- [3-(3-Methoxy-phenyl)-2-methyl-pent-3-enyl]-dimethyl-amin. Die Ausbeute beträgt 13,6 kg (58,3 mol) (98% der Theorie) mit einer HPLC Reinheit von 90% und einem Z/E Verhältnis von 70:30.

### Beispiel 4:

In einer 100I Doppelmantelreaktionsanlage mit elektrischem Ankerrüher, Pt100 Temperaturmeßeinrichtung und ölbasierendem Kühl/Heizsystem wurden 15kg (59,68 mol) einer Mischung aus (2S,3S)-1-Dimethylamino-3-(3-methoxy-phenyl)-2-methyl-pentan-3-ol (35 Gew.-%), (2R,3R)-1-Dimethylamino-3-(3-methoxy-phenyl)-2-methyl-pentan-3-ol (35 Gew.-%), (2R,3S)-1-Dimethylamino-3-(3-methoxy-phenyl)-2-methyl-pentan-3-ol (15 Gew.-%) und (2S,3R) -1-Dimethylamino-3-(3-methoxyphenyl)-2-methyl-pentan-3-ol(15 Gew-%) bei 20°C und einer Rührdrehzahl von 100 U/min vorgelegt und innerhalb von 10 min mit 26,25 I 36 Gew.-%iger wässriger Salzsäure versetzt. Die Reaktionsmischung wird auf 50°C innerhalb von 20 min erwärmt und bei dieser Temperatur 4-6 Stunden gerührt. Danach wird der Ansatz auf 25°C abgekühlt und mit 13I Wasser verdünnt. Unter Kühlung mit einer Manteltemperatur von 5°C wurden bei einer Innentemperatur von 20°C ca. 32 I 32 Gew.-%ige Natronlauge zugegeben bis ein pH - Wert von 10 - 12,5 erreicht wurde. Danach wurden 22,5 I Essigsäureethyester zugegeben und nach 10 min unter Rühren der Rührer zur Phasentrennung abgeschaltet. Die untere wässrige Phase wurde abgelassen und die obere organische Phase bei einer maximalen Innentemperatur von 50°C im Vakuum bis 10mbar abdestilliert. Der verbleibende hellgelbe ölige Rückstand ist die gewünschte Mischung aus (Z,E)-(2R)- [3-(3-Methoxy-phenyl)-2-methyl-pent-3-enyl]-dimethyl-amin und (Z,E)-(2S)- [3-(3-Methoxyphenyl)-2-methyl-pent-3-enyl]-dimethyl-amin. Die Ausbeute beträgt 13,6 kg (98% der Theorie) mit einer HPLC Reinheit von 90% und einem Z/E Verhältnis von 70:30.

### Beispiel 5:

In einem 250 ml Dreihalskolben mit Thermometer, mechanischem Druckluftrührer, Rückflußkühler und Ölbadheizung wurden 28,7g (0,1 mol) (2S,3S)-1-Dimethylamino-3-(3-methoxy-phenyl)-2-methyl-pentan-3-ol hydrochlorid vorgelegt und mit 150 ml Ameisensäure versetzt. Es wurde 4 Stunden am Rückfluß zum Sieden erhitzt. Die Mischung wurde abgekühlt, in einen 500ml Rundkolben gegossen und an einem Büchi 5I Rotationsverdampfer bei 60°C bis zu einem Druck von 10mbar die Ameisensäure abdestilliert. Der ölige Rückstand wurde mit 150ml Essigsäureethlester und 100ml Wasser versetzt. Mit 33% Gew.-iger Natronlauge wurde ein pH-Wert von 11 eingestellt, die Phasen getrennt und der Essigsäureethylester am Rotationsverdampfer bei 60°C bis zu einem Druck von 10 mbar abdestilliert. Der ölige Rückstand besteht aus (Z,E)-(2R)- [3-(3-Methoxyphenyl)-2-methyl-pent-3-enyl]-dimethyl-amin mit einer GC-Reinheit von 92% , einem Z/E-Verhältnis von 2,2:1 und einer Ausbeute von 21,0 g (90% der Theorie). Bei der Reinheitsuntersuchung wurden noch 0,37% nicht umgesetztes Ausgangsprodukt und 2,01 % des (Z,E)-[3-(3-Methoxy-phenyl)-2-methyl-pent-2-enyl]-dimethyl - aminhydrochlorids gefunden.

### Beispiel 6:

In einem 250 ml Dreihalskolben mit Thermometer, mechanischem Druckluftrührer, Rückflußkühler und Ölbadheizung wurden 28,7g (0,1mol) (2S,3S)-1-Dimethylamino-3-(3-methoxy-phenyl)-2-methyl-pentan-3-ol hydrochlorid vorgelegt und mit 75 ml Bromwasserstoffsäure 47 Gew.-%ig versetzt. Es wurde 1 Stunde auf 50°C erwärmt. Die Mischung wurde auf 20°C abgekühlt und bei 20°C unter Kühlung mit 33 Gew.-%iger Natronlauge einen pH-Wert von 11 eingestellt. Es wurde 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (Z,E)-(2R)- [3-(3-Methoxy-phenyl)-2-methyl-pent-3-enyl]-dimethyl-amin mit einer GC-Reinheit von 93% , einem Z/E-Verhältnis von 4:1 und einer Ausbeute von 21g (90% der Theorie). Bei der Reinheitsuntersuchung wurden noch 1,52% nicht umgesetztes Ausgangsprodukt und 2,1% des (Z,E)-[3-(3-Methoxy-phenyl)-2-methyl-pent-2-enyl]-dimethyl -aminhydrochlorids gefunden

### Beispiel 7:

In einem 250 ml Dreihalskolben mit Thermometer, mechanischem Druckluftrührer, Rückflußkühler und Ölbadheizung wurden 28,7g (0,1mol) (2R,3R)-1-Dimethylamino-3-(3-methoxy-phenyl)-2-methyl-pentan-3-ol hydrochlorid vorgelegt und mit 75 ml Bromwasserstoffsäure 47 Gew.-%ig versetzt. Es wurde 4 Stunden auf 35°C erwärmt. Die Mischung wurde auf 20°C abgekühlt und bei 20°C unter Kühlung mit 33 Gew.-%iger Natronlauge einen pH-Wert von 11 eingestellt. Es wurde 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (Z,E)-(2S)-[3-(3-Methoxy-phenyl)-2-methyl-pent-3-enyl]-dimethyl-amin mit einer GC-Reinheit von 90,5% , einem Z/E-Verhältnis von 2,9:1 und einer Ausbeute von 21 g (90% der Theorie). Bei der Reinheitsuntersuchung wurden noch 4,92% nicht umgesetztes Ausgangsprodukt und 1,5% des (Z,E)-[3-(3-Methoxy-phenyl)-2-methyl-pent-2-enyl]-dimethyl -aminhydrochlorids gefunden.

### Beispiel 8:

In einem 250 ml Dreihalskolben mit Thermometer, mechanischem Druckluftrührer, Rückflußkühler und Ölbadheizung wurden 28,7g (0,1mol) (2R,3R)-1-Dimethylamino-3-(3-methoxy-phenyl)-2-methyl-pentan-3-ol hydrochlorid vorgelegt und mit 75 ml Bromwasserstoffsäure 47 Gew.-%ig versetzt. Es wurde 4 Stunden auf 35°C erwärmt. Die Mischung wurde auf 20°C abgekühlt und bei 20°C unter Kühlung mit 33 Gew.-%iger Natronlauge einen pH-Wert von 11 eingestellt. Es wurde 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (Z,E)-(2S)-[3-(3-Methoxy-phenyl)-2-methyl-pent-3-enyl]-dimethyl-amin mit einer GC-Reinheit von 90,5% , einem Z/E-Verhältnis von 2,9:1 und einer Ausbeute von 21g (90% der Theorie). Bei der Reinheitsuntersuchung wurden noch 4,92% nicht umgesetztes Ausgangsprodukt und 1,5% des (Z,E)-[3-(3-Methoxy-phenyl)-2-methyl-pent-2-enyl]-dimethyl -aminhydrochlorids gefunden.

### Beispiel 9:

In einem 250 ml Dreihalskolben mit Thermometer, mechanischem Druckluftrührer, Rückflußkühler und Ölbadheizung wurden 28,7g (0,1mol) (2S,3R)-1-Dimethylamino-3-(3-methoxy-phenyl)-2-methyl-pentan-3-ol hydrochlorid vorgelegt und mit 150 ml wässriger 36 Gew.-%iger Salzsäure versetzt. Es wurde 19h Stunden auf 55°C erwärmt. Die Mischung wurde auf 20°C abgekühlt und bei 20°C unter Kühlung mit 33 Gew.-%iger Natronlauge einen pH-Wert von 11 eingestellt. Es wurde 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (Z,E)-(2R)-[3-(3-Methoxy-phenyl)-2-methyl-pent-3-enyl]-dimethyl-amin mit einer GC-Reinheit von 40% , einem Z/E-Verhältnis von 3,5:1 und einer Ausbeute von 21 g (90% der Theorie). Bei der Reinheitsuntersuchung wurde kein Ausgangsprodukt und 40% des (Z,E)-[3-(3-Methoxy-phenyl)-2-methyl-pent-2-enyl]-dimethyl -amins gefunden.

### Beispiel 10:

In einem 250 ml Dreihalskolben mit Thermometer, mechanischem Druckluftrührer, Rückflußkühler und Ölbadheizung wurden 28,7g (0,1mol) (2S,3R)-1-Dimethylamino-3-(3-methoxy-phenyl)-2-methyl-pentan-3-ol hydrochlorid vorgelegt und mit 150 ml wässriger 36 Gew.-%iger Salzsäure versetzt. Es wurde 1 h Stunde auf 100°C erwärmt. Die Mischung wurde auf 20°C abgekühlt und bei 20°C unter Kühlung mit 33 Gew.-%iger Natronlauge einen pH-Wert von 11 eingestellt. Es wurde 150ml Essigsäureethylester zugesetzt, 10 min gerührt, der Rührer abgestellt, die Phasen getrennt und der Essigsäureethylester am Rotationsverdampfer bei 60°C bis zu einem Druck von 10 mbar abdestilliert. Der ölige Rückstand besteht aus (Z,E)-(2R)- [3-(3-Methoxy-phenyl)-2-methyl-pent-3-enyl]-dimethyl-amin mit einer GC-Reinheit von 86% , einem Z/E-Verhältnis von 6,5:1 und einer Ausbeute von 21 g (90% der Theorie). Bei der Reinheitsuntersuchung wurde kein Ausgangsprodukt und 8,5% des (Z,E)-[3-(3-Methoxy-phenyl)-2-methyl-pent-2-enyl]-dimethyl-amins gefunden.

### Beispiel 11

In einer kühl- und heizbaren 50 L Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 10 kg (42,85 mol) (Z,E)-(2R)- [3-(3-Methoxyphenyl)-2-methyl-pent-3-enyl]-dimethyl-amin bei 25° C und einer Rührerdrehzahl von 850 ±150 U/Min in 25 L Ethanol abs. verg. gelöst. Die Reaktionsanlage wurde mit Stickstoff inertisiert. Unter Stickstoffschutzgas wurde die Lösung mit einer Suspension von 750 g Palladium auf Aktivkohle (5 Gew.-%) in 5I Ethanol versetzt. Nach erneutem Inertisieren wurde mit einem Wasserstoffvordruck von 5 bar und einem Innendruck von 1 bar hydriert bis die Wasserstoffaufnahme beendet war. Nach beendeter Reaktion wurde die Anlage erneut mit Stickstoff inertisiert und der Reaktionsansatz über einen Einschichtenfilter, belegt mit Filtererde, filtriert um den Katalysator zu entfernen. Das klare Filtrat wurde am Rotationsverdampfer unter kontinuierlich reduziertem Druck bis zur Massenkonstanz eingeengt. Das verbleibende klare Öl ist ein Gemisch aus dem gewünschten (2R,3R)-[3-(3-Methoxyphenyl)-2-methyl-pentyl]dimethylamin und (2R,3S)-[ 3-(3-Methoxy-phenyl)-2-methylpentyl]dimethylamin. Die Ausbeute beträgt 9,96 kg (42,3 mol) (99 % der Theorie) mit einer GC-Reinheit von 90 %. Das Diastereomerenverhältnis (R,R Enantiomer zu R,S Enantiomer) beträgt 2,8:1.

### Beispiel 12

In einer kühl- und heizbaren 501 Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 0,8 kg (3,43 mol) (Z,E)-(2R)- [3-(3-Methoxyphenyl)-2-methyl-pent-3-enyl]-dimethyl-amin bei 25° C und einer Rührerdrehzahl von 850 ±150 U/Min in 251 Ethanol abs. verg. gelöst. Die Reaktionsanlage wurde mit Stickstoff inertisiert. Unter Stickstoffschutzgas wurde die Lösung mit einer Suspension von 60 g Palladium auf Aktivkohle (5 Gew.-%) in 51 Ethanol versetzt. Nach erneutem Inertisieren wurde mit einem Wasserstoffvordruck von 5 bar und einem Innendruck von 1 bar hydriert bis die Wasserstoffaufnahme beendet ist. Nach beendeter Reaktion wurde die Anlage erneut mit Stickstoff inertisiert und der Reaktionsansatz über einen Einschichtenfilter, belegt mit Filtererde, filtriert um den Katalysator zu entfernen. Das klare Filtrat wurde am Rotationsverdampfer unter kontinuierlich reduziertem Druck bis zur Massenkonstanz eingeengt. Das verbleibende klare Öl war ein Gemisch aus dem gewünschten (2R,3R)-[3-(3-Methoxy-phenyl)-2-methyl-pentyl]dimethylamin und (2R,3S)-[ 3-(3-Methoxy-phenyl)-2-methyl-pentyl]dimethylamin. Die Ausbeute beträgt 0,80 kg (99 % der Theorie) mit einer GC-Reinheit von 94 %. Das Diastereomerenverhältnis (R,R Enantiomer zu R,S Enantiomer) beträgt 5,9:1.

### Beispiel 13:

In einer kühl- und heizbaren 501 Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 5 kg (21,43 mol) (Z,E)-(2R)- [3-(3-Methoxyphenyl)-2-methyl-pent-3-enyl]-dimethyl-amin bei 25° C und einer Rührerdrehzahl von 850 ±150 U/Min in 131 Ethanol abs. verg. gelöst. Die Reaktionsanlage wurde mit Stickstoff inertisiert.
Unter Stickstoffschutzgas wurden 375 g Palladium auf Aktivkohle (5 Gew.-%) in 0,675 kg 32 Gew.-%iger Salzsäure aufgeschlämmt. Die Katalysatorsuspension wurde unter Rühren zu der Reaktionslösung zugegeben. Nach erneutem Inertisieren wurde mit einem Wasserstoffvordruck von 5 bar und einem Innendruck von 1 bar hydriert bis die Wasserstoffaufnahme beendet ist.
Nach beendeter Reaktion wurde die Anlage mit Stickstoff inertisiert und der Reaktionsansatz über einen Einschichtenfilter, belegt mit Filtererde, filtriert um den Katalysator zu entfernen. Das schwach trübe Filtrat wurde am Rotationsverdampfer unter kontinuierlich reduziertem Druck bis zur Massenkonstanz eingeengt. Die verbleibende weisse Feststoffsuspension wurde in 10I Ethylacetat aufgenommen und bei 20° C mit 3,7110 Gew.-% iger Natronlauge versetzt und ein pH von 10 - 12 eingestellt. Die untere, wässrige Phase wurde abgetrennt und verworfen. Die obere, organische Phase wurde im Rotationsverdampfer bei 45 - 50° C und koninuierlich reduziertem Druck bis zur Massenkonstanz eingeengt. Das verbleibende klare Öl ist ein Gemisch aus dem gewünschten (2R,3R)-[3-(3-Methoxy-phenyl)-2-methylpentyl]dimethylamin und (2R,3S)-[ 3-(3-Methoxy-phenyl)-2-methylpentyl]dimethylamin. Die Ausbeute beträgt 4,5 kg (90 % der Theorie) mit einer GC-Reinheit von 90 %. Das Diastereomerenverhältnis (R,R Enantiomer zu R,S Enantiomer) beträgt 5,5:1 nach Isolierung der Base.

### Beispiel 14

In einer kühl- und heizbaren 50I Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 5 kg (21,43 mol) (Z,E)-(2R)- [3-(3-Methoxyphenyl)-2-methyl-pent-3-enyl]-dimethyl-amin bei 25° C und einer Rührerdrehzahl von 850 ±150 U/Min in 12,5I Ethanol abs. verg. gelöst. Die Reaktionsanlage wurde mit Stickstoff inertisiert. Unter Stickstoffschutzgas wurde die Lösung mit einer Suspension von 1,87 kg Palladium auf Aktivkohle (1Gew.-%) in 2,5I Ethanol und 630 g Wasser versetzt. Nach erneutem Inertisieren wurde mit einem Wasserstoffvordruck von 5 bar und einem Innendruck von 1 bar hydriert bis die Wasserstoffaufnahme beendet ist.
Nach beendeter Reaktion wurde die Anlage mit Stickstoff inertisiert und der Reaktionsansatz über einen Einschichtenfilter, belegt mit Filtererde, filtriert um den Katalysator zu entfernen. Das klare Filtrat wurde am Rotationsverdampfer unter kontinuierlich reduziertem Druck bis zur Massenkonstanz eingeengt. Das verbleibende klare Öl ist eine Gemisch aus dem gewünschten (-)(2R, 3R)-[3-(3-Methoxy-phenyl)-2-methyl-pentyl]dimethylamin und (2R,3S)-[ 3-(3-Methoxy-phenyl)-2-methyl-pentyl]dimethylamin. Die Ausbeute beträgt 4,90 kg (98 % der Theorie) mit einer GC-Reinheit von 89 %. Das Diastereomerenverhältnis (R,R Enantiomer zu R,S Enantiomer) beträgt 2,7:1 nach Isolierung der Base.

### Beispiel 15

In einer kühl- und heizbaren 50I Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 5kg (21,43 mol) (Z,E)-(2R)- [3-(3-Methoxy-phenyl)-2-methyl-pent-3-enyl]-dimethyl-amin bei 25° C und einer Rührerdrehzahl von 850 ±150 U/Min in 12,51 Ethanol abs. verg. gelöst. Die Reaktionsanlage wurde mit Stickstoff inertisiert. Unter Stickstoffschutzgas wurde die Lösung mit einer Suspension von 0,19kg Palladium auf Aktivkohle (10 Gew.-%) in 2,51 Ethanol und 630g Wasser versetzt. Nach erneutem Inertisieren wurde mit einem Wasserstoffvordruck von 5 bar und einem Innendruck von 1 bar hydriert bis die Wasserstoffaufnahme beendet ist. Nach beendeter Reaktion wurde die Anlage mit Stickstoff inertisiert und der Reaktionsansatz über einen Einschichtenfilter, belegt mit Filtererde, filtriert um den Katalysator zu entfernen. Das klare Filtrat wurde am Rotationsverdampfer unter kontinuierlich reduziertem Druck bis zur Massenkonstanz eingeengt. Das verbleibende klare Öl ist eine Gemisch aus dem gewünschten (2R,3R)-[3-(3-Methoxy-phenyl)-2-methyl-pentyl]dimethylamin und (2R,3S)-[ 3-(3-Methoxy-phenyl)-2-methyl-pentyl]dimethylamin. Die Ausbeute beträgt 4,90 kg (98 % der Theorie) mit einer GC-Reinheit von 87 %. Das Diastereomerenverhältnis (R,R Enantiomer zu R,S Enantiomer) beträgt 3,0:1 nach Isolierung der Base.

### Beispiel 16

In einer 100I Doppelmantelreaktionsanlage mit elektrischem Ankerrüher, Pt100 Temperaturmeßeinrichtung und ölbasierendem Kühl/Heizsystem wurden 5,76kg (22,9 mol) (2S,3R)-1-Dimethylamino-3-(3-methoxy-phenyl)-2-methyl-pentan-3-ol bei 20°C und einer Rührdrehzahl von 100 U/min vorgelegt und innerhalb von 10 min mit 12,22I 36 Gew.-%iger wässriger Salzsäure versetzt. Die Reaktionsmischung wird auf 70°C innerhalb von 30 min erwärmt und bei dieser Temperatur 1 Stunde gerührt.
Anschiessend wurde die Lösung auf 20° C abgekühlt und mit 10I 25 Gew.-% Natronlauge und 5kg NaCl versetzt. Es bildet sich eine weisse Suspension. Die Suspension wird in die Hydrierapparatur überführt.
In einer kühl- und heizbaren 501 Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurde zu der Suspension unter Stickstoffschutzgas eine Suspension von 0,230kg Palladium auf Aktivkohle (1 Gew.-%) in 2,5I Wasser zugegeben und bei 25° C und einer Rührerdrehzahl von 850 ±150 U/Min vermischt. Die Reaktionsanlage wurde mit Stickstoff inertisiert. Dann wurde mit einem Wasserstoffvordruck von 5 bar und einem Innendruck von 1 bar hydriert bis die Wasserstoffaufnahme beendet war.

Nach beendeter Reaktion wurde die Anlage mit Stickstoff inertisiert und der Reaktionsansatz über einen Einschichtenfilter, belegt mit Filtererde, filtriert um den Katalysator zu entfernen. Das klare Filtrat wurde mit 18I 32 Gew.-%iger Natronlauge versetzt und ein pH-Wert von 11 - 12 eingestellt, wobei ein Niederschlag auftrat. Es wurde tert. Butylmethylether hinzugegeben eine Phasentrennung durchgeführt. Die organische Phase wurde am Rotationsverdampfer unter kontinuierlich reduziertem Druck bis zur Massenkonstanz eingeengt. Das verbleibende klare Öl ist eine Gemisch aus dem gewünschten (2R,3R)-[3-(3-Methoxy-phenyl)-2-methylpentyl]dimethylamin und (2R,3S)-[ 3-(3-Methoxy-phenyl)-2-methylpentyl]dimethylamin. Die Ausbeute beträgt 4,10 kg (76 % der Theorie) mit einer GC-Reinheit von 90 %. Das Diastereomerenverhältnis (R,R Enantiomer zu R,S Enantiomer) beträgt 2,7:1 nach Isolierung der Base.

### Beispiel 17

In einer kühl- und heizbaren 50I Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 5,42kg (20 mol) (Z,E)-(2R)- [3-(3-Methoxy-phenyl)-2-methyl-pent-3-enyl]-dimethyl-amin Hydrochlorid bei 45° C und einer Rührerdrehzahl von 850 ±150 U/Min in 25I Wasser gelöst. Die Reaktionsanlage wurde mit Stickstoff inertisiert. Unter Stickstoffschutzgas wurde die Lösung mit einer Suspension von 0,086kg Palladium auf Aktivkohle (5 Gew.-%) in 2,5I Wasser versetzt. Nach erneutem Inertisieren wurde mit einem Wasserstoffvordruck von 5 bar und einem Innendruck von 1 bar hydriert bis die Wasserstoffaufnahme beendet ist. Nach beendeter Reaktion wurde die Anlage mit Stickstoff inertisiert und der Reaktionsansatz über einen Einschichtenfilter, belegt mit Filtererde, filtriert um den Katalysator zu entfernen. Das klare Filtrat wurde mit 1,5110 Gew.-%iger Natronlauge versetzt, wobei ein Niederschlag auftritt. Es wurde tert. Butylmethylether hinzugegeben eine Phasentrennung durchgeführt. Die organische Phase wurde am Rotationsverdampfer unter kontinuierlich reduziertem Druck bis zur Massenkonstanz eingeengt. Das verbleibende klare Öl ist eine Gemisch aus dem gewünschten (2R,3R)-[3-(3-Methoxy-phenyl)-2-methyl-pentyl]dimethylamin und (2R,3S)-[ 3-(3-Methoxy-phenyl)-2-methyl-pentyl]dimethylamin. Die Ausbeute beträgt 4,10kg (87 % der Theorie) mit einer GC-Reinheit von 85 %. Das Diastereomerenverhältnis (R,R Enantiomer zu R,S Enantiomer) beträgt 2,6:1 nach Isolierung der Base.

### Beispiel 18

In einer kühl- und heizbaren 50I Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurden 0,8 kg (3,44 mol) (Z,E)-(2R)- [3-(3-Methoxyphenyl)-2-methyl-pent-3-enyl]-dimethylamin bei 25° C und einer Rührerdrehzahl von 850 ±150 U/Min in 25I Ethanol abs. verg. gelöst. Die Reaktionsanlage wurde mit Stickstoff inertisiert.
Unter Stickstoffschutzgas wurden 60g Palladium auf Aktivkohle (5 Gew.-%) in 0,675 kg 32 Gew.-%iger Salzsäure aufgeschlämmt. Die Katalysatorsuspension wurde unter Rühren zu der Reaktionslösung zugegeben. Nach erneutem Inertisieren wurde mit einem Wasserstoffvordruck von 5 bar und einem Innendruck von 1 bar hydriert bis die Wasserstoffaufnahme beendet ist.
Nach beendeter Reaktion wurde die Anlage erneut mit Stickstoff inertisiert und der Reaktionsansatz über einen Einschichtenfilter, belegt mit Filtererde, filtriert um den Katalysator zu entfernen. Das klare Filtrat wurde am Rotationsverdampfer unter kontinuierlich reduziertem Druck bis zur Massenkonstanz eingeengt. Das verbleibende klare Öl war ein Gemisch aus dem gewünschten (2R,3R)-[3-(3-Methoxy-phenyl)-2-methyl-pentyl]dimethylamin und (2R,3S)-[ 3-(3-Methoxy-phenyl)-2-methyl-pentyl]dimethylamin. Die Ausbeute beträgt 0,80 kg (99 % der Theorie) mit einer GC-Reinheit von 94 %. Das Diastereomerenverhältnis (R,R Enantiomer zu R,S Enantiomer) beträgt 8,5:1.

### Beispiel 19

In einer 100I Doppelmantelreaktionsanlage mit elektrischem Ankerrüher, Pt100 Temperaturmeßeinrichtung und ölbasierendem Kühl/Heizsystem wurden 5,76kg (22,9 mol) (2S,3R)-1-Dimethylamino-3-(3-methoxy-phenyl)-2-methyl-pentan-3-ol bei 20°C und einer Rührdrehzahl von 100 U/min vorgelegt und innerhalb von 10 min mit 12,22I 36 Gew.-%iger wässriger Salzsäure versetzt. Die Reaktionsmischung wird auf 70°C innerhalb von 30 min erwärmt und bei dieser Temperatur 1 Stunde gerührt.
Anschiessend wurde die Lösung auf 20° C abgekühlt und mit 10125 Gew.-% Natronlauge und 5kg NaCl versetzt. Es bildet sich eine weisse Suspension. Die Suspension wird in die Hydrierapparatur überführt.
In einer kühl- und heizbaren 501 Doppelmantelhydrierapparatur mit festmontierter Deckelplatte mit Wasserstoff- und Stickstoffzufuhr, elektrischem Begasungsrührer, Stromstörer, PT 100 Temperaturmesseinrichtung, Schauglas, Handloch und Gascontroller "Büchi bpc" wurde zu der Suspension unter Stickstoffschutzgas eine Lösung von 0,288kg Palladium(II)-chlorid in 2,5I Wasser zugegeben und bei 25° C und einer Rührerdrehzahl von 850 ±150 U/Min vermischt. Die Reaktionsanlage wurde mit Stickstoff inertisiert. Dann wurde mit einem Wasserstoffvordruck von 5 bar und einem Innendruck von 1 bar hydriert bis die Wasserstoffaufnahme beendet war. Nach beendeter Reaktion wurde die Anlage mit Stickstoff inertisiert und der Reaktionsansatz über einen Einschichtenfilter, belegt mit Filtererde, filtriert um den Katalysator zu entfernen. Das klare Filtrat wurde mit 18I 32 Gew.-%iger Natronlauge versetzt und ein pH-Wert von 11-12 eingestellt, wobei ein Niederschlag auftrat. Es wurde tert. Butylmethylether hinzugegeben eine Phasentrennung durchgeführt. Die organische Phase wurde am Rotationsverdampfer unter kontinuierlich reduziertem Druck bis zur Massenkonstanz eingeengt. Das verbleibende klare Öl ist eine Gemisch aus dem gewünschten (2R,3R)-[3-(3-Methoxy-phenyl)-2-methylpentyl]dimethylamin und (2R,3S)-[ 3-(3-Methoxy-phenyl)-2-methylpentyl]dimethylamin. Die Ausbeute beträgt 4,10 kg (76 % der Theorie) mit einer GC-Reinheit von 90 %. Das Diastereomerenverhältnis (R,R Enantiomer zu R,S Enantiomer) beträgt 10:1 1 nach Isolierung der Base.

### Beispiel 17: GC-METHODE zur Analyse

### Probenvorbereitung:

Zu dem Probenmaterial wird tert.-BME hinzugegeben. Hydrochloride werden mit Dowex MWA-1 zur Base freigesetzt. Die klare organische Phase wird injiziert.

### Gaschromatographische Bedingungen:

| | |
|---|---|
| Kapillarsäule | : 6% Cyanopropyl-phenyl- 94 % Dimethylpolysiloxan |
| | z. B. OPTIMA 1301-DF1,0 µm; 30m x 0.32 |
| | mm I. D. |
| Trägergas | : Helium |
| Vordruck | : 70 kPa; Split: 20 ml/min |
| Ofentemperaturprogram | : Initial 160°C / 5 Min. |
| | Rate 5°C / Min. |
| | 190°C / 9 Min. |
| | Rate 10 ° / Min. |
| | 250 °C / 14 Minuten |
| Detektor | : FID |
| Detektortemperatur | : 260 °C |
| Injektortemperatur | : 250 °C |

## Patentansprüche

1. Verfahren zur Herstellung einer substituierten 3-Aryl-butyl-amin-Verbindung der allgemeinen Formel I, 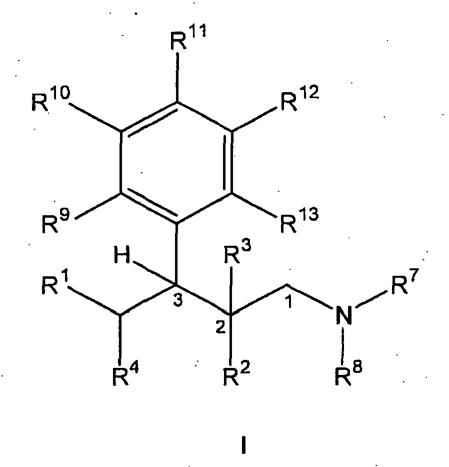 worin
R¹ ausgewählt ist aus H, C₁₋₃-Alkyl, verzweigt oder unverzweigt, gesättigt oder ungesättigt, unsubstituiert oder ein- oder mehrfach substituiert,
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄-₇-Cycloalkylrest bilden, unsubstituiert oder ein- oder mehrfach substituiert,
R⁴ ausgewählt ist aus H, C₁₋₃-Alkyl, verzweigt oder unverzweigt, gesättigt oder ungesättigt, unsubstituiert oder ein- oder mehrfach substituiert,
R⁷ und R⁸ jeweils unabhängig voneinander ausgewählt sind aus H oder C₁₋₃-Alkyl, verzweigt oder unverzweigt, gesättigt oder ungesättigt, unsubstituiert oder ein- oder mehrfach substituiert
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)2, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R¹⁸ jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates,
**dadurch gekennzeichnet, daß** in einem ersten Schritt a) eine 1-Amino-3-aryl-butan-3-ol-Verbindung der allgemeinen Formel II 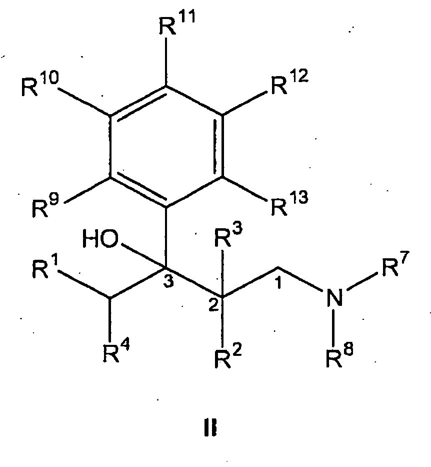 worin R¹, R², R³, R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, eingesetzt und unter Einwirkung einer Säure eliminiert wird zu einer substituierten 3-Aryl-but-3-enyl-amin-Verbindung der allgemeinen Formel III, 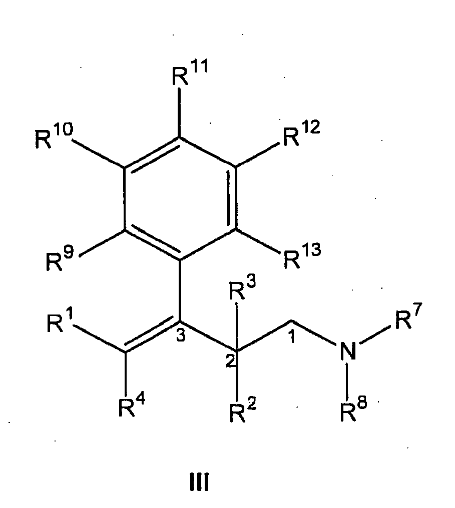 worin R¹, R², R³,R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, und in einem zweiten Schritt b) die entstehende substituierte 3-Aryl-but-3-enyl-amin-Verbindung gemäß der, allgemeinen Formel III dann zu einer substituierten 3-Aryl-butyl-amin-Verbindung der allgemeinen Formel I unter Beteiligung eines Metallkatalysators und Wasserstoff hydriert wird.

2. Verfahren gemäß Anspruch 1, **dadurch gekennzeichnet, daß** für Verbindungen gemäß **Formel I, Formel II und Formel III** gilt, daß
R⁴ ausgewählt ist aus H oder CH₃.

3. Verfahren gemäß Anspruch 1, **dadurch gekennzeichnet, daß** für Verbindungen gemäß **Formel I, Formel II und Formel III** gilt, daß
R¹ ausgewählt ist aus C₁₋₃-Alkyl, gesättigt oder ungesättigt, substituiert oder unsubstituiert, verzweigt oder unverzweigt.

4. Verfahren gemäß Anspruch 1, **dadurch gekennzeichnet, daß** für Verbindungen gemäß **Formel I, Formel 11 und Formel III** gilt, daß
R⁴ ausgewählt ist aus H oder CH₃,
**und/oder**
R¹ ausgewählt ist aus C₁₋₃-Alkyl, gesättigt oder ungesättigt, substituiert oder unsubstituiert, verzweigt oder unverzweigt.

5. Verfahren gemäß einem der Ansprüche 1 bis 4, **dadurch gekennzeichnet, daß** für Verbindungen gemäß **Formel I, Formel II und Formel III** gilt, daß
R⁷ und R⁸ jeweils unabhängig voneinander ausgewählt sind aus H oder CH₃.

6. Verfahren gemäß einem der Ansprüche 1 bis 5, **dadurch gekennzeichnet, daß** für Verbindungen gemäß **Formel I, Formel 11 und Formel III** gilt, daß
R¹ ausgewählt ist aus
C₁₋₃-Alkyl, gesättigt und unsubstituiert, verzweigt oder unverzweigt.

7. Verfahren gemäß einem der Ansprüche 1 bis 6, **dadurch gekennzeichnet, daß** für Verbindungen gemäß **Formel I, Formel 11 und Formel III** gilt, daß
R² und R³ unabhängig voneinander ausgewählt sind aus
H, C₁₋₄-Alkyl, gesättigt und unsubstituiert, verzweigt oder unverzweigt.

8. Verfahren gemäß Anspruch 7, **dadurch gekennzeichnet, daß** für Verbindungen gemäß **Formel I, Formel II und Formel III** gilt, daß
R³ = H und R² ≠ H.

9. Verfahren gemäß einem der Ansprüche 1 bis 6, **dadurch gekennzeichnet, daß** für Verbindungen gemäß **Formel I, Formel II und Formel III** gilt, daß
R² und R³ zusammen einen C₅₋₆-Cycloalkylrest bilden, gesättigt oder ungesättigt, unsubstituiert oder ein- oder mehrfach substituiert.

10. Verfahren gemäß einem der Ansprüche 1 bis 9, **dadurch gekennzeichnet, daß** für Verbindungen gemäß **Formel I, Formel 11 und Formel III** gilt, daß
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
oder R¹² und R¹¹ einen 3,4-OCH=CH-Ring bilden.

11. Verfahren gemäß Anspruch 1, **dadurch gekennzeichnet, daß** für Verbindungen gemäß **Formel I, Formel 11 und Formel III** gilt, daß
R¹ ausgewählt ist aus
C₁₋₃-Alkyl, gesättigt und unsubstituiert, verzweigt oder unverzweigt;
und/oder
R² und R³ unabhängig voneinander ausgewählt sind aus
H, C₁₋₄-Alkyl, gesättigt und unsubstituiert, verzweigt oder unverzweigt;
oder
R² und R³ zusammen einen C₅-₆-Cycloalkylrest bilden, gesättigt oder ungesättigt, unsubstituiert oder ein- oder mehrfach substituiert,
**und/oder**
R⁴ ausgewählt ist aus H,
**und/oder**
R⁷ und R⁸ jeweils unabhängig voneinander ausgewählt sind aus
H oder CH₃,
und/oder
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
oder R¹² und R¹¹ einen 3,4-OCH=CH-Ring bilden.

12. Verfahren gemäß einem der Ansprüch 1 bis 11, **dadurch gekennzeichnet, daß** für Verbindungen gemäß **Formel I** mit R³ = H und R² ≠ H gilt, daß diese in den Konfigurationen la oder lb 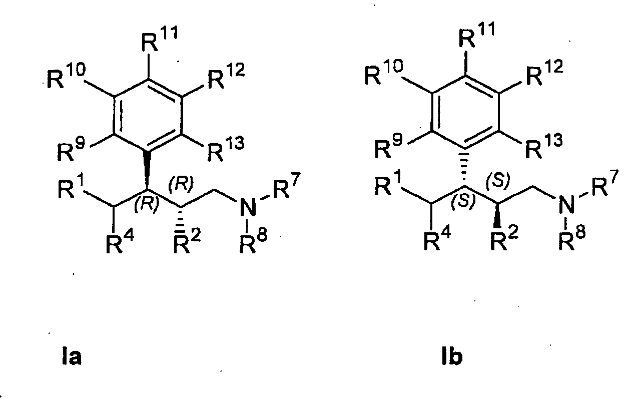 vorliegen.

13. Verfahren gemäß einem der Ansprüch 1 bis 12, **dadurch gekennzeichnet, daß** für Verbindungen gemäß **Formel II** mit R³ = H und R2 ≠ H gilt, daß diese in den Konfigurationen IIa oder IIb 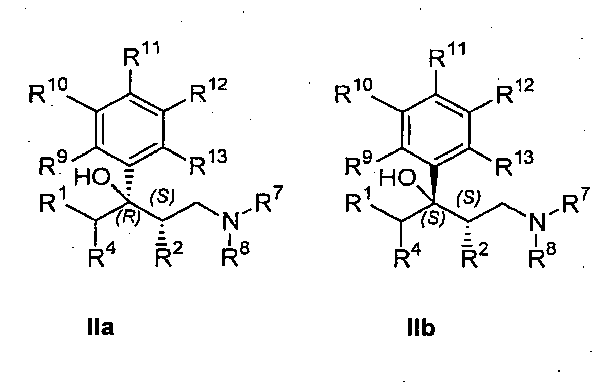 oder in den Konfigurationen IIc und IId 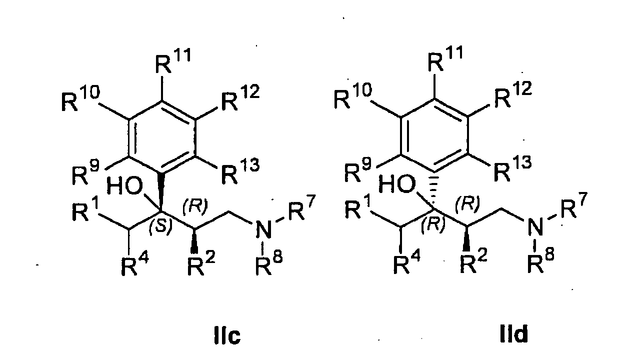 vorliegen.

14. Verfahren gemäß einem der Ansprüch 1 bis 13, **dadurch gekennzeichnet, daß** für Verbindungen gemäß **Formell III** mit R³ = H, R² ≠ H, R⁴ = H und R¹ ≠ H gilt, daß diese in den Konfigurationen IIIa oder IIIb vorliegen 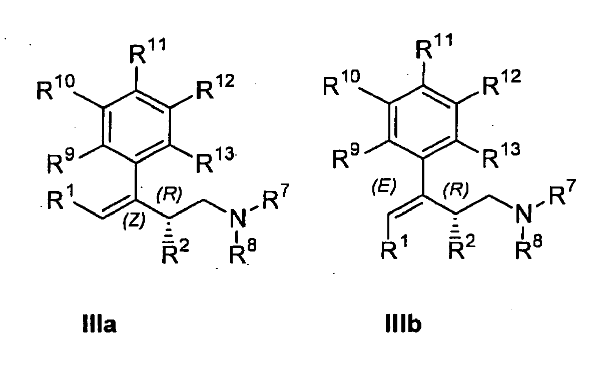 oder für Verbindungen gemäß **Formell III** mit R³ = H, R² ≠ H, R⁴ = H und R¹ ≠ H gilt, daß diese in den Konfigurationen IIIc oder IIId 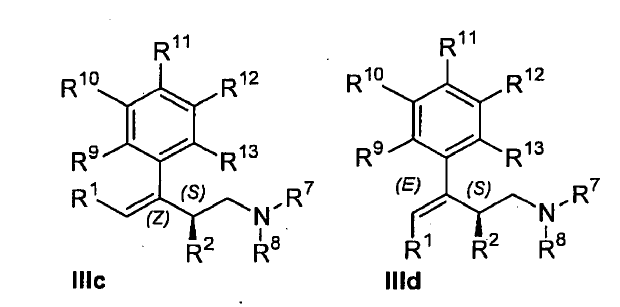 vorliegen.

15. Verfahren gemäß Anspruch 1, **dadurch gekennzeichnet, daß** für die VerbindungNerbindungen gemäß **Formeln I** eilt, daß mindestens eine davon, vorzugsweise als freie Base oder als Hydrochlorid, ausgewählt ist aus folgender Gruppe:
■ 3-(3-Dimethylamino-1-ethyl-2-methyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
■ (±)-(2RS,3RS)-[3-(3-Methoxy-phenyl)-2-methyl-pentyl]-dimethylamin,
■ *rac*(2RS,3RS)-[3-(3-Methoxy-phenyl)-2-methyl-pentyl]-dimethylamin,
■ 3{[3-(p-Isopropyl-phenyl-carbamoyl)-oxy-phenyl]-2-methyl-pentyl}-dimethylamin,
■ (2R,3R)-{3[3-(p-Isopropyl-phenyl-carbamoyl)-oxy-phenyl]-2-methyl-pentyl}-dimethylamin,
■ (2S,3S)-{3[3-(p-Isopropyl-phenyl-carbamoyl)-oxy-phenyl]-2-methyl-pentyl}-dimethylamin,
■ (2SR,3SR)-{3[3-(p-Isopropyl-phenyl-carbamoyl)-oxy-phenyl]-2-methyl-pentyl}-dimethylamin,

16. Verfahren gemäß Anspruch 1, **dadurch gekennzeichnet, daß** für die eingesetzte Verbindung/eingesetzten Verbindungen gemäß **Formel II** gilt, daß mindestens eine davon, vorzugsweise als freie Base oder als Hydrochlorid, ausgewählt ist aus folgender Gruppe:
■ 3-(3-Dimethylamino-1-ethyl-1-hydroxy-2-methyl-propyl)-phenol,
■ (1S,2S)-3-(3-Dimethylamino-1-ethyl-1-hydroxy-2-methylpropyl)-phenol,
■ (1R,2S)-3-(3-Dimethylamino-1-ethyl-1-hydroxy-2-methylpropyl)-phenol,
■ (1RS,2SS)-3-(3-Dimethylamino-1-ethyl-1-hydroxy-2-methyl-propyl)-phenol,
■ (1S,2R)-3-(3-Dimethylamino-1-ethyl-1-hydroxy-2-methylpropyl)-phenol,
■ (1R,2R)-3-(3-Dimethylamino-1-ethyl-1-hydroxy-2-methyl-propyl)-phenol,
■ (1RS,2RR)-3-(3-Dimethylamino-1-ethyl-1-hydroxy-2-methylpropyl)-phenol,
■ [3-(3-Methoxy-phenyl)-2-methyl-pentan-3-ol]-dimethylamin,
■ (2S,3S)-[3-(3-Methoxy-phenyl)-2-methyl-pentan-3-ol]-dimethylamin,
■ (2S,3R)-[3-(3-Methoxy-phenyl)-2-methyl-pentan-3-ol]-dimethylamin,
■ (2SS,3RS)-[3-(3-Methoxy-phenyl)-2-methyl-pentan-3-ol]-dimethylamin,
■ (2R,3S)-[3-(3-Methoxy-phenyl)-2-methyl-pentan-3-ol]-dimethylamin,
■ (2R,3R)-[3-(3-Methoxy-phenyl)-2-methyl-pentan-3-ol]-dimethylamin,
■ (2RR,3RS)-[3-(3-Methoxy-phenyl)-2-methyl-pentan-3-ol]-dimethylamin,
■ {3[3-(p-lsopropyl-phenyl-carbamoyl)-oxy-phenyl]-2-methyl-pentan-3-ol}-dimethylamin,
■ (2S,3R)-3[3-(p-Isopropyl-phenyl-carbamoyl)-oxy-phenyl]-2-methyl-pentan-3-ol}-dimethylamin,
■ (2S,3S)-{3[3-(p-Isopropyl-phenyl-carbamoyl)-oxy-phenyl]-2-methyl-pentan-3-ol)-dimethylamin,
■ (2SS,3RS)-{3[3-(p-Isopropyl-phenyl-carbamoyl)-oxy-phenyl]-2-methyl-pentan-3-ol}-dimethylamin,
■ (2R,3S)-{3[3-(p-lsopropyl-phenyl-carbamoyl)-oxy-phenyl]-2-methyl-pentan-3-ol}-dimethylamin,
■ (2R,3R)-{3[3-(p-Isopropyl-phenyl-carbamoyl)-oxy-phenyl]-2-methyl-pentan-3-ol}-dimethylamin,
■ (2RR,3RS)-{3[3-(p-Isopropyl-phenyl-carbamoyl)-oxy-phenyl]-2-methyl-pentan-3-ol}-dimethylamin.

17. Verfahren gemäß Anspruch 1, **dadurch gekennzeichnet, daß** für die VerbindungNerbindungen gemäß **Formel III** gilt, daß mindestens eine davon, vorzugsweise als freie Base oder als Hydrochlorid, ausgewählt ist aus folgender Gruppe:
■ 3-(3-Dimethylamino-1-ethenyl-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl-2-methyl-propyl)-phenol,
■ (Z,E)-(2S)-3-(3-Dimethylamino-1-ethenyl-2-methyl-propyl)-phenol,
■ 3-(3-Dimethylamino-1-etheny -2-methyl-propyl)-phenol,
■ (Z)-(2R)-3-(3-Dimethylamino-1-ethenyl-1-2-methyl-propyl)-phenol,
■ (E)-(2R)-3-(3-Dimethylamino-1-ethenyl-1- 2-methyl-propyl)-phenol,
■ (Z,E)-(2R)-3-(3-Dimethylamino-1-ethenyl-1- 2-methylpropyl)-phenol,
■ (Z)-(2S)-3-(3-Dimethylamino-1-ethenyl-1-2-methyl-propyl)-phenol,
■ (E)-(2S)-(3-Dimethylamino-1-ethenyl-1-2-methyl-propyl)-phenol,
■ (Z,E)-(2S)-3-(3-Dimethylamino-1-ethenyl- 2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin.
■ {3[3-(p-Isopropyl-phenyl-carbamoyl)-oxy-phenyl]-2-methyl-pent-3-enyl}-dimethylamin,
■ (Z)-(2R)-{3[3-(p-Isopropyl-phenyl-carbamoyl)-oxy-phenyl]-2-methyl-pent-3-enyl}-dimethylamin,
■ (E)-(2R)-{3[3-(p-Isopropyl-phenyl-carbamoyl)-oxy-phenyl]-2-methyl-pent-3-enyl}-dimethylamin,
■ (Z,E)-(2R)-{3[3-(p-Isopropyl-phenyl-carbamoyl)-oxy-phenyl]-2-methyl-pent-3-enyl}-dimethylamin,
■ (Z)-(2S)-{3[3-(p-Isopropyl-phenyl-carbamoyl)-oxy-phenyl]-2-methyl-pent-3-enyl}-dimethylamin,
■ (E)-(2S)-{3[3-(p-Isopropyl-phenyl-carbamoyl)-oxy-phenyl]-2-methyl-pent-3-enyl}-dimethylamin,
■ (Z,E)-(2S)-{3[3-(p-Isopropyl-phenyl-carbamoyl)-oxy-phenyl]-2-methyl-pent-3-enyl}-dimethylamin,

18. Verfahren gemäß Anspruch 1, **dadurch gekennzeichnet, daß** bei der eingesetzten Verbindung gemäß Formel II an Position 2 gemäß Formel II ein chirales Zentrum vorliegt.

19. Verfahren gemäß Anspruch 1, **dadurch gekennzeichnet, daß** bei der Verbindung gemäß Formel I an Position 2 gemäß Formel I ein chirales Zentrum vorliegt.

20. Verfahren gemäß Anspruch 1, **dadurch gekennzeichnet, daß** bei der Verbindung gemäß Formel III an Position 2 gemäß Formel III ein chirales Zentrum vorliegt.

21. Verfahren gemäß Anspruch 1, **dadurch gekennzeichnet, daß** die eingesetzte Verbindung nach Formel II enantiomerenrein ist.

22. Verfahren gemäß Anspruch 1, **dadurch gekennzeichnet, daß** die eingesetzte Verbindung nach Formel II diastereomerenrein ist.

23. Verfahren gemäß Anspruch 1, **dadurch gekennzeichnet, daß** die eingesetzte Verbindung nach Formel II enantiomeren- und diastereomerenrein ist.

24. Verfahren gemäß Anspruch 1, **dadurch gekennzeichnet, daß** die eingesetzte Verbindung gemäß Formel II ausgewählt ist aus:
• (2S,3S)-[3-(3-Methoxy-phenyl)-2-methyl-pentan-3-ol]-dimethylamin,
• (25,3R)-[3-(3-Methoxy-phenyl)-2-methyl-pentan-3-ol]-dimethylamin
oder
eine Mischung von (2S,3S)-[3-(3-Methoxy-phenyl)-2-methyl-pentan-3-ol]-dimethylamin und (2S,3R)-[3-(3-Methoxy-phenyl)-2-methyl-pentan-3-ol]-dimethylamin, bzw. (2SS,3RS)-[3-(3-Methoxy-phenyl)-2-methyl-pentan-3-ol]-dimethylamin ist.

25. Verfahren gemäß Anspruch 1, **dadurch gekennzeichnet, daß** die eingesetzte Verbindung gemäß Formel II ausgewählt ist aus:
• (2R,3R)-[3-(3-Methoxy-phenyl)-2-methyl-pentan-3-ol]-dimethylamin,
• (2R,3S)-[3-(3-Methoxy-phenyl)-2-methyl-pentan-3-ol]-dimethylamin
oder
eine Mischung von (2R,3R)-[3-(3-Methoxy-phenyl)-2-methyl-pentan-3-ol]-dimethylamin und (2R,3S)-[3-(3-Methoxy-phenyl)-2-methyl-pentan-3-ol]-dimethylamin, bzw. (2RR,3RS)-[3-(3-Methoxy-phenyl)-2-methyl-pentan-3-ol]-dimethylamin ist.

26. Verfahren gemäß Anspruch 1, **dadurch gekennzeichnet, daß** die eingesetzte Verbindung gemäß Formel II ausgewählt ist aus:
• (2S,3S)-1-Dimethylamino-3-(3-methoxy-phenyl)-2-methyl-pentan-3-ol,
• (2S,3R)-1-Dimethylamino-3-(3-methoxy-phenyl)-2-methyl-pentan-3-ol
oder
eine Mischung von (2S,3S)-1-Dimethylamino-3-(3-methoxy-phenyl)-2-methyl-pentan-3-ol und (2S,3R)-1-Dimethylamino-3-(3-methoxy-phenyl)-2-methyl-pentan-3-ol, bzw. (2SS,3RS)-[3-(3-Methoxy-phenyl)-2-methyl-pentan-3-ol]-dimethylamin ist.

27. Verfahren gemäß Anspruch 1, **dadurch gekennzeichnet, daß** die eingesetzte Verbindung gemäß Formel II ausgewählt ist aus:
• (2R,3R)-1-Dimethylamino-3-(3-methoxy-phenyl)-2-methyl-pentan-3-ol,
• (2R,3S)-1-Dimethylamino-3-(3-methoxy-phenyl)-2-methyl-pentan-3-ol
oder
eine Mischung von (2R,3R)-1-Dimethylamino-3-(3-methoxy-phenyl)-2-methyl-pentan-3-ol und (2R,3S)-1-Dimethylamino-3-(3-methoxy-phenyl)-2-methyl-pentan-3-ol, bzw. (2RR,3RS)-[3-(3-Methoxy-phenyl)-2-methyl-pentan-3-ol]-dimethylamin ist.

28. Verfahren gemäß einem der Ansprüche 1 bis 17, **dadurch gekennzeichnet, daß** in Schritt a) organische Säuren oder Halogen/Wasserstoffsäuren verwendet werden.

29. Verfahren gemäß Anspruch 18, **dadurch gekennzeichnet, daß** in Schritt a) Ameisensäure, Salzsäure oder Bromwasserstoffsäure verwendet werden.

30. Verfahren gemäß Anspruch 18, **dadurch gekennzeichnet, daß** in Schritt a) Ameisensäure verwendet wird.

31. Verfahren gemäß Anspruch 18, **dadurch gekennzeichnet, daß** in Schritt a) Salzsäure verwendet wird.

32. Verfahren gemäß Anspruch 18, **dadurch gekennzeichnet, daß** in Schritt a) Bromwasserstoffsäure verwendet wird.

33. Verfahren gemäß Anspruch 18, **dadurch gekennzeichnet, daß** die Säure in Schritt a) in hoher Konzentration eingesetzt wird.

34. Verfahren gemäß Anspruch 21, **dadurch gekennzeichnet, daß** die Salzsäure in Schritt a) >20%-ig ist.

35. Verfahren gemäß einem der Ansprüche 1 bis 24, **dadurch gekennzeichnet, daß** nach dem Schritt a) die eliminierten Verbindungen gemäß Formel III mit Salzsäuregas kristallisiert werden.

36. Verfahren gemäß einem der Ansprüche 1 bis 25, **dadurch gekennzeichnet, daß** die Reaktionszeit von Schritt a) zwischen 2 und 10h beträgt.

37. Verfahren gemäß einem der Ansprüche 1 bis 26, **dadurch gekennzeichnet, daß** in Schritt a) die Reaktionstemperatur zwischen 35 und 100°C beträgt.

38. Verfahren gemäß einem der Ansprüche 1 bis 27, **dadurch gekennzeichnet, daß** in Schritt a) das Lösungsmittel ausgewählt ist aus:
H₂O oder Alkohol oder wässrigen alkoholischen Lösungen.

39. Verfahren gemäß einem der Ansprüche 18 bis 24, **dadurch gekennzeichnet, daß** in Schritt a) das Lösungsmittel wässrige Säure ist.

40. Verfahren gemäß einem der Ansprüche 18 bis 24, **dadurch gekennzeichnet, daß** in Schritt a) die eingesetzte Verbindung gemäß Formel II in wässriger Säure gelöst wird.

41. Verfahren gemäß einem der Ansprüche 21 oder 24, **dadurch gekennzeichnet, daß** in Schritt a) die eingesetzte Verbindung gemäß Formel II in wässriger Salzsäure gelöst wird.

42. Verfahren gemäß Anspruch 1, **dadurch gekennzeichnet, daß** in Schritt b) das Lösungsmittel ausgewählt ist aus:
H₂O oder Alkohol oder wässrigen alkoholischen oder wässrigen sauren Lösungen.

43. Verfahren gemäß Anspruch 1, **dadurch gekennzeichnet, daß** in Schritt b) das Lösungsmittel ausgewählt ist aus:
H₂O oder Ethanol oder wässriger ethanolischer Lösung oder wässriger Salzsäure.

44. Verfahren gemäß Anspruch 1, **dadurch gekennzeichnet, daß** in Schritt b) der verwendete Katalysator ein Edelmetall enthält.

45. Verfahren gemäß Anspruch 44, **dadurch gekennzeichnet, daß** in Schritt b) der verwendete Katalysator Palladium auf Aktivkohle oder Palladium(II)-Chlorid ist.

46. Verfahren gemäß Anspruch 44, **dadurch gekennzeichnet, daß** in Schritt b) der verwendete Katalysator Palladium auf Aktivkohle (1 - 10 Gew.-%) ist.

47. Verfahren gemäß Anspruch 1, **dadurch gekennzeichnet, daß** die Temperatur in Schritt b) zwischen 20 und 40°C gehalten wird.

48. Verfahren gemäß Anspruch 1, **dadurch gekennzeichnet, daß** in Schritt b) vor der Hydrierung eine Schutzgasatmosphäre angelegt wird.

49. Verfahren gemäß Anspruch 1, **dadurch gekennzeichnet, daß** in Schritt b) der Hydrierungsschritt bei einem Wasserstoffvordruck von 3-10bar stattfindet
und/ oder
der Hydrierungsschritt bei einem Wasserstoffinnendruck von 0,5-3 bar stattfindet.

50. Verfahren gemäß Anspruch 1, **dadurch gekennzeichnet, daß** in Schritt b) zu Beginn die Ausgangsprodukte im Lösungsmittel stark verdünnt sind/werden.

51. Verfahren gemäß Anspruch 1, **dadurch gekennzeichnet, daß** das Lösungsmittel für beide Schritte a) und b) wässrige saure Lösung.

52. Verfahren gemäß Anspruch 1 und 51, **dadurch gekennzeichnet, daß** zwischen Schritt a) und Schritt b) kein Produkt isoliert wird.

53. Verfahren gemäß Anspruch 52, **dadurch gekennzeichnet, daß** zu Beginn die Ausgangsprodukte im Lösungsmittel stark verdünnt sind/werden.

54. Verfahren gemäß einem der Ansprüche 1 oder 52, **dadurch gekennzeichnet, daß** die eingesetzte Verbindung gemäß Formel II in wässriger Säure gelöst wird.

55. Verfahren gemäß Anspruch 54, **dadurch gekennzeichnet, daß** die eingesetzte Verbindung gemäß Formel II in wässriger Salzsäure gelöst wird.

56. Verfahren zur Herstellung einer substituierten 3-Aryl-butyl-amin-Verbindung der allgemeinen Formel I, 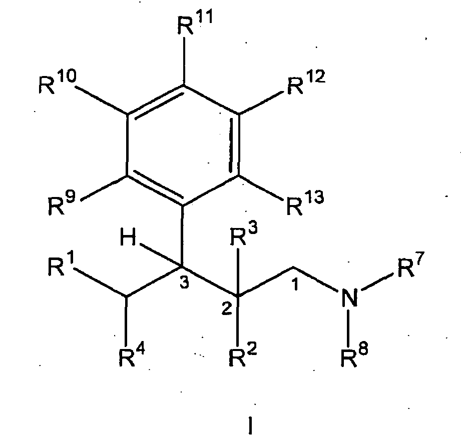 worin
R¹ ausgewählt ist aus H, C₁₋₃-Alkyl, verzweigt oder unverzweigt, gesättigt oder ungesättigt, unsubstituiert oder ein- oder mehrfach substituiert;
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄₋₇-Cycloalkylrest bilden, unsubstituiert oder ein- oder mehrfach substituiert,
R⁴ ausgewählt ist aus H, C₁₋₃-Alkyl, verzweigt oder unverzweigt, gesättigt oder ungesättigt, unsubstituiert oder ein- oder mehrfach substituiert,
R⁷ und R⁸ jeweils unabhängig voneinander ausgewählt sind aus H oder C₁₋₃-Alkyl, verzweigt oder unverzweigt, gesättigt oder ungesättigt, unsubstituiert oder ein- oder mehrfach substituiert
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH3, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R¹⁸ jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates,
**dadurch gekennzeichnet, daß** eine substituierte 3-Aryl-but-3-enyl-amin-Verbindung der allgemeinen Formel III, 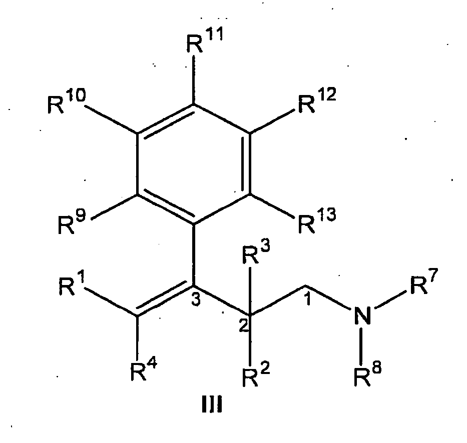 worin R¹, R², R³,R⁴, R⁷, R⁸, R⁹, R¹⁰, R¹¹, R¹² und R¹³ die vorstehend genannte Bedeutung haben, jeweils ggf. in Form eines ihrer reinen Stereoisomeren, insbesondere Enantiomeren oder Diastereomeren; ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, unter Beteiligung eines Metallkatalysators und Wasserstoff zu einer substituierten 3-Aryl-butyl-amin-Verbindung der allgemeinen Formel I hydriert wird.

57. Verfahren gemäß Anspruch 56, **dadurch gekennzeichnet, daß** für Verbindungen gemäß **Formel I und Formel III** gilt, daß R⁴ ausgewählt ist aus H oder CH₃.

58. Verfahren gemäß Anspruch 56, **dadurch gekennzeichnet, daß** für Verbindungen gemäß **Formel I und Formel III** gilt, daß
R¹ ausgewählt ist aus C₁₋₃-Alkyl, gesättigt oder ungesättigt, substituiert oder unsubstituiert, verzweigt oder unverzweigt.

59. Verfahren gemäß Anspruch 56, **dadurch gekennzeichnet, daß** für Verbindungen gemäß **Formel I und Formel III** gilt, daß
R⁴ ausgewählt ist aus H oder CH₃,
**und/oder**
R¹ ausgewählt ist aus C₁₋₃-Alkyl, gesättigt öder ungesättigt, substituiert oder unsubstituiert, verzweigt oder unverzweigt.

60. Verfahren gemäß einem der Ansprüche 56 bis 59, **dadurch gekennzeichnet, daß** für Verbindungen gemäß **Formel I und Formel III** gilt, daß
R⁷ und R⁸ jeweils unabhängig voneinander ausgewählt sind aus H oder CH₃.

61. Verfahren gemäß einem der Ansprüche 56 bis 60, **dadurch gekennzeichnet, daß** für Verbindungen gemäß **Formel I und Formel III** gilt, daß
R¹ ausgewählt ist aus
C₁₋₃-Alkyl, gesättigt und unsubstituiert, verzweigt oder unverzweigt.

62. Verfahren gemäß einem der Ansprüche 56 bis 61, **dadurch gekennzeichnet, daß** für Verbindungen gemäß **Formel I und Formel III** gilt, daß
R² und R³ unabhängig voneinander ausgewählt sind aus
H, C₁₋₄-Alkyl, gesättigt und unsubstituiert, verzweigt oder unverzweigt.

63. Verfahren gemäß Anspruch 62, **dadurch gekennzeichnet, daß** für Verbindungen gemäß **Formel I und Formel III** gilt, daß
R³ = H und R² ≠ H.

64. Verfahren gemäß einem der Ansprüche 56 bis 61, **dadurch gekennzeichnet, daß** für Verbindungen gemäß **Formel I und Formel III** gilt, daß
R² und R³ zusammen einen C₅₋₆-Cycloalkylrest bilden, gesättigt oder ungesättigt, unsubstituiert oder ein- oder mehrfach substituiert.

65. Verfahren gemäß einem der Ansprüche 56 bis 64, **dadurch gekennzeichnet, daß** für Verbindungen gemäß **Formel und Formel III** gilt, daß
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
oder R¹² und H¹¹ einen 3,4-OCH-CH-Ring bilden

66. Verfahren gemäß Anspruch 56, **dadurch gekennzeichnet, daß** für Verbindungen gemäß **Formel I und Formel III** gilt, daß
R¹ ausgewählt ist aus
C₁₋₃-Alkyl, gesättigt und unsubstituiert, verzweigt oder unverzweigt;
**und/oder**
R² und R³ unabhängig voneinander ausgewählt sind aus
H, C₁₋₄-Alkyl, gesättigt und unsubstituiert, verzweigt oder unverzweigt;
oder
R² und R³ zusammen einen C₅₋₆-Cycloalkylrest bilden, gesättigt oder ungesättigt, unsubstituiert oder ein- oder mehrfach substituiert,
**und/oder**
R⁴ ausgewählt ist aus H,
und/oder
R⁷ und R⁸ jeweils unabhängig voneinander ausgewählt sind aus
H oder CH₃,
und/oder
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
oder R¹² und R¹¹ einen 3,4-OCH=CH-Ring bilden.

67. Verfahren gemäß einem der Ansprüch 56 bis 66, **dadurch gekennzeichnet, daß** für Verbindungen gemäß **Formel I** mit R³ = H und R² ≠ H gilt, daß diese in den Konfigurationen la oder lb 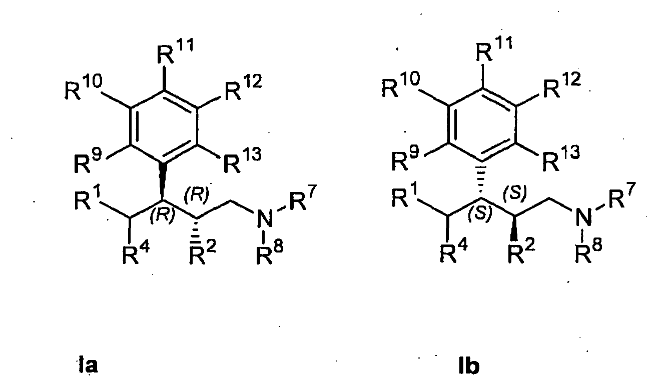 vorliegen.

68. Verfahren gemäß einem der Ansprüch 56 bis 67, **dadurch gekennzeichnet, daß** für Verbindungen gemäß **Formell III** mit R³ = H, R² ≠ H, R⁴ = H und R¹ ≠ H gilt, daß diese in den Konfigurationen IIIa oder IIIb vorliegen 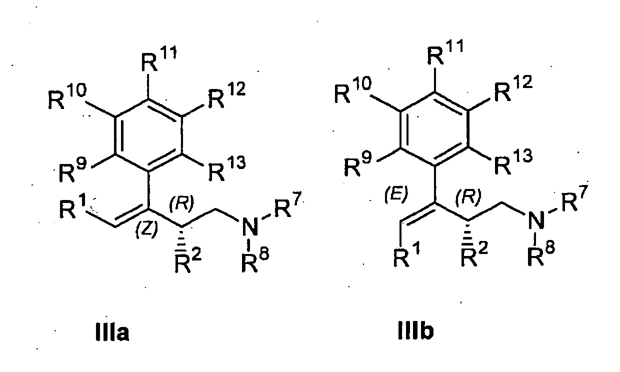 oder für Verbindungen gemäß **Formell III** mit R³ = H, R² ≠ H, R⁴ = H und R¹ ≠ H gilt, daß diese in den Konfigurationen IIIc oder IIId 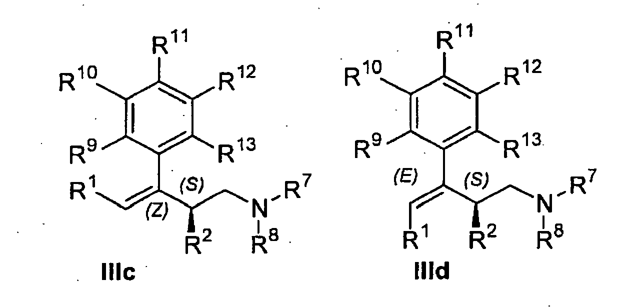 vorliegen.

69. Verfahren gemäß Anspruch 56, **dadurch gekennzeichnet, daß** für die VerbindungNerbindungen gemäß **Formel I** gilt, daß mindestens eine davon, vorzugsweise als freie Base oder als Hydrochlorid, ausgewählt ist aus folgender Gruppe:
■ 3-(3-Dimethylamino-1-ethyl-2-methyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,25)-3-(3-Dimethylamino-1-ethyl-2-methyl-propyl)-phenol,
■ (±)-(1RS,2RS)-3-(3-Dimethylamino-1-ethyl-2-methyl-propyl)-phenol,
■ *rac*-(1RS,2RS)-3-(3-Dimethylamino-1-ethyl-2-methyl-propyl)-phenol,
■ [3-(3-Methoxy-phenyl)-2-methyl-pentyl]-dimethylamin,
■ (2R,3R)-[3-(3-Methoxy-phenyl)-2-methyl-pentyl]-dimethylamin,
■ (-)-(2R,3R)-[3-(3-Methoxy-phenyl)-2-methyl-pentyl]-dimethylamin,
■ (2S,3S)-[3-(3-Methoxy-phenyl)-2-methyl-pentyl]-dimethylamin,
■ (+)-(2S,3S)-[3-(3-Methoxy-phenyl)-2-methyl-pentyl]-dimethylamin,
■ (±)-(2RS,3RS)-[3-(3-Methoxy-phenyl)-2-methyl-pentyl]-dimethylamin,
■ *rac*(2RS,3RS)-[3-(3-Methoxy-phenyl)-2-methyl-pentyl]-dimethylamin,
■ 3{[3-(p-lsopropyl-phenyl-carbamoyl)-oxy-phenyl]-2-methylpentyl}-dimethylamin,
■ (2R,3R)-{3[3-(p-Isopropyl-phenyl-carbamoyl)-oxy-phenyl]-2-methyl-pentyl}-dimethylamin,
■ (2S,3S)-{3[3-(p-Isopropyl-phenyl-carbamoyl)-oxy-phenyl]-2-methyl-pentyl}dirnethylamin,
■ (2SR,3SR)-{3[3-(p-Isopropyl-phenyl-carbamoyl)-oxy-phenyl]-2-methyl-pentyl}-dimethylamin,

70. Verfahren gemäß Anspruch 56, **dadurch gekennzeichnet, daß** für die eingesetzte/n Verbindung/Verbindungen gemäß Formel III gilt, daß mindestens eine davon, vorzugsweise als freie Base oder als Hydrochlorid, ausgewählt ist aus folgender Gruppe:
■ 3-(3-Dimethylamino-1-ethenyl-2-methyl-propyl)-phenol,
■ (Z)-(2R)-3-(3-Dimethylamino-1-ethenyl-2-methyl-propyl)-phenol,
■ (E)-(2R)-3-(3-Dimethylamino-1-ethenyl-2-methyl-propyl)-phenol,
■ (Z,E)-(2R)-3-(3-Dimethylamino-1-ethenyl-2-methyl-propyl)-phenol,
■ (Z)-(2S)-3-(3-Dimethylamino-1-ethenyl-2-methyl-propyl)-phenol,
■ (E)-(2S)-(3-Dimethylamino-1-ethenyl-2-methyl-propyl)-phenol,
■ (Z,E)-(2S)-3-(3-Dimethylamino-1-ethenyl-2-methyl-propyl)-phenol,
■ 3-(3-Dimethylamino-1-etheny -2-methyl-propyl)-phenol,
■ (Z)-(2R)-3-(3-Dimethylamino-1-ethenyl-1- 2-methyl-propyl)-phenol,
■ (E)-(2R)-3-(3-Dimethylamino-1-ethenyl-1- 2-methyl-propyl)-phenol,
■ (Z,E)-(2R)-3-(3-Dimethylamino-1-ethenyl-1- 2-methyl-propyl)-phenol,
■ (Z)-(2S)-3-(3-Dimethylamino-1-ethenyl-1- 2-methyl-propyl)-phenol,
■ (E)-(2S)-(3-Dimethylamino-1-ethenyl-1- 2-methyl-propyl)-phenol,
■ (Z,E)-(2S)-3-(3-Dimethylamino-1-ethenyl- 2-methyl-propyl)-phenol,
■ [3-(3-Methoxy-phenyl)-2-methyl-pent-3-enyl]-dimethylamin,
■ (Z)-(2R)-[3-(3-Methoxy-phenyl)-2-methyl-pent-3-enyl]-dimethylamin,
■ (E)-(2R)-[3-(3-Methoxy-phenyl)-2-methyl-pent-3-enyl]-dimethylamin,
■ (Z,E)-(2R)-[3-(3-Methoxy-phenyl)-2-methyl-pent-3-enyl]-dimethylamin,
■ (Z)-(2S)-[3-(3-Methoxy-phenyl)-2-methyl-pent-3-enyl]-dimethylamin,
■ (E)-(2S)-[3-(3-Methoxy-phenyl)-2-methyl-pent-3-enyl]-dimethylamin,
■ (Z,E)-(2S)-[3-(3-Methoxy-phenyl)-2-methyl-pent-3-enyl]-dimethylamin.
■ {3[3-(p-Isopropyl-phenyl-carbamoyl)-oxy-phenyl]-2-methyl-pent-3-enyl}-dimethylamin,
■ (Z)-(2R)-{3[3-(p-Isopropyl-phenyl-carbamoyl)-oxy-phenyl]-2-methyl-pent-3-enyl}-dimethylamin,
■ (E)-(2R)-{3[3-(p-Isopropyl-phenyl-carbamoyl)-oxy-phenyl]-2-methyl-pent-3-enyl}-dimethylamin,
■ (Z,E)-(2R)-{3[3-(p-Isopropyl-phenyl-carbamoyl)-oxy-phenyl]-2-methyl-pent-3-enyl}-dimethylamin,
■ (Z)-(2S)-{3[3-(p-Isopropyl-phenyl-carbamoyl)-oxy-phenyl]-2-methyl-pent-3-enyl}-dimethylamin,
■ (E)-(2S)-{3[3-(p-Isopropyl-phenyl-carbamoyl)-oxy-phenyl]-2-methyl-pent-3-enyl}-dimethylamin,
■ (Z,E)-(2S)-{3[3-(p-Isopropyl-phenyl-carbamoyl)-oxy-phenyl]-2-methyl-pent-3-enyl}-dimethylamin,

71. Verfahren gemäß Anspruch 56, **dadurch gekennzeichnet, daß** bei der eingesetzten Verbindung gemäß Formel III an Position 2 gemäß Formel III ein chirales Zentrum vorliegt.

72. Verfahren gemäß Anspruch 56, **dadurch gekennzeichnet, daß** bei der Verbindung gemäß Formel I an Position 2 gemäß Formel I ein chirales Zentrum vorliegt.

73. Verfahren gemäß Anspruch 56, **dadurch gekennzeichnet, daß** die eingesetzte Verbindung nach Formel III enantiomerenrein ist.

74. Verfahren gemäß Anspruch 56, **dadurch gekennzeichnet, daß** die eingesetzte Verbindung nach Formel III diastereomerenrein ist.

75. Verfahren gemäß Anspruch 56, **dadurch gekennzeichnet, daß** die eingesetzte Verbindung nach Formel III enantiomeren- und diastereomerenrein ist.

76. Verfahren gemäß Anspruch 56, **dadurch gekennzeichnet, daß** die eingesetzte Verbindung gemäß Formel III ausgewählt ist aus:
• (Z)-(2R)-[3-(3-Methoxy-phenyl)-2-methyl-pent-3-enyl]-dimethylamin,
• (E)-(2R)-[3-(3-Methoxy-phenyl)-2-methyl-pent-3-enyl]-dimethylamin
oder
eine Mischung von (Z)-(2R)-[3-(3-Methoxy-phenyl)-2-methyl-pent-3-enyl]-dimethylamin und (E)-(2R)-[3-(3-Methoxy-phenyl)-2-methyl-pent-3-enyl]-dimethylamin bzw. (Z,E)-(2R)-[3-(3-Methoxy-phenyl)-2-methyl-pent-3-enyl]-dimethylamin ist.

77. Verfahren gemäß Anspruch 56, **dadurch gekennzeichnet, daß** die eingesetzte Verbindung gemäß Formel III ausgewählt ist aus:
• (Z)-(2S)-[3-(3-Methoxy-phenyl)-2-methyl-pent-3-enyl]-dimethylamin,
• (E)-(25)-[3-(3-Methoxy-phenyl)-2-methyl-pent-3-enyl]-dimethylamin
oder
eine Mischung von (Z)-(2S)-[3--(3-Methoxy-phenyl)-2-methyl-pent-3-enyl]-dimethylamin und (E)-(2S)-[3-(3-Methoxy-phenyl)-2-methyl-pent-3-enyl]-dimethylamin, bzw. (Z,E)-(2S)-[3-(3-Methoxy-phenyl)-2-methyl-pent-3-enyl]-dimethylamin ist.

78. Verfahren gemäß Anspruch 56, **dadurch gekennzeichnet, daß** das Lösungsmittel ausgewählt ist aus:
H₂O oder Alkohol oder wässrigen alkoholischen oder wässrigen sauren Lösungen.

79. Verfahren gemäß Anspruch 56, **dadurch gekennzeichnet, daß** das Lösungsmittel ausgewählt ist aus:
H₂O oder Ethanol oder wässriger ethanolischer Lösung oder wässriger Salzsäure.

80. Verfahren gemäß Anspruch 56, **dadurch gekennzeichnet, daß** der verwendete Katalysator ein Edelmetall enthält.

81. Verfahren gemäß Anspruch 80, **dadurch gekennzeichnet, daß** der verwendete Katalysator Palladium auf Aktivkohle oder Palladium(II)-Chlorid ist.

82. Verfahren gemäß Anspruch 80, **dadurch gekennzeichnet, daß** der verwendete Katalysator Palladium auf Aktivkohle (1 - 10 Gew.-%) ist.

83. Verfahren gemäß Anspruch 56, **dadurch gekennzeichnet, daß** die Temperatur zwischen 20 und 40°C gehalten wird.

84. Verfahren gemäß Anspruch 56, **dadurch gekennzeichnet, daß** vor der Hydrierung eine Schutzgasatmosphäre angelegt wird.

85. Verfahren gemäß Anspruch 56, **dadurch gekennzeichnet, daß** der Hydrierungsschritt bei einem Wasserstoffvordruck von 3-10bar stattfindet
und/oder
der Hydrierungsschritt bei einem Wasserstoffinnendruck von 0,5-3 bar stattfindet.

86. Verfahren gemäß Anspruch 56, **dadurch gekennzeichnet, daß** zu Beginn die Ausgangsprodukte im Lösungsmittel stark verdünnt sind/werden.

87. Verfahren gemäß Anspruch 56, **dadurch gekennzeichnet, daß** die eingesetzte Verbindung gemäß Formel III in wässriger Säure gelöst wird.

88. Verfahren gemäß Anspruch 87, **dadurch gekennzeichnet, daß** die eingesetzte Verbindung gemäß Formel III in wässriger Salzsäure gelöst wird.

89. Verfahren gemäß einem der Ansprüche 1 oder 56, **dadurch gekennzeichnet, daß** am Ende des Verfahren die Produkte in organischem Lösungsmittel gefällt werden.

90. Verfahren gemäß einem der Ansprüche 1 oder 56, **dadurch gekennzeichnet, daß** am Ende des Verfahrens die Produkte mit Säure bzw. Säuregas, gefällt werden.

91. Verfahren gemäß einem der Ansprüche 1 oder 56, **dadurch gekennzeichnet, daß** am Ende des Verfahrens die Produkte in organischem Lösungsmittel mit Säure bzw. Säuregas gefällt werden.

## Claims

1. Process for the preparation of a substituted 3-aryl-butyl-amine compound of the general formula I 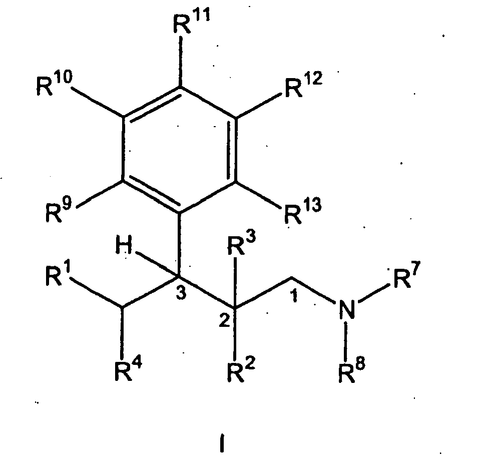 wherein
R¹ is chosen from H, C₁₋₃-alkyl, branched or unbranched, saturated or unsaturated, unsubstituted or mono- or polysubstituted,
R² and R³ in each case independently of one another are chosen from H or C₁₋₄-alkyl, branched or unbranched, saturated or unsaturated, unsubstituted or mono- or polysubstituted,
or
R² and R³ together form a saturated C₄₋₇-cycloalkyl radical, unsubstituted or mono- or polysubstituted,
R⁴ is chosen from H, C₁₋₃-alkyl, branched or unbranched, saturated or unsaturated, unsubstituted or mono- or polysubstituted,
R⁷ and R⁸ in each case independently of one another are chosen from H or C₁₋₃-alkyl, branched or unbranched, saturated or unsaturated, unsubstituted or mono- or polysubstituted,
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono-or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl) CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂, where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
in each case in the form of one of its pure stereoisomers, in particular enantiomers or diastereomers, its racemates or in the form of a mixture of stereoisomers, in particular the enantiomers or diastereomers, in any desired mixing ratio, or in each case in the form of a physiologically acceptable salt, or in each case in the form of a solvate,
**characterized in that** in a first step a) a 1-amino-3-aryl-butan-3-ol compound of the general formula II 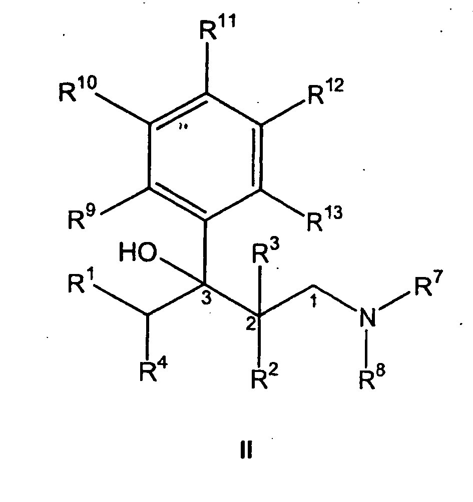 wherein R¹, R², R³, R⁴, R⁷, R⁸, R⁹, R¹⁰, R¹¹, R¹² and R¹³ have the abovementioned meaning, in each case optionally in the form of one of its pure stereoisomers, in particular enantiomers or diastereomers, its racemates or in the form of a mixture of stereoisomers, in particular the enantiomers or diastereomers, in any desired mixing ratio, or in each case in the form of a physiologically acceptable salt, or in each case in the form of a solvate, is employed and elimination is carried out under the action of an acid to give a substituted 3-aryl-but-3-enyl-amine compound of the general formula III 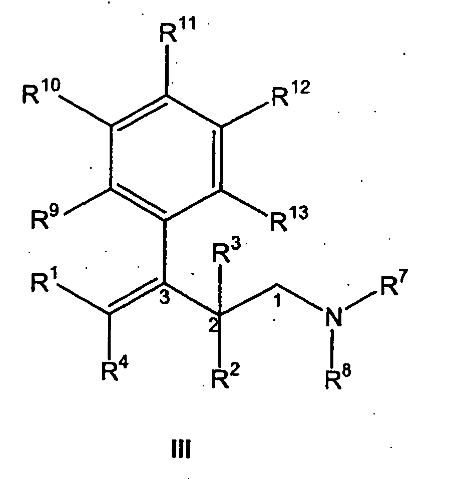 wherein R¹, R², R³, R⁴, R⁷, R⁸, R⁹, R¹⁰, R¹¹, R¹² and R¹³ have the abovementioned meaning, in each case optionally in the form of one of its pure stereoisomers, in particular enantiomers or diastereomers, its racemates or in the form of a mixture of stereoisomers, in particular the enantiomers or diastereomers, in any desired mixing ratio, or in each case in the form of a physiologically acceptable salt, or in each case in the form of a solvate, and in a second step b) the substituted 3-aryl-but-3-enyl-amine compound according to the general formula III formed is then hydrogenated under the participation of a metal catalyst and hydrogen to give a substituted 3-aryl-butyl-amine compound of the general formula I.

2. Process according to claim 1, **characterized in that** for compounds according t**o formula I, formula II and formula III**
R⁴ is chosen from H or CH₃.

3. Process according to claim 1, **characterized in that** for compounds according to formula I, **formula II and formula III**
R¹ is chosen from C₁₋₃-alkyl, saturated or unsaturated, substituted or unsubstituted, branched or unbranched.

4. Process according to claim 1, **characterized in that** for compounds according to formula I, **formula II and formula III**
R⁴ is chosen from H or CH₃,
**and/or**
R¹ is chosen from C₁₋₃-alkyl, saturated or unsaturated, substituted or unsubstituted, branched or unbranched.

5. Process according to one of claims 1 to 4, **characterized in that** for compounds according to **formula I, formula II and formula III**
R⁷ and R⁸ in each case independently of one another are chosen from H or CH₃.

6. Process according to one of claims 1 to 5, **characterized in that** for compounds according to formula I, **formula II and formula III**
R¹ is chosen from
C₁₋₃-alkyl, saturated and unsubstituted, branched or unbranched.

7. Process according to one of claims 1 to 6, **characterized in that** for compounds according to **formula I, formula II and formula III**
R² and R³ independently of one another are chosen from
H, C₁₋₄-alkyl, saturated and unsubstituted, branched or unbranched.

8. Process according to claim 7, **characterized in that** for compounds according to **formula I, formula II and formula III**
R³ = H and R² ≠ H.

9. Process according to one of claims 1 to 6, **characterized in that** for compounds according to **formula I, formula II and formula III**
R² and R³ together form a C₅₋₆-cycloalkyl radical, saturated or unsaturated, unsubstituted or mono-or polysubstituted.

10. Process according to one of claims 1 to 9, **characterized in that** for compounds according to **formula I, formula II and formula III**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
or R¹² and R¹¹ form a 3,4-OCH=CH ring.

11. Process according to claim 1, **characterized in that** for compounds according to **formula I, formula II and formula III**
R¹ is chosen from
C₁₋₃-alkyl, saturated and unsubstituted, branched or unbranched;
**and/or**
R² and R³ independently of one another are chosen from
H, C₁₋₄-alkyl, saturated and unsubstituted, branched or unbranched;
or
R² and R³ together form a C₅₋₆-cycloalkyl radical, saturated or unsaturated, unsubstituted or mono-or polysubstituted,
**and/or**
R⁴ is chosen from H,
**and/or**
R⁷ and R⁸ in each case independently of one another are chosen from
H or CH₃,
and/or
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
or R¹² and R¹¹ form a 3,4-OCH=CH ring.

12. Process according to one of claims 1 to 11, **characterized in that** for compounds according to **formula I** where R³ = H and R² ≠ H these are in the configurations Ia or Ib

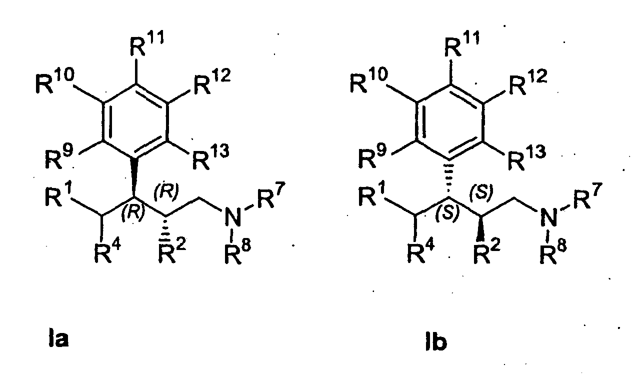

13. Process according to one of claims 1 to 12, **characterized in that** for compounds according to formula II where R³ = H and R² ≠ H these are in the configurations IIa or IIb 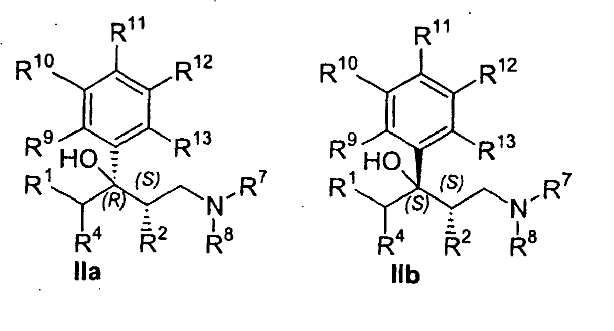 or in the configurations IIc and IId

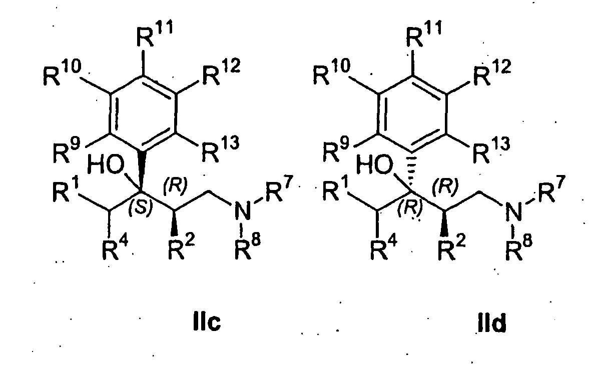

14. Process according to one of claims 1 to 13, **characterized in that** for compounds according to **formula III** where R³ = H, R² ≠ H, R⁴ = H and R¹ ≠ H these are in the configurations IIIa or IIIb 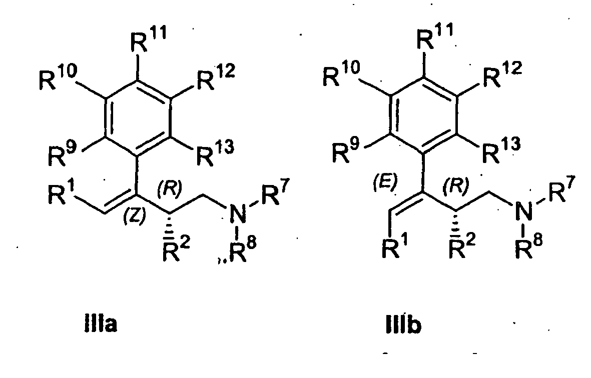 or for compounds according to formula III where R³ = H, R² ≠ H, R⁴ = H and R¹ ≠ H these are in the configurations IIIc or IIId

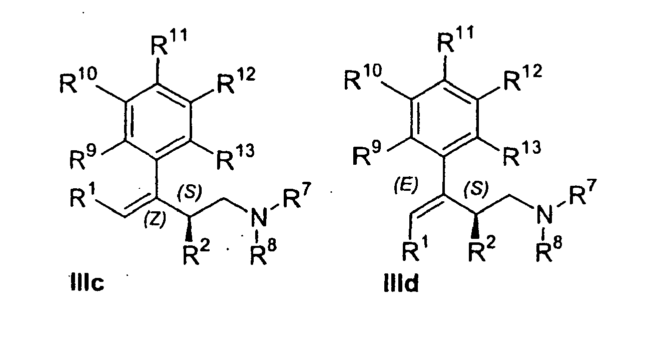

15. Process according to claim 1, **characterized in that** for the compound/compounds according to **formula I** at least one of these, preferably as the free base or as the hydrochloride, is chosen from the following group:
■ 3-(3-dimethylamino-1-ethyl-2-methyl-propyl)-phenol,
■ (1R,2R)-3-(3-dimethylamino-1-ethyl-2-methylpropyl)-phenol
■ (-)-(1R,2R)-3-(3-dimethylamino-1-ethyl-2-methylpropyl)-phenol,
■ (1S,2S)-3-(3-dimethylamino-1-ethyl-2-methylpropyl)-phenol
■ (+)-(1S,2S)-3-(3-dimethylamino-1-ethyl-2-methylpropyl)-phenol
■ (±)-(1RS,2RS)-3-(3-dimethylamino-1-ethyl-2-methyl-propyl)-phenol
■ *rac*-(1RS,2RS)-3-(3-dimethylamino-1-ethyl-2-methyl-propyl)-phenol,
■ [3-(3-methoxy-phenyl)-2-methyl-pentyl]-dimethylamine,
■ (2R,3R)-[3-(3-methoxy-phenyl)-2-methyl-pentyl]-dimethylamine,
■ (-)-(2R,3R)-[3-(3-methoxy-phenyl)-2-methylpentyl]-dimethylamine,
■ (2S,3S)-[3-(3-methoxy-phenyl)-2-methyl-pentyl]-dimethylamine,
■ (+)-(2S,3S)-[3-(3-methoxy-phenyl)-2-methylpentyl]-dimethylamine,
■ (±)-(2RS,3RS)-[3-(3-methoxy-phenyl)-2-methylpentyl]-dimethylamine,
■ *rac*(2RS,3RS)-[3-(3-methoxy-phenyl)-2-methylpentyl]-dimethylamine,
■ 3{[3-(p-isopropyl-phenyl-carbamoyl)-oxy-phenyl]-2-methyl-pentyl}-dimethylamine,
■ (2R,3R)-{3[3-(p-isopropyl-phenyl-carbamoyl)-oxyphenyl]-2-methyl-pentyl}-dimethylamine,
■ (2S,3S)-{3[3-(p-isopropyl-phenyl-carbamoyl)-oxyphenyl]-2-methyl-pentyl}-dimethylamine,
■ (2SR,3SR)-{3[3-(p-isopropyl-phenyl-carbamoyl)-oxy-phenyl]-2-methyl-pentyl}-dimethylamine.

16. Process according to claim 1, **characterized in that** for the compound/compounds according to **formula II** employed at least one of these, preferably as the free base or as the hydrochloride, is chosen from the following group:
■ 3-(3-dimethylamino-1-ethyl-1-hydroxy-2-methylpropyl)-phenol,
■ (1S,2S)-3-(3-dimethylamino-1-ethyl-1-hydroxy-2-methyl-propyl)-phenol,
■ (1R,25)-3-(3-dimethylamino-1-ethyl-1-hydroxy-2-methyl-propyl)-phenol,
■ (1RS,2SS)-3-(3-dimethylamino-1-ethyl-1-hydroxy-2-methyl-propyl)-phenol,
■ (1S,2R)-3-(3-dimethylamino-1-ethyl-1-hydroxy-2-methyl-propyl)-phenol,
■ (1R,2R)-3-(3-dimethylamino-1-ethyl-1-hydroxy-2-methyl-propyl)-phenol,
■ (1RS,2RR)-3-(3-dimethylamino-1-ethyl-1-hydroxy-2-methyl-propyl)-phenol,
■ [3-(3-methoxy-phenyl)-2-methyl-pentan-3-ol]-dimethylamine,
■ (2S,3S)-[3-(3-methoxy-phenyl)-2-methyl-pentan-3-ol]-dimethylamine,
■ (2S,3R)-[3-(3-methoxy-phenyl)-2-methyl-pentan-3-ol]-dimethylamine,
■ (2SS,3RS)-[3-(3-methoxy-phenyl)-2-methyl-pentan-3-ol]-dimethylamine,
■ (2R,3S)-[3-(3-methoxy-phenyl)-2-methyl-pentan-3-ol]-dimethylamine,
■ (2R,3R)-[3-(3-methoxy-phenyl)-2-methyl-pentan-3-ol]-dimethylamine
■ (2RR,3RS)-[3-(3-methoxy-phenyl)-2-methyl-pentan-3-ol]-dimethylamine,
■ {3[3-(p-isopropyl-phenyl-carbamoyl)-oxy-phenyl]-2-methyl-pentan-3-ol}-dimethylamine,
■ (2S,3R)-{3[3-(p-isopropyl-phenyl-carbamoyl)-oxyphenyl]-2-methyl-pentan-3-ol}-dimethylamine,
■ (2S,3S)-{3[3-(p-isopropyl-phenyl-carbamoyl)-oxyphenyl]-2-methyl-pentan-3-ol}-dimethylamine
■ (2SS,3RS)-{3[3-(p-isopropyl-phenyl-carbamoyl)-oxy-phenyl]-2-methyl-pentan-3-ol}-dimethylamine,
■ (2R,3S)-{3[3-(p-isopropyl-phenyl-carbamoyl)-oxyphenyl]-2-methyl-pentan-3-ol}-dimethylamine,
■ (2R,3R)-{3[3-(p-isopropyl-phenyl-carbamoyl)-oxyphenyl]-2-methyl-pentan-3-ol}-dimethylamine,
■ (2RR,3RS)-{3[3-(p-isopropyl-phenyl-carbamoyl)-oxy-phenyl]-2-methyl-pentan-3-ol}-dimethylamine.

17. Process according to claim 1, **characterized in that** for the compound/compounds according to **formula III** at least one of these, preferably as the free base or as the hydrochloride, is chosen from the following group:
■ 3-(3-dimethylamino-1-ethenyl-2-methyl-propyl)-phenol,
■ (Z)-(2R)-3-(3-dimethylamino-1-ethenyl-2-methylpropyl)-phenol,
■ (E)-(2R)-3-(3-dimethylamino-1-ethenyl-2-methylpropyl)-phenol,
■ (Z,E)-(2R)-3-(3-dimethylamino-1-ethenyl-2-methylpropyl)-phenol,
■ (Z)-(2S)-3-(3-dimethylamino-1-ethenyl-2-methylpropyl)-phenol,
■ (E)-(2S)-(3-dimethylamino-1-ethenyl-2-methylpropyl)-phenol,
■ (Z,E)-(2S)-3-(3-dimethylamino-1-ethenyl-2-methylpropyl)-phenol,
■ 3-(3-dimethylamino-1-ethenyl-2-methyl-propyl)-phenol,
■ (Z)-(2R)-3-(3-dimethylamino-1-ethenyl-1-2-methylpropyl)-phenol,
■ (E)-(2R)-3-(3-dimethylamino-1-ethenyl-1-2-methylpropyl)-phenol,
■ (Z,E)-(2R)-3-(3-dimethylamino-1-ethenyl-1-2-methyl-propyl)-phenol,
■ (Z)-(2S)-3-(3-dimethylamino-1-ethenyl-1-2-methylpropyl)-phenol,
■ (E)-(2S)-(3-dimethylamino-1-ethenyl-1-2-methylpropyl)-phenol,
■ (Z,E)-(2S)-3-(3-dimethylamino-1-ethenyl-2-methylpropyl)-phenol,
■ [3-(3-methoxy-phenyl)-2-methyl-pent-3-enyl]-dimethylamine,
■ (Z)-(2R)-[3-(3-methoxy-phenyl)-2-methyl-pent-3-enyl]-dimethylamine,
■ (E)-(2R)-[3-(3-methoxy-phenyl)-2-methyl-pent-3-enyl]-dimethylamine,
■ (Z,E)-(2R)-[3-(3-methoxy-phenyl)-2-methyl-pent-3-enyl]-dimethylamine,
■ (Z)-(2S)-[3-(3-methoxy-phenyl)-2-methyl-pent-3-enyl]-dimethylamine,
■ (E)-(2S)-[3-(3-methoxy-phenyl)-2-methyl-pent-3-enyl]-dimethylamine,
■ (Z,E)-(2S)-[3-(3-methoxy-phenyl)-2-methyl-pent-3-enyl]-dimethylamine,
■ {3[3-(p-isopropyl-phenyl-carbamoyl)-oxy-phenyl]-2-methyl-pent-3-enyl}-dimethylamine,
■ (Z)-(2R)-{3[3-(p-isopropyl-phenyl-carbamoyl)-oxyphenyl]-2-methyl-pent-3-enyl}-dimethylamine,
■ (E)-(2R)-{3[3-(p-isopropyl-phenyl-carbamoyl)-oxyphenyl]-2-methyl-pent-3-enyl}-dimethylamine,
■ (Z,E)-(2R)-{3[3-(p-isopropyl-phenyl-carbamoyl)-oxy-phenyl]-2-methyl-pent-3-enyl}-dimethylamine,
■ (Z)-(2S)-{3[3-(p-isopropyl-phenyl-carbamoyl)-oxyphenyl]-2-methyl-pent-3-enyl}-dimethylamine,
■ (E)-(2S)-{3[3-(p-isopropyl-phenyl-carbamoyl)-oxyphenyl]-2-methyl-pent-3-enyl}-dimethylamine,
■ (Z,E)-(2S)-{3[3-(p-isopropyl-phenyl-carbamoyl)-oxy-phenyl]-2-methyl-pent-3-enyl}-dimethylamine.

18. Process according to claim 1, **characterized in that** a chiral centre is present in the compound according to formula II employed, at position 2 according to formula II.

19. Process according to claim 1, **characterized in that** a chiral centre is present in the compound according to formula I, at position 2 according to formula I.

20. Process according to claim 1, **characterized in that** a chiral centre is present in the compound according to formula III, at position 2 according to formula III.

21. Process according to claim 1, **characterized in that** the compound according to formula II employed is enantiomerically pure.

22. Process according to claim 1, **characterized in that** the compound according to formula II employed is diastereomerically pure.

23. Process according to claim 1, **characterized in that** the compound according to formula II employed is enantiomerically and diastereomerically pure.

24. Process according to claim 1, **characterized in that** the compound according to formula II employed is chosen from:
• (2S),3S)-[3-(3-methoxy-phenyl)-2-methyl-pentan-3-ol]-dimethylamine,
• (2S,3R)-[3-(3-methoxy-phenyl)-2-methyl-pentan-3-ol]-dimethylamine
or
is a mixture of (2S,3S)-[3-(3-methoxy-phenyl)-2-methyl-pentan-3-ol]-dimethylamine and (2S,3R)-[3-(3-methoxy-phenyl)-2-methyl-pentan-3-ol]-dimethylamine, or (2SS,3RS)-[3-(3-methoxy-phenyl)-2-methyl-pentan-3-ol]-dimethylamine.

25. Process according to claim 1, **characterized in that** the compound according to formula II employed is chosen from:
• (2R,3R)-[3-(3-methoxy-phenyl)-2-methyl-pentan-3-ol]-dimethylamine,
• (2R,3S)-[3-(3-methoxy-phenyl)-2-methyl-pentan-3-ol]-dimethylamine
or
is a mixture of (2R,3R)-[3-(3-methoxy-phenyl)-2-methyl-pentan-3-ol]-dimethylamine and (2R,3S)-[3-(3-methoxy-phenyl)-2-methyl-pentan-3-ol]-dimethylamine, or (2RR,3RS)-[3-(3-methoxy-phenyl)-2-methyl-pentan-3-ol]-dimethylamine.

26. Process according to claim 1, **characterized in that** the compound according to formula II employed is chosen from:
• (2S,3S)-1-dimethylamino-3-(3-methoxyphenyl)-2-methyl-pentan-3-ol,
• (2S,3R)-1-dimethylamino-3-(3-methoxyphenyl)-2-methyl-pentan-3-ol
or
is a mixture of (2S,3S)-1-dimethylamino-3-(3-methoxyphenyl)-2-methyl-pentan-3-ol and (2S,3R)-1-dimethylamino-3-(3-methoxy-phenyl)-2-methyl-pentan-3-ol, or (2SS,3RS)-[3-(3-methoxy-phenyl)-2-methyl-pentan-3-ol]-dimethylamine.

27. Process according to claim 1, **characterized in that** the compound according to formula II employed is chosen from:
• (2R,3R)-1-dimethylamino-3-(3-methoxyphenyl)-2-methyl-pentan-3-ol,
• (2R,3S)-1-dimethylamino-3-(3-methoxyphenyl)-2-methyl-pentan-3-ol
or
is a mixture of (2R,3R)-1-dimethylamino-3-(3-methoxyphenyl)-2-methyl-pentan-3-ol and (2R,3S)-1-dimethylamino-3-(3-methoxy-phenyl)-2-methyl-pentan-3-ol, or (2RR,3RS)-[3-(3-methoxy-phenyl)-2-methyl-pentan-3-ol]-dimethylamine.

28. Process according to one of claims 1 to 17, **characterized in that** organic acids or halogen halide acids are used in step a).

29. Process according to claim 18, **characterized in that** formic acid, hydrochloric acid or hydrobromic acid are used in step a).

30. Process according to claim 18**, characterized in that** formic acid is used in step a).

31. Process according to claim 18, **characterized in that** hydrochloric acid is used in step a).

32. Process according to claim 18, **characterized in that** hydrobromic acid is used in step a).

33. Process according to claim 18, **characterized in that** the acid in step a) is employed in a high concentration.

34. Process according to claim 21, **characterized in that** the hydrochloric acid in step a) is > 20%.

35. Process according to one of claims 1 to 24, **characterized in that**, after step a), the compounds according to formula III which have undergone elimination are crystallized with hydrochloric acid gas.

36. Process according to one of claims 1 to 25, **characterized in that** the reaction time of step a) is between 2 and 10 h.

37. Process according to one of claims 1 to 26, **characterized in that** the reaction temperature in step a) is between 35 and 100°C.

38. Process according to one of claims 1 to 27, **characterized in that** the solvent in step a) is chosen from:
H₂O or alcohol or aqueous alcohol solutions.

39. Process according to one of claims 18 to 24, **characterized in that** the solvent in step a) is aqueous acid.

40. Process according to one of claims 18 to 24, **characterized in that,** in step a), the compound according to formula II employed is dissolved in aqueous acid.

41. Process according to one of claims 21 or 24, **characterized in that,** in step a), the compound according to formula II employed is dissolved in aqueous hydrochloric acid.

42. Process according to claim 1, **characterized in that,** in step b), the solvent is chosen from:
H₂O or alcohol or aqueous alcoholic or aqueous acidic solutions.

43. Process according to claim 1, **characterized in that,** in step b), the solvent is chosen from:
H₂O or ethanol or aqueous ethanolic solution or aqueous hydrochloric acid.

44. Process according to claim 1, **characterized in that,** in step b), the catalyst used comprises a noble metal.

45. Process according to claim 44, **characterized in that,** in step b), the catalyst used is palladium-on-active charcoal or palladium(II) chloride.

46. Process according to claim 44, **characterized in that,** in step b), the catalyst used is palladium-on-active charcoal (1 - 10 wt.%).

47. Process according to claim 1, **characterized in that** the temperature in step b) is kept between 20 and 40°C.

48. Process according to claim 1, **characterized in that,** in step b), an inert gas atmosphere is applied before the hydrogenation.

49. Process according to claim 1, **characterized in that,** in step b), the hydrogenation step takes place under a hydrogen pre-pressure of 3-10 bar
and/or
the hydrogenation step takes place under a hydrogen internal pressure of 0.5-3 bar.

50. Process according to claim 1, **characterized in that,** in step b), the starting substances are highly dilute/diluted in the solvent at the start.

51. Process according to claim 1, **characterized in that** the solvent for both steps a) and b) is an aqueous acidic solution.

52. Process according to claim 1 and 51, **characterized in that** no product is isolated between step a) and step b).

53. Process according to claim 52, **characterized in that** the starting substances are highly dilute/diluted in the solvent at the start.

54. Process according to one of claims 1 or 52, **characterized in that** the compound according to formula II employed is dissolved in aqueous acid.

55. Process according to claim 54, **characterized in that** the compound according to formula II employed is dissolved in aqueous hydrochloric acid.

56. Process for the preparation of a substituted 3-aryl-butyl-amine compound of the general formula I 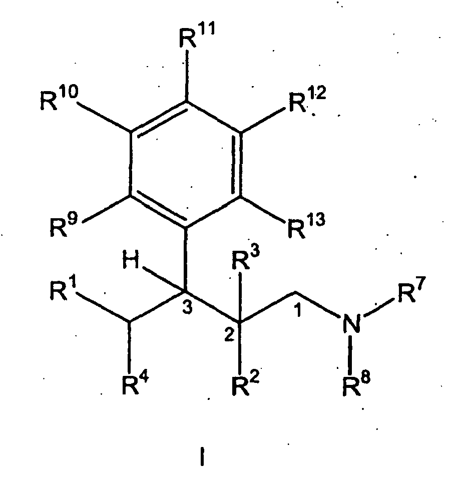 wherein
R¹ is chosen from H, C₁₋₃-alkyl, branched or unbranched, saturated or unsaturated, unsubstituted or mono- or polysubstituted,
R² and R³ in each case independently of one another are chosen from H or C₁₋₄-alkyl, branched or unbranched, saturated or unsaturated, unsubstituted or mono- or polysubstituted,
or
R² and R³ together form a saturated C₄₋₇-cycloalkyl radical, unsubstituted or mono- or polysubstituted,
R⁴ is chosen from H, C₁₋₃-alkyl, branched or unbranched, saturated or unsaturated, unsubstituted or mono- or polysubstituted,
R⁷ and R⁸ in each case independently of one another are chosen from H or C₁₋₃-alkyl, branched or unbranched, saturated or unsaturated, unsubstituted or mono- or polysubstituted,
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂, where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
in each case in the form of one of its pure stereoisomers, in particular enantiomers or diastereomers, its racemates or in the form of a mixture of stereoisomers, in particular the enantiomers or diastereomers, in any desired mixing ratio, or in each case in the form of a physiologically acceptable salt, or in each case in the form of a solvate,
**characterized in that** a substituted 3-aryl-but-3-enyl-amine compound of the general formula III 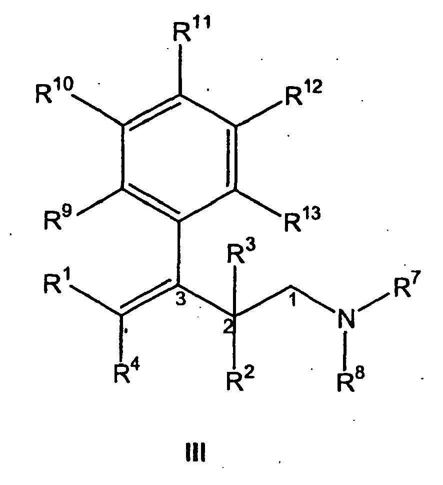 wherein R¹, R², R₃, R⁴, R⁷, R⁸, R⁹, R¹⁰, R¹¹, R¹² and R¹³ have the abovementioned meaning, in each case optionally in the form of one of its pure stereoisomers, in particular enantiomers or diastereomers, its racemates or in the form of a mixture of stereoisomers, in particular the enantiomers or diastereomers, in any desired mixing ratio, or in each case in the form of a physiologically acceptable salt, or in each case in the form of a solvate, is hydrogenated with the participation of a metal catalyst and hydrogen to give a substituted 3-aryl-butyl-amine compound of the general formula I.

57. Process according to claim 56, **characterized in that** for compounds according to formula I and formula III
R⁴ is chosen from H or CH₃.

58. Process according to claim 56, **characterized in that** for compounds according to formula I and formula III
R¹ is chosen from C₁₋₃-alkyl, saturated or unsaturated, substituted or unsubstituted, branched or unbranched.

59. Process according to claim 56, **characterized in that** for compounds according to formula I and formula III,
R⁴ is chosen from H or CH₃,
and/or
R¹ is chosen from C₁₋₃-alkyl, saturated or unsaturated, substituted or unsubstituted, branched or unbranched.

60. Process according to one of claims 56 to 59, **characterized in that** for compounds according to formula I and formula III
R⁷ and R⁸ in each case independently of one another are chosen from H or CH₃.

61. Process according to one of claims 56 to 60, **characterized in that** for compounds according to formula I and formula III
R¹ is chosen from
C₁₋₃-alkyl, saturated and unsubstituted, branched or unbranched.

62. Process according to one of claims 56 to 61, **characterized in that** for compounds according to formula I and formula III
R² and R³ independently of one another are chosen from
H, C₁₋₄-alkyl, saturated and unsubstituted, branched or unbranched.

63. Process according to claim 62, **characterized in that** for compounds according to formula I and formula III
R³ = H and R² ≠ H.

64. Process according to one of claims 56 to 61, **characterized in that** for compounds according to formula I and formula III
R² and R³ together form a C₅₋₆-cycloalkyl radical, saturated or unsaturated, unsubstituted or mono-or polysubstituted.

65. Process according to one of claims 56 to 64, **characterized in that** for compounds according to formula I and formula III
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
or R¹² and R¹¹ form a 3,4-OCH=CH ring.

66. Process according to claim 56, **characterized in that** for compounds according to formula I and formula III
R¹ is chosen from
C₁₋₃-alkyl, saturated and unsubstituted, branched or unbranched;
and/or
R² and R³ independently of one another are chosen from
H, C₁₋₄-alkyl, saturated and unsubstituted, branched or unbranched;
or
R² and R³ together form a C₅₋₆-cycloalkyl radical, saturated or unsaturated, unsubstituted or mono-or polysubstituted,
and/or
R⁴ is chosen from H,
and/or
R⁷ and R⁸ in each case independently of one another are chosen from
H or CH₃,
and/or
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched; or R¹² and R¹¹ form a 3,4-OCH=CH ring.

67. Process according to one of claims 56 to 66, **characterized in that** for compounds according to formula I where R³ = H and R² ≠ H these are in the configurations Ia or Ib

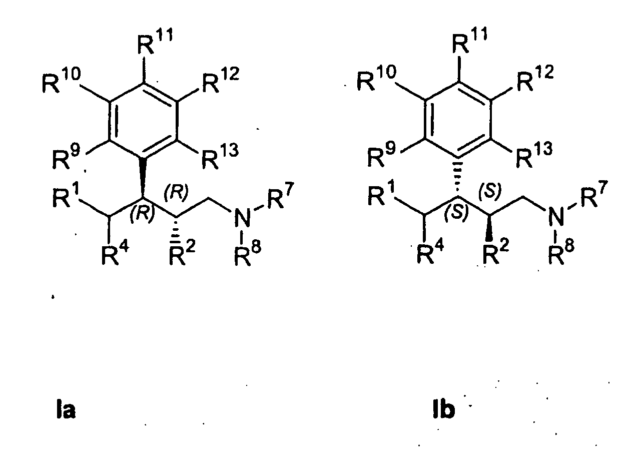

68. Process according to one of claims 56 to 67, **characterized in that** for compounds according to formula III where R³ = H, R² ≠ H, R⁴ = H and R¹ ≠ H these are in the configurations IIIa or IIIb 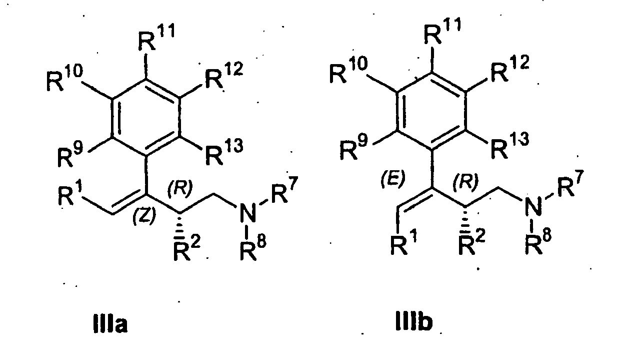 or for compounds according to formula III where R³ = H, R² ≠ H, R⁴ = H and R¹ ≠ H these are in the configurations IIIc or IIId

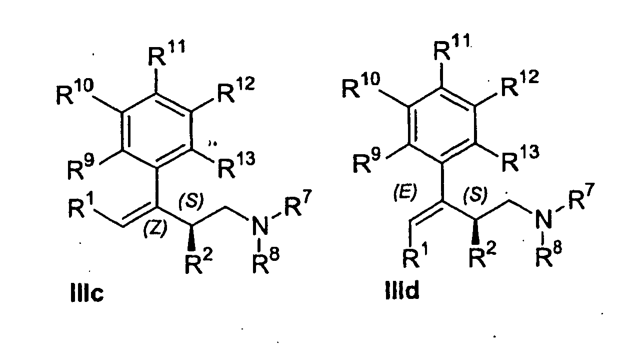

69. Process according to claim 56, **characterized in that** for the compound/compounds according to formula I at least one of these, preferably as the free base or as the hydrochloride, is chosen from the following group:
■ 3-(3-dimethylamino-1-ethyl-2-methyl-propyl)-phenol,
■ (1R,2R)-3-(3-dimethylamino-1-ethyl-2-methylpropyl)-phenol
■ (-)-(1R,2R)-3-(3-dimethylamino-1-ethyl-2-methylpropyl)-phenol,
■ (1S,2S)-3-(3-dimethylamino-1-ethyl-2-methylpropyl)-phenol
■ (+)-(1S,2S)-3-(3-dimethylamino-1-ethyl-2-methylpropyl)-phenol
■ (±)-(1RS,2RS)-3-(3-dimethylamino-1-ethyl-2-methyl-propyl)-phenol
■ *rac*-(1RS,2RS)-3-(3-dimethylamino-1-ethyl-2-methyl-propyl)-phenol,
■ [3-(3-methoxy-phenyl)-2-methyl-pentyl]-dimethylamine,
■ (2R,3R)-[3-(3-methoxy-phenyl)-2-methyl-pentyl]-dimethylamine,
■ (-)-(2R,3R)-[3-(3-methoxy-phenyl)-2-methylpentyl]-dimethylamine,
■ (2S,3S)-[3-(3-methoxy-phenyl)-2-methyl-pentyl]-dimethylamine,
■ (+)-(2S,3S)-[3-(3-methoxy-phenyl)-2-methylpentyl]-dimethylamine,
■ (±)-(2RS,3RS)-[3-(3-methoxy-phenyl)-2-methylpentyl]-dimethylamine,
■ rac(2RS,3RS)-[3-(3-methoxy-phenyl)-2-methylpentyl]-dimethylamine,
■ 3{[3-(p-isopropyl-phenyl-carbamoyl)-oxy-phenyl]-2-methyl-pentyl}-dimethylamine,
■ (2R,3R)-{3[3-(p-isopropyl-phenyl-carbamoyl)-oxyphenyl]-2-methyl-pentyl}-dimethylamine,
■ (2S,3S)-{3[3-(p-isopropyl-phenyl-carbamoyl)-oxyphenyl]-2-methyl-pentyl}-dimethylamine,
■ (2SR,3SR)-{3[3-(p-isopropyl-phenyl-carbamoyl)-oxy-phenyl]-2-methyl-pentyl}-dimethylamine.

70. Process according to claim 56, **characterized in that** for the compound/compounds according to formula III employed at least one of these, preferably as the free base or as the hydrochloride, is chosen from the following group:
■ 3-(3-dimethylamino-1-ethenyl-2-methyl-propyl)-phenol,
■ (Z)-(2R)-3-(3-dimethylamino-1-ethenyl-2-methylpropyl)-phenol,
■ (E)-(2R)-3-(3-dimethylamino-1-ethenyl-2-methylpropyl)-phenol,
■ (Z,E)-(2R)-3-(3-dimethylamino-1-ethenyl-2-methylpropyl)-phenol,
■ (Z)-(2S)-3-(3-dimethylamino-1-ethenyl-2-methylpropyl)-phenol,
■ (E)-(2S)-(3-dimethylamino-1-ethenyl-2-methylpropyl)-phenol,
■ (Z,E)-(2S)-3-(3-dimethylamino-1-ethenyl-2-methylpropyl)-phenol,
■ 3-(3-dimethylamino-1-ethenyl-2-methyl-propyl)-phenol,
■ (Z)-(2R)-3-(3-dimethylamino-1-ethenyl-1-2-methylpropyl)-phenol
■ (E)-(2R)-3-(3-dimethylamino-1-ethenyl-1-2-methylpropyl)-phenol
■ (Z,E)-(2R)-3-(3-dimethylamino-1-ethenyl-1-2-methyl-propyl)-phenol
■ (Z)-(2S)-3-(3-dimethylamino-1-ethenyl-1-2-methylpropyl)-phenol
■ (E)-(2S)-(3-dimethylamino-1-ethenyl-1-2-methylpropyl)-phenol
■ (Z,E)-(2S)-3-(3-dimethylamino-1-ethenyl-2-methylpropyl)-phenol
■ [3-(3-methoxy-phenyl)-2-methyl-pent-3-enyl]-dimethylamine,
■ (Z)-(2R)-[3-(3-methoxy-phenyl)-2-methyl-pent-3-enyl]-dimethylamine,
■ (E)-(2R)-[3-(3-methoxy-phenyl)-2-methyl-pent-3-enyl]-dimethylamine,
■ (Z,E)-(2R)-[3-(3-methoxy-phenyl)-2-methyl-pent-3-enyl]-dimethylamine,
■ (Z)-(2S)-[3-(3-methoxy-phenyl)-2-methyl-pent-3-enyl]-dimethylamine,
■ (E)-(2S)-[3-(3-methoxy-phenyl)-2-methyl-pent-3-enyl]-dimethylamine,
■ (Z,E)-(2S)-[3-(3-methoxy-phenyl)-2-methyl-pent-3-enyl]-dimethylamine,
■ {3[3-(p-isopropyl-phenyl-carbamoyl)-oxy-phenyl]-2-methyl-pent-3-enyl}-dimethylamine,
■ (Z)-(2R)-{3[3-(p-isopropyl-phenyl-carbamoyl)-oxyphenyl]-2-methyl-pent-3-enyl}-dimethylamine,
■ (E)-(2R)-{3[3-(p-isopropyl-phenyl-carbamoyl)-oxyphenyl]-2-methyl-pent-3-enyl}-dimethylamine,
■ (Z,E)-(2R)-{3[3-(p-isopropyl-phenyl-carbamoyl)-oxy-phenyl]-2-methyl-pent-3-enyl}-dimethylamine,
■ (Z)-(2S)-{3[3-(p-isopropyl-phenyl-carbamoyl)-oxyphenyl]-2-methyl-pent-3-enyl}-dimethylamine,
■ (E)-(2S)-{3[3-(p-isopropyl-phenyl-carbamoyl)-oxyphenyl]-2-methyl-pent-3-enyl}-dimethylamine,
■ (Z, E)-(2S)-{3[3-(p-isopropyl-phenyl-carbamoyl)-oxy-phenyl]-2-methyl-pent-3-enyl}-dimethylamine.

71. Process according to claim 56, **characterized in that** a chiral centre is present in the compound according to formula III employed, at position 2 according to formula III.

72. Process according to claim 56, **characterized in that** a chiral centre is present in the compound according to formula I, at position 2 according to formula I.

73. Process according to claim 56, **characterized in that** the compound according to formula III employed is enantiomerically pure.

74. Process according to claim 56, **characterized in that** the compound according to formula III employed is diastereomerically pure.

75. Process according to claim 56, **characterized in that** the compound according to formula III employed is enantiomerically and diastereomerically pure.

76. Process according to claim 56, **characterized in that** the compound according to formula III employed is chosen from:
• (Z)-(2R)-[3-(3-methoxy-phenyl)-2-methyl-pent-3-enyl]-dimethylamine,
• (E)-(2R)-[3-(3-methoxy-phenyl)-2-methyl-pent-3-enyl]-dimethylamine
or
is a mixture of (Z)-(2R)-[3-(3-methoxy-phenyl)-2-methyl-pent-3-enyl]-dimethylamine and (E)-(2R)-[3-(3-methoxy-phenyl)-2-methyl-pent-3-enyl]-dimethylamine, or (Z,E)-(2R)-[3-(3-methoxy-phenyl)-2-methyl-pent-3-enyl]-dimethylamine.

77. Process according to claim 56, **characterized in that** the compound according to formula III employed is chosen from:
• (Z)-(2S)-[3-(3-methoxy-phenyl)-2-methyl-pent-3-enyl]-dimethylamine,
• (E)-(2S)-[3-(3-methoxy-phenyl)-2-methyl-pent-3-enyl]-dimethylamine
or
is a mixture of (Z)-(2S)-[3-(3-methoxy-phenyl)-2-methyl-pent-3-enyl]-dimethylamine and (E)-(2S)-[3-(3-methoxy-phenyl)-2-methyl-pent-3-enyl]-dimethylamine, or (Z,E)-(2S)-[3-(3-methoxy-phenyl)-2-methyl-pent-3-enyl]-dimethylamine.

78. Process according to claim 56, **characterized in that** the solvent is chosen from:
H₂O or alcohol or aqueous alcoholic or aqueous acidic solutions.

79. Process according to claim 56, **characterized in that** the solvent is chosen from:
H₂O or ethanol or aqueous ethanolic solution or aqueous hydrochloric acid.

80. Process according to claim 56, **characterized in that** the catalyst used comprises a noble metal.

81. Process according to claim 80, **characterized in that** the catalyst used is palladium-on-active charcoal or palladium(II) chloride.

82. Process according to claim 80, **characterized in that** the catalyst used is palladium-on-active charcoal (1 - 10 wt.%).

83. Process according to claim 56, **characterized in that** the temperature is kept between 20 and 40°C.

84. Process according to claim 56, **characterized in that** an inert gas atmosphere is applied before the hydrogenation.

85. Process according to claim 56, **characterized in that** the hydrogenation step takes place under a hydrogen pre-pressure of 3-10 bar
and/or
the hydrogenation step takes place under a hydrogen internal pressure of 0.5-3 bar.

86. Process according to claim 56, **characterized in that** the starting substances are highly dilute/diluted in the solvent at the start.

87. Process according to claim 56, **characterized in that** the compound according to formula III employed is dissolved in aqueous acid.

88. Process according to claim 87, **characterized in that** the compound according to formula III employed is dissolved in aqueous hydrochloric acid.

89. Process according to one of claims 1 or 56, **characterized in that** the products are precipitated in an organic solvent at the end of the process.

90. Process according to one of claims 1 or 56, **characterized in that** the products are precipitated with acid or acid gas at the end of the process.

91. Process according to one of claims 1 or 56, **characterized in that** the products are precipitated in an organic solvent with acid or acid gas at the end of the process.

## Revendications

1. Procédé de production d'un composé substitué de 3-aryl-butylamine de formule générale I, 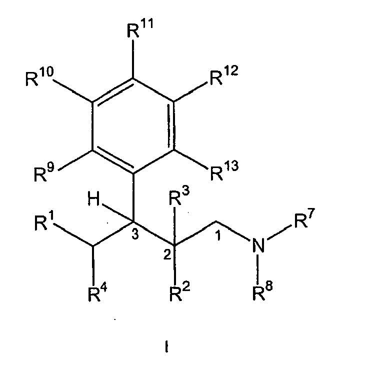 dans laquelle
R¹ est choisi entre H, un reste alkyle en C₁ à C₃, ramifié ou non ramifié, saturé ou non saturé, non substitué ou substitué une ou plusieurs fois,
R² et R³ sont tous deux choisis, indépendamment l'un de l'autre, entre H ou un reste alkyle en C₁ à C₄, ramifié ou non ramifié, saturé ou non saturé, non substitué ou substitué une ou plusieurs fois,
ou bien
R² et R³ forment ensemble un reste cycloalkyle en C₄ à C₇ saturé, non substitué ou substitué une ou plusieurs fois,
R⁴ est choisi entre H, un reste alkyle en C₁ à C₃, ramifié ou non ramifié, saturé ou non saturé, non substitué ou substitué une ou plusieurs fois,
R⁷ et R⁸ sont tous deux choisis, indépendamment l'un de l'autre, entre H ou un reste alkyle en C₁ à C₃, ramifié ou non ramifié, saturé ou non saturé, non substitué ou substitué une ou plusieurs fois,
R⁹ à R¹³ sont tous deux choisis, indépendamment l'un de l'autre, entre H, F, Cl, Br, I, CH₂F, CHF₂, CF₃, OH, SH, OR¹⁴, OCF₃, SR¹⁴, NR¹⁷R¹⁸, SOCH₃, SOCF₃, SO₂CH₃, SO₂CF₃, CN, COOR¹⁴, NO₂, CONR¹⁷R¹⁸, un reste alkyle en C₁ à C₆, ramifié ou non ramifié, saturé ou non saturé, non substitué ou substitué une ou plusieurs fois ; un reste phényle, non substitué ou substitué une ou plusieurs fois ;
R¹⁴ étant choisi entre un reste alkyle en C₁ à C₆ ; un reste pyridyle, thiényle, thiazolyle, phényle, benzyle ou phénéthyle, chacun non substitué ou substitué une ou plusieurs fois ; un groupe PO-(O-alkyle en C₁ à C₄)₂, CO-(O-alkyle en C₁ à C₅), CONH-C₆H₄-(alkyle en C₁ à C₃), CO-(alkyle en C₁ à C₅), CO-CHR¹⁷-NHR¹⁸, CO-C₆H₄-R¹⁵, R¹⁵ étant un radical ortho-OCO-(alkyle en C₁ à C₃) ou méta-ou para-CH₂N(R¹⁶)₂ où R¹⁶ est un radical alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ; R¹⁷ et R¹⁸ étant tous deux choisis, indépendamment l'un de l'autre, entre H ; un reste alkyle en C₁ à C₆, ramifié ou non ramifié, saturé ou non saturé, non substitué ou substitué une ou plusieurs fois ; un reste phényle, benzyle ou phénéthyle, chacun non substitué ou substitué une ou plusieurs fois
ou bien
R⁹ et R¹⁰ ou R¹⁰ et R¹¹ forment ensemble un noyau OCH₂O, OCH₂CH₂O, OCH=CH, CH=CHO, CH=C(CH₃)O, OC(CH₃)=CH, (CH₂)₄ ou OCH=CHO,
dans chaque cas sous forme de l'un de ses stéréo-isomères purs, en particulier des énantiomères ou des diastéréo-isomères, de ses racémates ou sous forme d'un mélange de stéréo-isomères, en particulier des énantiomères ou des diastéréo-isomères, dans un rapport de mélange quelconque, ou dans chaque cas sous forme d'un sel physiologiquement acceptable, ou dans chaque cas sous forme d'un produit de solvatation,
**caractérisé en ce que** dans une première étape a), un composé de 1-amino-3-arylbutane-3-ol de formule générale II 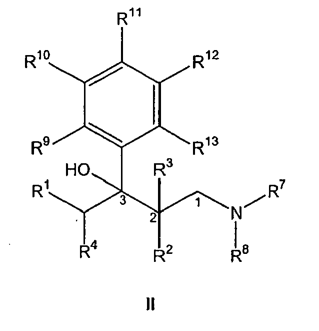 dans laquelle R¹, R², R³, R⁴, R⁷, R⁸, R⁹, R¹⁰, R¹¹, R¹² et R¹³ ont la définition indiquée ci-dessus, dans chaque cas éventuellement sous forme de l'un de ses stéréo-isomères purs, en particulier des énantiomères ou des diastéréo-isomères, de ses racémates ou sous forme d'un mélange de stéréo-isomères, en particulier des énantiomères ou des diastéréo-isomères, dans un rapport de mélange quelconque, ou dans chaque cas sous forme d'un sel physiologiquement acceptable, ou dans chaque cas sous forme d'un produit de solvatation, est mis en réaction et transformé par élimination sous l'action d'un acide en un composé substitué de 3-aryl-but-3-énylamine de formule générale III, 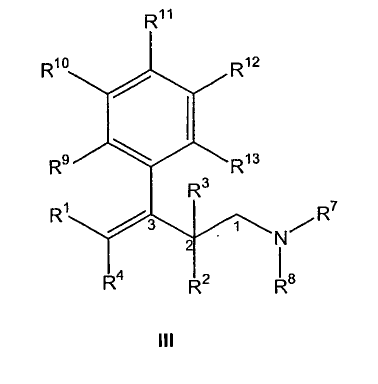 dans laquelle R¹, R², R³, R⁴, R⁷, R⁸, R⁹, R¹⁰, R¹¹, R¹² et R¹³ ont la définition mentionnée ci-dessus, dans chaque cas éventuellement sous forme de l'un de ses stéréo-isomères, en particulier des énantiomères ou des diastéréo-isomères, de ses racémates ou sous forme d'un mélange de stéréo-isomères, en particulier des énantiomères ou des diastéréo-isomères, dans un rapport de mélange quelconque, ou dans chaque cas sous forme d'un sel physiologiquement acceptable, ou dans chaque cas sous forme d'un produit de solvatation, et dans une seconde étape b), le composé substitué de 3-aryl-but-3-énylamine produit répondant à la formule générale III est ensuite hydrogéné avec la participation d'un catalyseur métallique et d'hydrogène en un composé substitué de 3-aryl-butylamine de formule générale I.

2. Procédé suivant la revendication 1, **caractérisé en ce que**, dans des composés de formule I, de formule II et de formule III,
R⁴ est choisi entre H ou CH₃.

3. Procédé suivant la revendication 1, **caractérisé en ce que,** dans des composés de formule I, de formule II et de formule III,
R¹ est choisi entre des restes alkyle en C₁ à C₃, saturés ou non saturés, substitués ou non substitués, ramifiés ou non ramifiés.

4. Procédé suivant la revendication 1, **caractérisé en ce que,** dans des composés de formule I, de formule II et de formule III,
R⁴ est choisi entre H ou CH₃,
et/ou
R¹ est choisi entre des restes alkyle en C₁ à C₃, saturés ou non saturés, substitués ou non substitués, ramifiés ou non ramifiés.

5. Procédé suivant l'une des revendications 1 à 4, **caractérisé en ce que**, dans des composés de formule I, de formule II et de formule III,
R⁷ et R⁸ sont tous deux choisis indépendamment l'un de l'autre entre H ou CH₃.

6. Procédé suivant l'une des revendications 1 à 5, **caractérisé en ce que** dans des composés de formule I, de formule II et de formule III,
R¹ est choisi entre
des restes alkyle en C₁ à C₃, saturés et non substitués, ramifiés ou non ramifiés.

7. Procédé suivant l'une des revendications 1 à 6, **caractérisé en ce que,** dans des composés de formule I, de formule II et de formule III,
R² et R³ sont choisis indépendamment l'un de l'autre entre H, des restes alkyle en C₁ à C₄, saturés et non substitués, ramifiés ou non ramifiés.

8. Procédé suivant la revendication 7, **caractérisé en ce que,** dans des composés de formule I, de formule II et de formule III,
R³ représente H et R² est différent de H.

9. Procédé suivant l'une des revendications 1 à 6, **caractérisé en ce que,** dans des composés de formule I, de formule II et de formule III,
R² et R³ forment ensemble un reste cycloalkyle en C₅ ou C₆, saturé ou non saturé, non substitué ou substitué une ou plusieurs fois.

10. Procédé suivant l'une des revendications 1 à 9, **caractérisé en ce que,** dans des composés de formule I, de formule II et de formule III,
R⁹ à R¹³, dont 3 ou 4 d'entre eux doivent représenter H, sont choisis indépendamment les uns des autres entre H, Cl, F, OH, CF₂H, CF₃ ou des restes alkyle en C₁ à C₄, saturés et non substitués, ramifiés ou non ramifiés ; OR¹⁴ ou SR¹⁴, R¹⁴ étant choisi entre des restes alkyle en C₁ à C₃, saturés et non substitués, ramifiés ou non ramifiés ;
ou bien R¹² et R¹¹ forment un noyau 3,4-OCH=CH.

11. Procédé suivant la revendication 1, **caractérisé en ce que,** dans des composés de formule I, de formule II et de formule III,
R¹ est choisi entre
des restes alkyle en C₁ à C₃, saturés et non substitués, ramifiés ou non ramifiés ;
et/ou
R² et R³ sont choisis indépendamment l'un de l'autre entre H, des restes alkyle en C₁ à C₄, saturés et non substitués, ramifiés ou non ramifiés ;
ou bien
R² et R³ forment ensemble un reste cycloalkyle en C₅ ou C₆, saturé ou non saturé, non substitué ou substitué une ou plusieurs fois,
et/ou
R⁴ représente H,
et/ou
R⁷ et R⁸ sont tous deux choisis, indépendamment l'un de l'autre, entre
H ou CH₃,
et/ou
R⁹ à R¹³, dont trois ou quatre d'entre eux doivent représenter H, sont choisis indépendamment les uns des autres entre
H, Cl, F, OH, CF₂H, CF₃ ou des restes alkyle en C₁ à C₄, saturés et non substitués, ramifiés ou non ramifiés ; OR¹⁴ ou SR¹⁴, R¹⁴ étant choisi entre des restes alkyle en C₁ à C₃, saturés et non substitués, ramifiés ou non ramifiés ;
ou bien R¹² et R¹¹ forment un noyau 3,4-OCH=CH.

12. Procédé suivant l'une des revendications 1 à 11, **caractérisé en ce que** des composés de formule I, dans laquelle R³ représente H et R² est différent de H, existent avec les configurations Ia ou Ib

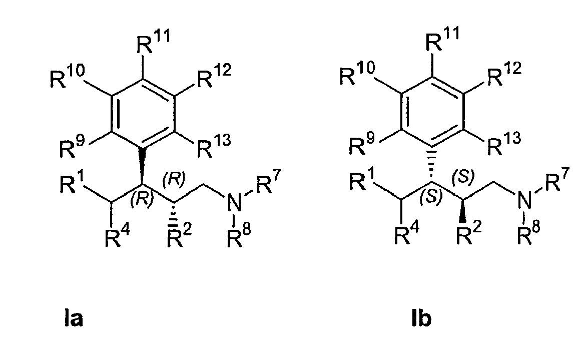

13. Procédé suivant l'une des revendications 1 à 12, **caractérisé en ce** des composés de formule II, dans laquelle R³ représente H et R² est différent de H, existent avec les configurations IIa ou IIb 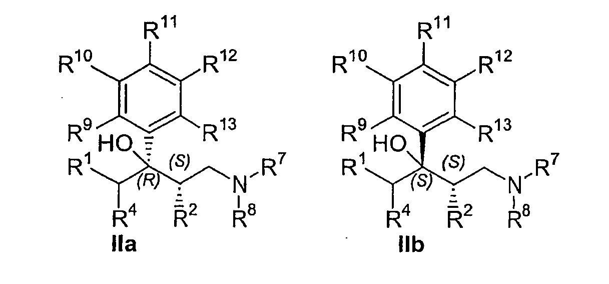 ou avec les configurations IIc et IId

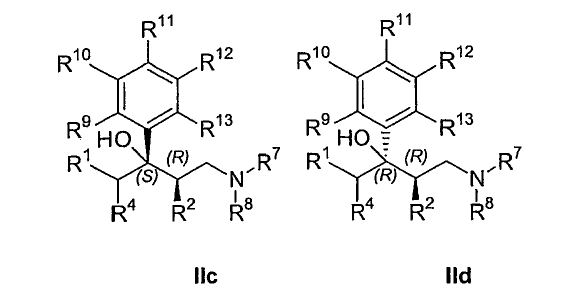

14. Procédé suivant l'une des revendications 1 à 13, **caractérisé en ce que** des composés de formule III, dans laquelle R³ représente H, R² est différent de H, R⁴ représente H et R¹ est différent de H, existent avec les configurations IIIa ou IIIb 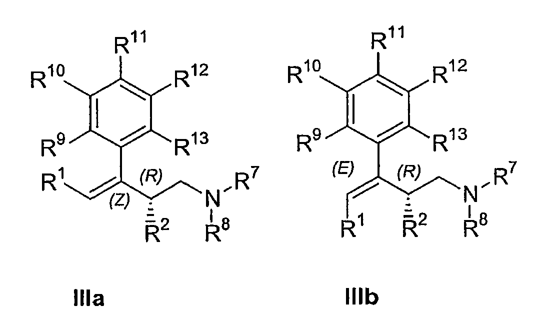 ou bien des composés de formule III, dans laquelle R³ représente H, R² est différent de H, R⁴ représente H et R¹ est différent de H, existent avec les configurations IIIc ou IIId

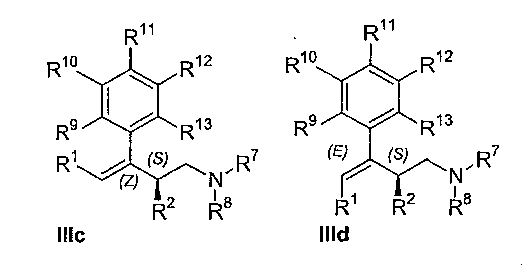

15. Procédé suivant la revendication 1, **caractérisé en ce que** le composé ou au moins l'un des composés de formule I, avantageusement sous forme de la base libre ou sous forme du chlorhydrate, est choisi dans le groupe suivant :
• 3-(3-diméthylamino-1-éthyl-2-méthyl-propyl)-phénol,
• (1R,2R)-3-(3-diméthylamino-1-éthyl-2-méthyl-propyl)-phénol,
• (-)-(1R,2R)-3-(3-diméthylamino-1-éthyl-2-méthyl-propyl)-phénol,
• (1S,2S)-3-(3-diméthylamino-1-éthyl-2-méthyl-propyl)-phénol,
• (+)-(1S,2S)-3-(3-diméthylamino-1-éthyl-2-méthyl-propyl)-phénol,
• (±)-(1RS,2RS)-3-(3-diméthylamino-1-éthyl-2-méthyl-propyl)-phénol,
• *rac*-(1RS,2RS)-3-(3-diméthylamino-1-éthyl-2-méthyl-propyl)-phénol,
• [3-(3-méthoxy-phényl)-2-méthyl-pentyl]-diméthylamine,
• (2R,3R)-[3-(3-méthoxy-phényl)-2-méthyl-pentyl]-diméthylamine,
• (-)-(2R,3R)-[3-(3-méthoxy-phényl)-2-méthyl-pentyl]-diméthylamine,
• (2S,3S)-[3-(3-méthoxy-phényl)-2-méthyl-pentyl]-diméthylamine,
• (+)-(2S,3S)-[3-(3-méthoxy-phényl)-2-méthyl-pentyl]-diméthylamine,
• (±)-(2RS,3RS)-[3-(3-méthoxy-phényl)-2-méthyl-pentyl]-diméthylamine,
• *rac*-(2RS,3RS)-[3-(3-méthoxy-phényl)-2-méthyl-pentyl]-diméthylamine,
• 3-{[3-(p-isopropyl-phényl-carbamoyl)-oxy-phényl]-2-méthyl-pentyl}-diméthylamine,
• (2R,3R)-{3-[3-(p-isopropyl-phényl-carbamoyl)-oxy-phényl]-2-méthyl-pentyl}-diméthylamine,
• (2S,3.S)-{3-[3-(p-isopropyl-phényl-carbamoyl)-oxy-phényl]-2-méthyl-pentyl}-diméthylamine,
• (2SR,3SR)-{3-[3-(p-isopropyl-phényl-carbamoyl)-oxy-phényl]-2-méthyl-pentyl}-diméthylamine.

16. Procédé suivant la revendication 1, **caractérisé en ce que,** concernant le composé mis en réaction/les composés mis en réaction répondant à la formule II, l'un d'eux au moins, avantageusement sous forme de base libre ou sous forme de chlorhydrate, est choisi dans le groupe suivant :
• 3-(3-diméthylamino-1-éthyl-1-hydroxy-2-méthyl-propyl)-phénol,
• (1S,2S)-3-(3-diméthylamino-1-éthyl-1-hydroxy-2-méthyl-propyl)-phénol,
• (1R,2S)-3-(3-diméthylamino-1-éthyl-1-hydroxy-2-méthyl-propyl)-phénol,
• (1RS,2SS)-3-(3-diméthylamino-1-éthyl-1-hydroxy-2-méthyl-propyl)-phénol,
• (1S,2R)-3-(3-diméthylamino-1-éthyl-1-hydroxy-2-méthyl-propyl)-phénol,
• (1R,2R)-3-(3-diméthylamino-1-éthyl-1-hydroxy-2-méthyl-propyl)-phénol,
• (1RS,2RR)-3-(3-diméthylamino-1-éthyl-1-hydroxy-2-méthyl-propyl)-phénol,
• [3-(3-méthoxy-phényl)-2-méthyl-pentane-3-ol]-diméthylamine,
• (2S,3S)-[3-(3-méthoxy-phényl)-2-méthyl-pentane-3-ol]-diméthylamine,
• (2S,3R)-[3-(3-méthoxy-phényl)-2-méthyl-pentane-3-ol]-diméthylamine,
• (2SS,3RS)-[3-(3-méthoxy-phényl)-2-méthyl-pentane-3-ol]-diméthylamine,
• (2R,3S)-[3-(3-méthoxy-phényl)-2-méthyl-pentane-3-ol]-diméthylamine,
• (2R,3R)-[3-(3-méthoxy-phényl)-2-méthyl-pentane-3-ol]-diméthylamine,
• (2RR,3RS)-[3-(3-méthoxy-phényl)-2-méthyl-pentane-3-ol]-diméthylamine,
• {3-[3-(p-isopropyl-phényl-carbamoyl)-oxy-phényl]-2-méthyl-pentane-3-ol}-diméthylamine,
• (2S,3R)-{3-[3-(p-isopropyl-phényl-carbamoyl)-oxy-phényl]-2-méthyl-pentane-3-ol}-diméthylamine,
• (2S,3S)-{3-[3-(p-isopropyl-phényl-carbamoyl)-oxy-phényl]-2-méthyl-pentane-3-ol}-diméthylamine,
• (2SS,3RS)-{3-[3-(p-isopropyl-phényl-carbamoyl)-oxy-phényl]-2-méthyl-pentane-3-ol}-diméthylamine,
• (2R,3S)-{3-[3-(p-isopropyl-phényl-carbamoyl)-oxy-phényl]-2-méthyl-pentane-3-ol}-diméthylamine,
• (2R,3R)-{3-[3-(p-isopropyl-phényl-carbamoyl)-oxy-phényl]-2-méthyl-pentane-3-ol}-diméthylamine,
• (2RR,3RS)-{3-[3-(p-isopropyl-phényl-carbamoyl)-oxy-phényl]-2-méthyl-pentane-3-ol}-diméthylamine.

17. Procédé suivant la revendication 1, **caractérisé en ce que,** concernant le composé/les composés de formule III, au moins l'un de ces composés, avantageusement sous forme de base libre ou sous forme de chlorhydrate, est choisi dans le groupe suivant :
• 3-(3-diméthylamino-1-éthényl-2-méthyl-propyl)-phénol,
• (Z)-(2R)-3-(3-diméthylamino-1-éthényl-2-méthyl-propyl)-phénol,
• (E)-(2R)-3-(3-diméthylamino-1-éthényl-2-méthyl-propyl)-phénol,
• (Z,E)-(2R)-3-(3-diméthylamino-1-éthényl-2-méthyl-propyl)-phénol,
• (Z)-(2S)-3-(3-diméthylamino-1-éthényl-2-méthyl-propyl)-phénol,
• (E)-(2S)-(3-diméthylamino-1-éthényl-2-méthyl-propyl)-phénol,
• (Z,E)-(2S)-3-(3-diméthylamino-1-éthényl-2-méthyl-propyl)-phénol,
• 3-(3-diméthylamino-1-éthényl-2-méthyl-propyl)-phénol,
• (Z)-(2R)-3-(3-diméthylamino-1-éthényl-1-2-méthyl-propyl)-phénol,
• (E)-(2R)-3-(3-diméthylamino-1-éthényl-1-2-méthyl-propyl)-phénol,
• (Z,E)-(2R)-3-(3-diméthylamino-1-éthényl-1-2-méthyl-propyl)-phénol,
• (Z)-(2S)-3-(3-diméthylamino-1-éthényl-1-2-méthyl-propyl)-phénol,
• (E)-(2S)-(3-diméthylamino-1-éthényl-1-2-méthyl-propyl)-phénol,
• (Z,E)-(2S)-3-(3-diméthylamino-1-éthényl-2-méthyl-propyl)-phénol,
• [3-(3-méthoxy-phényl)-2-méthyl-pent-3-ényl]-diméthylamine,
• (Z)-(2R)-[3-(3-méthoxy-phényl)-2-méthyl-pent-3-ényl]-diméthylamine,
• (E)-(2R)-[3-(3-méthoxy-phényl)-2-méthyl-pent-3-ényl]-diméthylamine,
• (Z,E)-(2R)-[3-(3-méthoxy-phényl)-2-méthyl-pent-3-ényl]-diméthylamine,
• (Z)-(2S)-[3-(3-méthoxy-phényl)-2-méthyl-pent-3-ényl]-diméthylamine,
• (E)-(2S)-[3-(3-méthoxy-phényl)-2-méthyl-pent-3-ényl]-diméthylamine,
• (Z,E)-(2S)-[3-(3-méthoxy-phényl)-2-méthyl-pent-3-ényl]-diméthylamine,
• {3-[3-(p-isopropyl-phényl-carbamoyl)-oxy-phényl]-2-méthyl-pent-3-ényl}-diméthylamine,
• (Z)-(2R)-{3-[3-(p-isopropyl-phényl-carbamoyl)-oxy-phényl]-2-méthyl-pent-3-ényl}-diméthylamine,
• (E)-(2R)-{3-[3-(p-isopropyl-phényl-carbamoyl)-oxy-phényl]-2-méthyl-pent-3-ényl}-diméthylamine,
• (Z,E)-(2R)-{3-[3-(p-isopropyl-phényl-carbamoyl)-oxy-phényl]-2-méthyl-pent-3-ényl}-diméthylamine,
• (Z)-(2S)-{3-[3-(p-isopropyl-phényl-carbamoyl)-oxy-phényl]-2-méthyl-pent-3-ényl}-diméthylamine,
• (E)-(2S)-{3-[3-(p-isopropyl-phényl-carbamoyl)-oxy-phényl]-2-méthyl-pent-3-ényl}-diméthylamine,
• (Z,E)-(2S)-{3-[3-(p-isopropyl-phényl-carbamoyl)-oxy-phényl]-2-méthyl-pent-3-ényl}-diméthylamine.

18. Procédé suivant la revendication 1, **caractérisé en ce qu'**un centre de chiralité est présent pour le composé de formule II mis en réaction, en position 2 selon la formule II.

19. Procédé suivant la revendication 1, **caractérisé en ce qu'**un centre de chiralité existe dans le composé de formule I en position 2 selon la formule I.

20. Procédé suivant la revendication 1, **caractérisé en ce qu'**un centre de chiralité existe dans le composé de formule III en position 2 selon la formule III.

21. Procédé suivant la revendication 1, **caractérisé en ce que** le composé de formule II mis en réaction est énantiomériquement pur.

22. Procédé suivant la revendication 1, **caractérisé en ce que** le composé de formule II mis en réaction est diastéréo-isomériquement pur.

23. Procédé suivant la revendication 1, **caractérisé en ce que** le composé de formule II mis en réaction est énantiomériquement pur et diastéréo-isomériquement pur.

24. Procédé suivant la revendication 1, **caractérisé en ce que** le composé de formule II mis en réaction est choisi entre :
• la (2S,3S)-[3-(3-méthoxy-phényl)-2-méthyl-pentane-3-ol]-diméthylamine,
• la (2S,3R)-[3-(3-méthoxy-phényl)-2-méthyl-pentane-3-ol]-diméthylamine,
ou bien
un mélange de (2S,3S)-[3-(3-méthoxy-phényl)-2-méthyl-pentane-3-ol]-diméthylamine et de (2S,3R)-[3-(3-méthoxy-phényl)-2-méthyl-pentane-3-ol]-diméthylamine, ou de (2SS,3RS)-[3-(3-méthoxy-phényl)-2-méthyl-pentane-3-ol]-diméthylamine,

25. Procédé suivant la revendication 1, **caractérisé en ce que** le composé de formule II mis en réaction est choisi entre :
• la (2R,3R)-[3-(3-méthoxy-phényl)-2-méthyl-pentane-3-ol]-diméthylamine,
• la (2R,3S)-[3-(3-méthoxy-phényl)-2-méthyl-pentane-3-ol]-diméthylamine,
ou bien
un mélange de (2R,3R)-[3-(3-méthoxy-phényl)-2-méthyl-pentane-3-ol]-diméthylamine et de (2R,3S)-[3-(3-méthoxy-phényl)-2-méthyl-pentane-3-ol]-diméthylamine ou de (2RR,3RS)-[3-(3-méthoxy-phényl)-2-méthyl-pentane-3-ol]-diméthylamine.

26. Procédé suivant la revendication 1, **caractérisé en ce que** le composé de formule II mis en réaction est choisi entre :
• le (2S,3S)-1-diméthylamino-3-(3-méthoxy-phényl)-2-méthyl-pentane-3-ol,
• le (2S,3R)-1-diméthylamino-3-(3-méthoxy-phényl)-2-méthyl-pentane-3-ol
ou
un mélange de (2S,3S)-1-diméthylamino-3-(3-méthoxy-phényl)-2-méthyl-pentane-3-ol et de (2S,3R)-1-diméthylamino-3-(3-méthoxy-phényl)-2-méthyl-pentane-3-ol ou de (2SS,3RS)-[3-(3-méthoxy-phényl)-2-méthyl-pentane-3-ol]-diméthylamine.

27. Procédé suivant la revendication 1, **caractérisé en ce que** le composé de formule II mis en réaction est choisi entre :
• le (2R,3R)-1-diméthylamino-3-(3-méthoxy-phényl)-2-méthyl-pentane-3-ol,
• le (2R,3S)-1-diméthylamino-3-(3-méthoxy-phényl)-2-méthyl-pentane-3-ol,
ou
un mélange de (2R,3R)-1-diméthylamino-3-(3-méthoxy-phényl)-2-méthyl-pentane-3-ol et de (2R,3S)-1-diméthylamino-3-(3-méthoxy-phényl)-2-méthyl-pentane-3-ol ou de (2RR,3RS)-[3-(3-méthoxy-phényl)-2-méthyl-pentane-3-ol]-diméthylamine.

28. Procédé suivant l'une des revendications 1 à 17, **caractérisé en ce qu'**on utilise dans l'étape a) des acides organiques ou des hydracides halogénés.

29. Procédé suivant la revendication 18, **caractérisé en ce qu'**on utilise dans l'étape a) de l'acide formique, de l'acide chlorhydrique ou de l'acide bromhydrique.

30. Procédé suivant la revendication 18, **caractérisé en ce qu'**on utilise dans l'étape a) l'acide formique.

31. Procédé suivant la revendication 18, **caractérisé en ce qu'**on utilise dans l'étape a) l'acide chlorhydrique.

32. Procédé suivant la revendication 18, **caractérisé en ce qu'**on utilise dans l'étape a) l'acide bromhydrique.

33. Procédé suivant la revendication 18, **caractérisé en ce que** l'acide est utilisé dans l'étape a) à une haute concentration.

34. Procédé suivant la revendication 21, **caractérisé en ce que** l'acide chlorhydrique dans l'étape a) est à plus de 20 %.

35. Procédé suivant l'une des revendications 1 à 24, **caractérisé en ce que,** après l'étape a), les composés éliminés de formule III sont cristallisés avec du gaz chlorhydrique.

36. Procédé suivant l'une des revendications 1 à 25, **caractérisé en ce que** la durée de réaction de l'étape a) s'élève entre 2 et 10 heures.

37. Procédé suivant l'une des revendications 1 à 26, **caractérisé en ce que,** dans l'étape a), la température de réaction s'élève entre 35 et 100°C.

38. Procédé suivant l'une des revendications 1 à 27, **caractérisé en ce que,** dans l'étape a), le solvant est choisi entre :
l'eau ou un alcool ou des solutions alcooliques aqueuses.

39. Procédé suivant l'une des revendications 18 à 24, **caractérisé en ce que,** dans l'étape a), le solvant est un acide aqueux.

40. Procédé suivant l'une des revendications 18 à 24, **caractérisé en ce que,** dans l'étape a), le composé de formule II utilisé est dissous dans un acide aqueux.

41. Procédé suivant l'une des revendications 21 ou 24, **caractérisé en ce que,** dans l'étape a), le composé utilisé de formule II est dissous dans de l'acide chlorhydrique aqueux.

42. Procédé suivant la revendication 1, **caractérisé en ce que,** dans l'étape b), le solvant est choisi entre :
H₂O ou un alcool ou des solutions alcooliques aqueuses ou acides aqueuses.

43. Procédé suivant la revendication 1, **caractérisé en ce que,** dans l'étape b), le solvant est choisi entre :
l'eau ou l'éthanol ou une solution éthanolique aqueuse ou l'acide chlorhydrique aqueux.

44. Procédé suivant la revendication 1, **caractérisé en ce que,** dans l'étape b), le catalyseur utilisé contient un métal noble.

45. Procédé suivant la revendication 44, **caractérisé en ce que,** dans l'étape b), le catalyseur utilisé est du palladium déposé sur du charbon actif ou du chlorure de palladium (II).

46. Procédé suivant la revendication 44, **caractérisé en ce que,** dans l'étape b), le catalyseur utilisé est du palladium déposé sur du charbon actif (à 1 - 10 % en poids).

47. Procédé suivant la revendication 1, **caractérisé en ce que** la température dans l'étape b) est maintenue entre 20 et 40°C.

48. Procédé suivant la revendication 1, **caractérisé en ce que,** dans l'étape b), une atmosphère de gaz protecteur est créée avant l'hydrogénation.

49. Procédé suivant la revendication 1, **caractérisé en ce que,** dans l'étape b), l'opération d'hydrogénation a lieu à une pression préalable d'hydrogène de 3 à 10 bars
et/ou
l'opération d'hydrogénation a lieu à une pression interne d'hydrogène de 0,5 à 3 bars.

50. Procédé suivant la revendication 1, **caractérisé en ce que,** dans l'étape b), les composés de départ, au début, sont fortement dilués ou se diluent fortement dans le solvant.

51. Procédé suivant la revendication 1, **caractérisé en ce que** le solvant pour les deux étapes a) et b) est une solution acide aqueuse.

52. Procédé suivant les revendications 1 et 51, **caractérisé en ce qu'**aucun produit n'est isolé entre l'étape a) et l'étape b).

53. Procédé suivant la revendication 52, **caractérisé en ce que** les composés de départ, au début, sont fortement dilués ou se diluent fortement dans le solvant.

54. Procédé suivant l'une des revendications 1 ou 52, **caractérisé en ce que** le composé de formule II mis en réaction est dissous dans un acide aqueux.

55. Procédé suivant la revendication 54, **caractérisé en ce que** le composé de formule II mis en réaction est dissous dans de l'acide chlorhydrique aqueux.

56. Procédé de production d'un composé substitué de 3-aryl-butylamine de formule générale I, 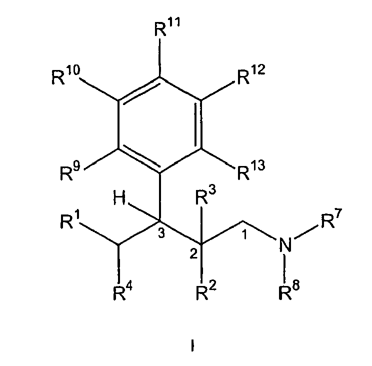 dans laquelle
R¹ est choisi entre H, un reste alkyle en C₁ à C₃, ramifié ou non ramifié, saturé ou non saturé, non substitué ou substitué une ou plusieurs fois,
R² et R³ sont tous deux choisis, indépendamment l'un de l'autre, entre H ou un reste alkyle en C₁ à C₄, ramifié ou non ramifié, saturé ou non saturé, non substitué ou substitué une ou plusieurs fois,
ou bien
R² et R³ forment ensemble un reste cycloalkyle en C₄ à C₇ saturé, non substitué ou substitué une ou plusieurs fois,
R⁴ est choisi entre H, un reste alkyle en C₁ à C₃, ramifié ou non ramifié, saturé ou non saturé, non substitué ou substitué une ou plusieurs fois,
R⁷ et R⁸ sont tous deux choisis, indépendamment l'un de l'autre, entre H ou un reste alkyle en C₁ à C₃, ramifié ou non ramifié, saturé ou non saturé, non substitué ou substitué une ou plusieurs fois,
R⁹ à R¹³ sont tous deux choisis, indépendamment l'un de l'autre, entre H, F, Cl, Br, I, CH₂F, CHF₂, CF₃, OH, SH, OR¹⁴, OCF₃, SR¹⁴, NR¹⁷R¹⁸, SOCH₃, SOCF₃, SO₂CH₃, SO₂CF₃, CN, COOR¹⁴, NO₂, CONR¹⁷R¹⁸, un reste alkyle en C₁ à C₆, ramifié ou non ramifié, saturé ou non saturé, non substitué ou substitué une ou plusieurs fois ; un reste phényle, non substitué ou substitué une ou plusieurs fois ;
R¹⁴ étant choisi entre un reste alkyle en C₁ à C₆ ; un reste pyridyle, thiényle, thiazolyle, phényle, benzyle ou phénéthyle, chacun non substitué ou substitué une ou plusieurs fois ; un groupe PO-(O-alkyle en C₁ à C₄)₂, CO-(O-alkyle en C₁ à C₅), CONH-C₆H₄-(alkyle en C₁ à C₃), CO-(alkyle en C₁ à C₅), CO-CHR¹⁷-NHR¹⁸, CO-C₆H₄-R¹⁵, R¹⁵ étant un radical ortho-OCO-(alkyle en C₁ à C₃) ou méta-ou para-CH₂N(R¹⁶)₂ où R¹⁶ est un radical alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ; R¹⁷ et R¹⁸ étant tous deux choisis, indépendamment l'un de l'autre, entre H ; un reste alkyle en C₁ à C₆, ramifié ou non ramifié, saturé ou non saturé, non substitué ou substitué une ou plusieurs fois ; un reste phényle, benzyle ou phénéthyle, chacun non substitué ou substitué une ou plusieurs fois
ou bien
R⁹ et R¹⁰ ou R¹⁰ et R¹¹ forment ensemble un noyau OCH₂O, OCH₂CH₂O, OCH=CH, CH=CHO, CH=C(CH₃)O, OC(CH₃)=CH, (CH₂)₄ ou OCH=CHO,
dans chaque cas sous forme de l'un de ses stéréo-isomères purs, en particulier des énantiomères ou des diastéréo-isomères, de ses racémates ou sous forme d'un mélange de stéréo-isomères, en particulier des énantiomères ou des diastéréo-isomères, dans un rapport de mélange quelconque, ou dans chaque cas sous forme d'un sel physiologiquement acceptable, ou dans chaque cas sous forme d'un produit de solvatation,
**caractérisé en ce qu'**un composé substitué de 3-aryl-but-3-énylamine de formule générale III, 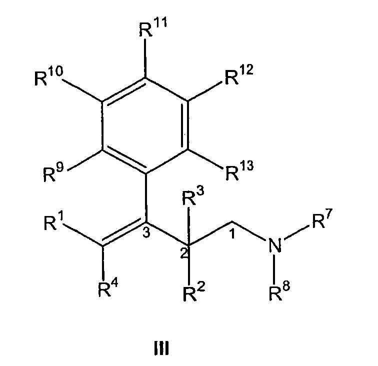 dans laquelle R¹, R², R³, R⁴, R⁷, R⁸, R⁹, R¹⁰, R¹¹, R¹² et R¹³ ont la définition mentionnée ci-dessus, dans chaque cas éventuellement sous forme de l'un de ses stéréo-isomères purs, en particulier des énantiomères ou des diastéréo-isomères, de ses racémates ou sous forme d'un mélange de stéréo-isomères, en particulier des énantiomères ou des diastéréo-isomères, dans un rapport de mélange quelconque, ou dans chaque cas sous forme d'un sel physiologiquement acceptable, ou dans chaque cas sous forme d'un produit de solvatation, est hydrogéné en un composé substitué de 3-aryl-butylamine de formule générale I avec participation d'un catalyseur métallique et d'hydrogène.

57. Procédé suivant la revendication 56, **caractérisé en ce que,** dans les composés de formule I et de formule III,
R⁴ est choisi entre H ou CH₃.

58. Procédé suivant la revendication 56, **caractérisé en ce que,** dans des composés de formule I et de formule III,
R¹ est choisi entre des restes alkyle en C₁ à C₃, saturés ou non saturés, substitués ou non substitués, ramifiés ou non ramifiés.

59. Procédé suivant la revendication 56, **caractérisé en ce que,** dans des composés de formule I et de formule III,
R⁴ est choisi entre H ou CH₃,
et/ou
R¹ est choisi entre des restes alkyle en C₁ à C₃, saturés ou non saturés, substitués ou non substitués, ramifiés ou non ramifiés.

60. Procédé suivant l'une des revendications 56 à 59, **caractérisé en ce que,** dans des composés de formule I et de formule III,
R⁷ et R⁸ sont tous deux choisis, indépendamment l'un de l'autre, entre H ou CH₃.

61. Procédé suivant l'une des revendications 56 à 60, **caractérisé en ce que,** dans des composés de formule I et de formule III,
R¹ est choisi entre
des restes alkyle en C₁ à C₃, saturés et non substitués, ramifiés ou non ramifiés.

62. Procédé suivant l'une des revendications 56 à 61, **caractérisé en ce que,** dans des composés de formule I et de formule III,
R² et R³ sont choisis, indépendamment l'un de l'autre, entre
H, des restes alkyle en C₁ à C₄, saturés et non substitués, ramifiés ou non ramifiés.

63. Procédé suivant la revendication 62, **caractérisé en ce que,** dans des composés de formule I et de formule III,
R³ représente H et R² est différent de H.

64. Procédé suivant l'une des revendications 56 à 61, **caractérisé en ce que,** dans des composés de formule I et de formule III,
R² et R³ forment ensemble un reste cycloalkyle en C₅ ou C₆, saturé ou non saturé, non substitué ou substitué une ou plusieurs fois.

65. Procédé suivant l'une des revendications 56 à 64, **caractérisé en ce que,** dans des composés de formule I et de formule III,
les restes R⁹ à R¹³, dont trois ou quatre d'entre eux doivent représenter H, sont choisis indépendamment les uns des autres entre
H, Cl, F, OH, CF₂H, CF₃ ou des restes alkyle en C₁ à C₄, saturés et non substitués, ramifiés ou non ramifiés ; OR¹⁴ ou SR¹⁴, R¹⁴ étant choisi entre des restes alkyle en C₁ à C₃, saturés et non substitués, ramifiés ou non ramifiés ;
ou bien R¹² et R¹¹ forment un noyau 3,4-OCH=CH.

66. Procédé suivant la revendication 56, **caractérisé en ce que,** dans des composés de formule I et de formule III,
R¹ est choisi entre
des restes alkyle en C₁ à C₃, saturés et non substitués, ramifiés ou non ramifiés ;
et/ou
R² et R³ sont choisis indépendamment l'un de l'autre entre H, des restes alkyle en C₁ à C₄, saturés et non substitués, ramifiés ou non ramifiés ;
ou bien
R² et R³ forment ensemble un reste cycloalkyle en C₅ ou C₆, saturé ou non saturé, non substitué ou substitué une ou plusieurs fois,
et/ou
R⁴ est choisi entre H,
et/ou
R⁷ et R⁸ sont tous deux choisis, indépendamment l'un de l'autre, entre
H ou CH₃,
et/ou
les restes R⁹ à R¹³, dont 3 ou 4 d'entre deux doivent représenter H, sont choisis indépendamment les uns des autres entre
H, Cl, F, OH, CF₂H, CF₃ ou des restes alkyle en C₁ à C₄, saturés et non substitués, ramifiés ou non ramifiés ; OR¹⁴ ou SR¹⁴, R¹⁴ étant choisi entre des restes alkyle en C₁ à C₃, saturés et non substitués, ramifiés ou non ramifiés ;
ou bien R¹² et R¹¹ forment un noyau 3,4-OCH=CH.

67. Procédé suivant l'une des revendications 56 à 66, **caractérisé en ce que** des composés de formule I dans laquelle R³ représente H et R² est différent de H, existent avec les configurations Ia ou Ib

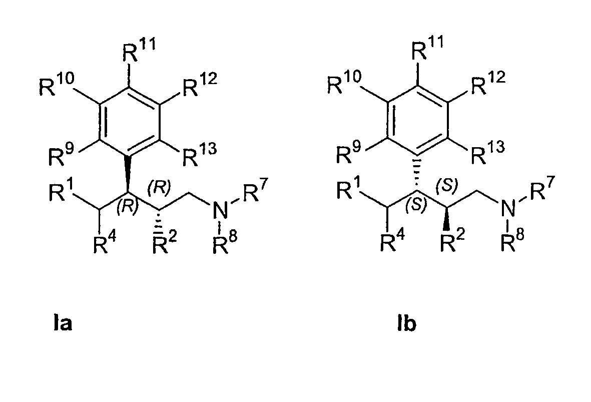

68. Procédé suivant l'une des revendications 56 à 67, **caractérisé en ce que** des composés de formule III dans laquelle R³ représente H, R² est différent de H, R⁴ représente H et R¹ est différent de H, existent avec les configurations IIIa ou IIIb 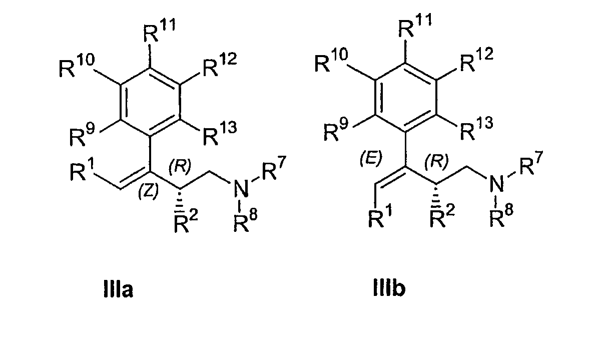 ou bien des composés de formule III dans laquelle R³ représente H, R² est différent de H, R⁴ représente H et R¹ est différent de H, existent avec les configurations IIIc ou IIId

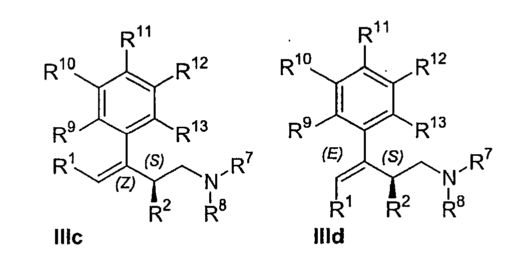

69. Procédé suivant la revendication 56, **caractérisé en ce que,** concernant le composé/les composés de formule I, l'un au moins de ces composés, avantageusement sous forme de base libre ou sous forme de chlorhydrate, est choisi dans le groupe suivant :
• 3-(3-diméthylamino-1-éthyl-2-méthyl-propyl)-phénol,
• (1R,2R)-3-(3-diméthylamino-1-éthyl-2-méthyl-propyl)-phénol,
• (-)-(1R,2R)-3-(3-diméthylamino-1-éthyl-2-méthyl-propyl)-phénol,
• (1S,2S)-3-(3-diméthylamino-1-éthyl-2-méthyl-propyl)-phénol,
• (+)-(1S,2S)-3-(3-diméthylamino-1-éthyl-2-méthyl-propyl)-phénol,
• (±)-(1RS,2RS)-3-(3-diméthylamino-1-éthyl-2-méthyl-propyl)-phénol,
• *rac*-(1RS,2RS)-3-(3-diméthylamino-1-éthyl-2-méthyl-propyl)-phénol,
• [3-(3-méthoxy-phényl)-2-méthyl-pentyl]-diméthylamine,
• (2R,3R)-[3-(3-méthoxy-phényl)-2-méthyl-pentyl]-diméthylamine,
• (-)-(2R,3R)-[3-(3-méthoxy-phényl)-2-méthyl-pentyl]-diméthylamine,
• (2S,3S)-[3-(3-méthoxy-phényl)-2-méthyl-pentyl]-diméthylamine,
• (+)-(2S,3S)-[3-(3-méthoxy-phényl)-2-méthyl-pentyl]-diméthylamine,
• (±)-(2RS,3RS)-[3-(3-méthoxy-phényl)-2-méthyl-pentyl]-diméthylamine,
• *rac*-(2RS,3RS)-[3-(3-méthoxy-phényl)-2-méthyl-pentyl]-diméthylamine,
• 3-{[3-(p-isopropyl-phényl-carbamoyl)-oxy-phényl]-2-méthyl-pentyl}-diméthylamine,
• (2R,3R)-{3-[3-(p-isopropyl-phényl-carbamoyl)-oxy-phényl]-2-méthyl-pentyl}-diméthylamine,
• (2S,3S)-{3-[3-(p-isopropyl-phényl-carbamoyl)-oxy-phényl]-2-méthyl-pentyl}-diméthylamine,
• (2SR,3SR)-{3-[3-(p-isopropyl-phényl-carbamoyl)-oxy-phényl]-2-méthyl-pentyl}-diméthylamine,

70. Procédé suivant la revendication 56, **caractérisé en ce que,** concernant le composé utilisé/les composés utilisés de formule III, au moins l'un d'entre eux, avantageusement sous forme de base libre ou sous forme de chlorhydrate, est choisi dans le groupe suivant :
• 3-(3-diméthylamino-1-éthényl-2-méthyl-propyl)-phénol,
• (Z)-(2R)-3-(3-diméthylamino-1-éthényl-2-méthyl-propyl)-phénol,
• (E)-(2R)-3-(3-diméthylamino-1-éthényl-2-méthyl-propyl)-phénol,
• (Z,E)-(2R)-3-(3-diméthylamino-1-éthényl-2-méthyl-propyl)-phénol,
• (Z)-(2S)-3-(3-diméthylamino-1-éthényl-2-méthyl-propyl)-phénol,
• (E)-(2S)-(3-diméthylamino-1-éthényl-2-méthyl-propyl)-phénol,
• (Z,E)-(2S)-3-(3-diméthylamino-1-éthényl-2-méthyl-propyl)-phénol,
• 3-(3-diméthylamino-1-éthényl-2-méthyl-propyl)-phénol,
• (Z)-(2R)-3-(3-diméthylamino-1-éthényl-1-2-méthyl-propyl)-phénol,
• (E)-(2R)-3-(3-diméthylamino-1-éthényl-1-2-méthyl-propyl)-phénol,
• (Z,E)-(2R)-3-(3-diméthylamino-1-éthényl-1-2-méthyl-propyl)-phénol,
• (Z)-(2S)-3-(3-diméthylamino-1-éthényl-1-2-méthyl-propyl)-phénol,
• (E)-(2S)-(3-diméthylamino-1-éthényl-1-2-méthyl-propyl)-phénol,
• (Z,E)-(2S)-3-(3-diméthylamino-1-éthényl-2-méthyl-propyl)-phénol,
• [3-(3-méthoxy-phényl)-2-méthyl-pent-3-ényl]-diméthylamine,
• (Z)-(2R)-[3-(3-méthoxy-phényl)-2-méthyl-pent-3-ényl]-diméthylamine,
• (E)-(2R)-[3-(3-méthoxy-phényl)-2-méthyl-pent-3-ényl]-diméthylamine,
• (Z,E)-(2R)-[3-(3-méthoxy-phényl)-2-méthyl-pent-3-ényl]-diméthylamine,
• (Z)-(2S)-[3-(3-méthoxy-phényl)-2-méthyl-pent-3-ényl]-diméthylamine,
• (E)-(2S)-[3-(3-méthoxy-phényl)-2-méthyl-pent-3-ényl]-diméthylamine,
• (Z,E)-(2S)-[3-(3-méthoxy-phényl)-2-méthyl-pent-3-ényl]-diméthylamine,
• {3-[3-(p-isopropyl-phényl-carbamoyl)-oxy-phényl]-2-méthyl-pent-3-ényl}-diméthylamine,
• (Z)-(2R)-{3-[3-(p-isopropyl-phényl-carbamoyl)-oxy-phényl]-2-méthyl-pent-3-ényl}-diméthylamine,
• (E)-(2R)-{3-[3-(p-isopropyl-phényl-carbamoyl)-oxy-phényl]-2-méthyl-pent-3-ényl}-diméthylamine,
• (Z,E)-(2R)-{3-[3-(p-isopropyl-phényl-carbamoyl)-oxy-phényl]-2-méthyl-pent-3-ényl}-diméthylamine,
• (Z)-(2S)-{3-[3-(p-isopropyl-phényl-carbamoyl)-oxy-phényl]-2-méthyl-pent-3-ényl}-diméthylamine,
• (E)-(2S)-{3-[3-(p-isopropyl-phényl-carbamoyl)-oxy-phényl]-2-méthyl-pent-3-ényl}-diméthylamine,
• (Z,E)-(2S)-{3-[3-(p-isopropyl-phényl-carbamoyl)-oxy-phényl]-2-méthyl-pent-3-ényl}-diméthylamine.

71. Procédé suivant la revendication 56, **caractérisé en ce qu'**un centre de chiralité existe dans le composé utilisé de formule III, en position 2 selon la formule III.

72. Procédé suivant la revendication 56, **caractérisé en ce qu'**un centre de chiralité existe dans le composé de formule I, en position 2 selon la formule I.

73. Procédé suivant la revendication 56, **caractérisé en ce que** le composé utilisé de formule III est énantiomériquement pur.

74. Procédé suivant la revendication 56, **caractérisé en ce que** le composé utilisé de formule III est diastéréo-isomériquement pur.

75. Procédé suivant la revendication 56, **caractérisé en ce que** le composé utilisé de formule III est énantiomériquement pur et diastéréo-isomériquement pur.

76. Procédé suivant la revendication 56, **caractérisé en ce que** le composé utilisé de formule III est choisi entre :
• la (Z)-(2R)-[3-(3-méthoxy-phényl)-2-méthyl-pent-3-ényl]-diméthylamine,
• la (E)-(2R)-[3-(3-méthoxy-phényl)-2-méthyl-pent-3-ényl]-diméthylamine,
ou
un mélange de (Z)-(2R)-[3-(3-méthoxy-phényl)-2-méthyl-pent-3-ényl]-diméthylamine et de (E)-(2R)-[3-(3-méthoxy-phényl)-2-méthyl-pent-3-ényl]-diméthylamine ou (Z,E)-(2R)-[3-(3-méthoxy-phényl)-2-méthyl-pent-3-ényl]-diméthylamine.

77. Procédé suivant la revendication 56, **caractérisé en ce que** le composé utilisé de formule III est choisi entre :
• la (Z)-(2S)-[3-(3-méthoxy-phényl)-2-méthyl-pent-3-ényl]-diméthylamine,
• la (E)-(2S)-[3-(3-méthoxy-phényl)-2-méthyl-pent-3-ényl]-diméthylamine
ou
un mélange de (Z)-(2S)-[3-(3-méthoxy-phényl)-2-méthyl-pent-3-ényl]-diméthylamine et (E)-(2S)-[3-(3-méthoxy-phényl)-2-méthyl-pent-3-ényl]-diméthylamine, ou (Z,E)-(2S)-[3-(3-méthoxy-phényl)-2-méthyl-pent-3-ényl]-diméthylamine.

78. Procédé suivant la revendication 56,
**caractérisé en ce que** le solvant est choisi entre :
l'eau ou un alcool ou des solutions alcooliques aqueuses ou acides aqueuses.

79. Procédé suivant la revendication 56, **caractérisé en ce que** le solvant est choisi entre :
l'eau ou l'éthanol ou une solution éthanolique aqueuse ou de l'acide chlorhydrique aqueux.

80. Procédé suivant la revendication 56, **caractérisé en ce que** le catalyseur utilisé contient un métal noble.

81. Procédé suivant la revendication 80, **caractérisé en ce que** le catalyseur utilisé est du palladium déposé sur du charbon actif ou du chlorure de palladium (II).

82. Procédé suivant la revendication 80, **caractérisé en ce que** le catalyseur utilisé est du palladium déposé sur du charbon actif (à 1 - 10 % en poids).

83. Procédé suivant la revendication 56, **caractérisé en ce que** la température est maintenue entre 20 et 40°C.

84. Procédé suivant la revendication 56, **caractérisé en ce qu'**une atmosphère de gaz protecteur est appliquée avant l'hydrogénation.

85. Procédé suivant la revendication 56, **caractérisé en ce que** l'étape d'hydrogénation est conduite à une pression préalable d'hydrogène de 3 - 10 bars
et/ou
l'étape d'hydrogénation est conduite à une pression interne d'hydrogène de 0,5 - 3 bars.

86. Procédé suivant la revendication 56, **caractérisé en ce qu'**au début, les composés de départ sont/ deviennent fortement dilués dans le solvant.

87. Procédé suivant la revendication 56, **caractérisé en ce que** le composé utilisé de formule III est dissous dans de l'acide aqueux.

88. Procédé suivant la revendication 87, **caractérisé en ce que** le composé utilisé de formule III est dissous dans de l'acide chlorhydrique aqueux.

89. Procédé suivant l'une des revendications 1 ou 56, **caractérisé en ce qu'**à la fin du procédé, les produits sont précipités dans un solvant organique.

90. Procédé suivant l'une des revendications 1 ou 56, **caractérisé en ce qu'**à la fin du procédé, les produits sont précipités avec un acide ou un gaz acide.

91. Procédé suivant l'une des revendications 1 ou 56, **caractérisé en ce qu'**à la fin du procédé, les produits sont précipités dans le solvant organique avec un acide ou un gaz acide.
